# EUROPÄISCHE PATENTANMELDUNG

(11) **EP 3 771 487 A1**
(43) Veröffentlichungstag der Anmeldung: **03.02.2021**
(21) Anmeldenummer: 20188427.7
(22) Anmeldetag: 29.07.2020
(51) Int. Cl.: B01J 13/00, B01J 13/02, B01J 20/10, B01J 20/28, B01J 20/32

(54) **VERFAHREN ZUR SIMULTANEN BESTIMMUNG VERSCHIEDENER ANALYTE IN EINER UMWELTPROBE UND/ODER EINEM AGRARROHSTOFF, GESTÜTZT AUF KERN-SCHALE-MIKROPARTIKEL**

(30) Priorität: 29.07.2019 DE 102019120455
(71) Anmelder: Bundesrepublik Deutschland, vertreten durch den Bundesminister für Wirtschaft und Energie, dieser vertreten durch den Präsidenten der, 12205 Berlin (DE)
(72) Erfinder: CARL, Peter, 10961 Berlin (DE); SCHWAAR, Timm, 12437 Berlin (DE); RURACK, Knut, 12247 Berlin (DE); SCHNEIDER, Rudolf, 14169 Berlin (DE); SARMA, Dominik, 12047 Berlin (DE)
(74) Vertreter: Zimmermann & Partner Patentanwälte mbB

(57) **Zusammenfassung**

Verfahren zur Herstellung einer Mischung von Kern/Schale-Mikropartikeln (1) zur simultanen Bestimmung verschiedener Analyte in einer Umweltprobe oder in einer Agrarrohstoffprobe, umfassend:
- Bereitstellen von zwei Kohorten (1.1, 1.2) von Kern/Schale Mikropartikeln (1), umfassend einen Polymerkern (2), der einen Luminophor (6) aufweist, und eine den Polymerkern (2) umschließende Silikat-Schale (3);
- Koppeln von zumindest zwei unterschiedlichen funktionellen Gruppen (4, 5) an die Silikat-Schale (3) der Kern/Schale-Mikropartikel (1), wobei eine erste der zumindest zwei unterschiedlichen funktionellen Gruppen (4, 5) ausgewählt ist unter einer -NH₂, -COOH,-CH(O), -NC, -C-SH, -CS-OH, -SO₂-OH, -SCN, -NCS oder -NCO - Gruppe (4); und eine zweite der zumindest zwei unterschiedlichen funktionellen Gruppen (4, 5) ausgewählt ist unter einem Polyethylenglycol (5), einem Sulfobetain (5) und einem Peptid (5) umfassend eine Aminosäuresequenz, die zumindest eine Sequenz von fünf Aminosäuren der Aminosäuresequenzen CYSYSYS oder CRERERE umfasst;
- Koppeln eines ersten anthropogenen Markers oder Toxins oder eines den ersten anthropogenen Marker oder das erste Toxin repräsentierenden ersten Haptens an die erste funktionelle Gruppe (1) von Kern/Schale-Mikropartikeln (1) einer ersten Kohorte (1.1);
- Koppeln eines zweiten anthropogenen Markers oder zweiten Toxins oder eines den zweiten anthropogenen Marker oder das zweite Toxin repräsentierenden zweiten Haptens an die erste funktionelle Gruppe (1) von Kern/Schale-Mikropartikeln (1) einer zweiten Kohorte (1.2);
wobei sich die erste und die zweite Kohorte hinsichtlich einer Art und/oder einer Konzentration des Luminophors (6) und/oder hinsichtlich eines mittleren Durchmessers und/oder hinsichtlich einer mittleren Dichte der Kern/Schale-Mikropartikel (1) unterscheiden;
und
- Vereinen und Mischen zumindest von Teilmengen der ersten (1.1) und der zweiten Kohorte (1.2) von Kern/Schale-Mikropartikeln (1).

## Beschreibung

### Technisches Gebiet

Die Erfindung liegt auf dem Gebiet der Anwendung von Lumineszenz-Immunoassays, basierend auf entsprechenden organischen Farbstoffen und anorganischen Markern, bspw. Carbon Nanodots (CND) oder Selten-Erd-Labeln wie Eu(III) und Tb(III) für Umweltproben, insbesondere für Abwasserproben und betrifft den simultanen Nachweis sogenannter anthropogener Marker und/oder Toxine und damit - je nach dem Ort der Probenahme - beispielsweise den Nachweis einer Abwassereinleitung in ein Gewässer, bzw. die Beurteilung der Güte eines Oberflächengewässers für die Trinkwassergewinnung oder für Erholungszwecke, oder die Überwachung der Wirksamkeit von Abwasserbehandlungsverfahren.

Ebenso liegt die Erfindung auf dem Gebiet der toxikologischen Überwachung landwirtschaftlicher Produkte und betrifft die Analyse von Agrarrohstoffen, insbesondere von Extrakten daraus, hinsichtlich ihres Gehaltes an Toxinen.

### Vorbekannter Stand der Technik

Multiplex-Assays, also der synchron geführte Nachweis unterschiedlicher Analyte in einer Probe mit einem einheitlichen Test werden, gemäß der sogenannten suspension array Technologie (SAT), vorrangig für Proben biologischen Ursprungs in der Biotechnologie, der klinischen Diagnostik und der biomedizinischen Forschung eingesetzt, um bei typischerweise geringer Probenmenge gegenüber traditionellen Methoden wie ELISA, Western Blotting, PCR und traditionellen Arrays die erforderliche Zeit, Arbeit und Kosten zu reduzieren. Beispielsweise umfasst die proprietäre xMAP®-Technologie diskrete Assays auf der Oberfläche von optisch kodierten Mikrokugeln, die dann in einem kompakten Analysator ausgelesen werden. Der Analysator liest unter Verwendung mehrerer Laser oder LEDs und Hochgeschwindigkeits-Digitalsignalprozessoren Multiplex-Assay-Ergebnisse aus, indem er die auf jeder einzelnen Mikrokugel auftretenden Reaktionen erfasst.

### Nachteile des Standes der Technik

Die vorbekannten Lösungen sind jedoch nur teilweise zufriedenstellend und erfordern einen hohen technischen Aufwand. Insbesondere gibt es keinen unmittelbar zur Verfügung stehenden Test zum parallelen Nachweis unterschiedlicher anthropogener Marker, Pestizide und/oder Toxine.

Zudem können typischerweise lediglich Amino-Gruppen tragende Moleküle an die kommerziell verfügbaren COOH-modifizierten Partikel gebunden werden. Weiterhin ist die unspezifische Bindung ein schwerwiegendes Problem, z.B. bei Bindungsassays wie indirekten Immunoassays zum Nachweis kleiner Moleküle. Bekannte SAT-Verfahren erfordern deshab zahlreiche Waschschritte zur Vorbereitung der Mikropartikel für den jeweiligen Test. Zusätzlich werden mit bekannten SAT-Verfahren die Antigene mit Hilfe von Trägerproteinen präsentiert. Diese Verfahren erfordern deshalb die gesonderte Präparation entsprechender Hapten-Trägerprotein-Konjugate. Eine solche Präparation umfasst mehrere Arbeitsschritte, ist oft nicht gut reproduzierbar und ist dadurch zeit- und kostenintensiv.

### Problemstellung

Es ist daher Aufgabe der vorliegenden Erfindung, kodierte Mikropartikel und ein robustes und dennoch hochempfindliches Verfahren ihrer Verwendung zum Nachweis anthropogener Analyte, einer Einleitung von Abwässern in Oberflächengewässer bzw. des Erfolgs von Abwasserbehandlungsverfahren bereitzustellen. Analog soll ein Verfahren zur Detektion von Toxinen in Agrarrohstoffen bereitgestellt werden. Dabei wird angestrebt, den Arbeits-, Zeit- und somit Kostenaufwand des darauf basierenden SAT-Verfahrens zu reduzieren.

### Erfindungsgemäße Lösung

Diese Aufgaben werden gelöst durch ein Verfahren der Herstellung von Kern/Schale-Mikropartikeln nach Anspruch 1 sowie ein auf die dabei erhaltenen Kern/Schale-Mikropartikel gestütztes Multiplex-Verfahren zur Abwasseranalyse und/oder Analyse von Agrarrohstoffen und ein entsprechendes Kit. Weitere Ausführungsformen, Modifikationen und Verbesserungen ergeben sich anhand der folgenden Beschreibung und der beigefügten Ansprüche.

Insbesondere wird erfindungsgemäß vorgeschlagen, solch bedeutsame anthropogene Marker wie Diclofenac (DCF) und Carbamazepin (CBZ) parallel zu Coffein (CAF) und Isolithocholsäure (ILA) simultan mit einem auf Kern/Schale-Mikropartikeln basierenden SAT-Test in Umweltproben nachzuweisen und/oder deren Konzentration in einem Gewässer oder einem Abwasserstrom zu überwachen. Als anthropogene Marker werden neben den genannten Analyten beispielsweise auch Coprostanol, Cholesterol, Kreatinin, Cotinin, Atenolol, Bisoprolol, Celiprolol, Clofibrinsäure, Diazepam, Dihydrocodein, Doxepin, Estron, Estradiol, Ethinylöstradiol, Galaxolid, Ibuprofen, Iopromid, Methadon, Metoprolol, Morphin, Norfloxacin, Oxazepam, Primidon, Propranolol, Roxithromycin, zahlreiche Sulfonamid-Antiinfektiva und Penicillin- sowie Penicillin-analoge Antibiotika, Tetracyclin, Gentamicin, Streptomycin, Sotalol und Tonalid, Kokain, Benzoylecgonin, THC, MDMA u.a. in wässrigen Proben bestimmt. Weitere Beispiele potentieller anthropogener Marker sind die Pestizide Glyphosat, Benzotriazol (und Methyl-Benzotriazolderivate), Mecoprop, Atrazin, Metolachlor, Carbofuran, Carbaryl, Imidacloprid, Fenitrothion, Chlorpyrifos-methyl, Triazophos; die Futtermittelzusatzstoffe Chloramphenicol, Clenbuterol, Tylosin; sowie materialbürtige Stoffe wie Phthalate, Nicht-Phthalat Weichmacher, Flammschutzmittel (PFOS, PFAS u.a.) und Bisphenole.

Weiter wird erfindungsgemäß vorgeschlagen, beispielsweise für die menschliche Ernährung, die Futtermittelindustrie und die Arzneimittelindustrie bedeutsame Toxine simultan mit einem auf Kern/Schale-Mikropartikeln basierenden SAT-Test zu bestimmen und dafür geeignete Kern/Schale-Mikropartikel bereitzustellen. Die betreffenden Toxine umfassen Mykotoxine, wie Aflatoxine (AF), Alternariol und Alternariol-monomethlyether, Cephalosporin, Chaetemin, Citrinin, Deoxynivalenol (DON), Fumagilin, Fumonisine, Fusarin C, Fusarinsäure, Gilotoxin, Griseofulvin, Kojisäure, Moniliformin, Mutterkornalkaloide (Ergotalkaloide), Mykophenolsäure, Nivalenol, Ochratoxin A (OTA), Patulin, Penicillin, Penitrem A, Roquefortin, Satratoxin, Sterigmatocystin, Tenuazonsäure, Trichotecene, (H)T-2-Toxin, Viomellein, Verrucosidin, Verruculogen, Xanthomegnin und Zearalenon (ZON). Es wird vorgeschlagen, zumindest zwei der genannten Toxine simultan mit einem auf Kern/Schale-Mikropartikeln basierenden SAT-Test in Agrarrohstoffen nachzuweisen bzw. deren Gehalt darin zu überwachen. Ebenso wird vorgeschlagen, mit dem vorgeschlagenen Bestimmungsverfahren Algentoxine (z. B. aus Cyanobakterien und Dinoflagellaten), wie Domoinsäure, Ciguatoxine und Maitotoxine, Okadasäure und deren Derivate (Dinophysistoxine DTX-1, DTX-2, DTX-3), Brevetoxine, Saxitoxine und Palytoxine und oben genannte Pestizide in Trinkwasserproben und Agrarrohstoffen und in Extrakten daraus zu bestimmen. Die bezeichneten Substanzen stellen durch ihr toxisches und cancerogenes Potential eine Gesundheitsgefahr dar.

Im Zusammenhang der vorliegenden Patentbeschreibung und der Patentansprüche werden unter dem Begriff "Analyt" sowohl die hier bezeichneten anthropogenen Marker als auch die hier beschriebenen Toxine subsummiert. Im Zusammenhang der vorliegenden Patentbeschreibung und der Patentansprüche umfassen die hier genannten Aflatoxine die Aflatoxine B1, B2, G1, G2 und M1. Von unmittelbar praktischem Vorteil ist die erfindungsgemäß erreichbare Kenntnis des Gehalts der Aflatoxine B1, M1 sowie des summarischen Gehalts der Aflatoxine B1, B2, G1 und G2 in Agrarrohstoffen bzw. Extrakten daraus. Unter Ergotalkaloiden werden hierbei die nachfolgend benannten Verbindungen subsummiert. Clavine: (Chanoclavine I und II, Paliclavin, Secolysergin, Secoagroclavin, Festuclavin, Pyroclavin, Costaclavin, Dihydroelymoclavin, Agroclavin, Elymoclavin, Molliclavin, Lysergin, Lysergen, Cividiclavin, Lysergol, Setoclavin und Penniclavin), Lysergsäure Paspalsäure, Lysergsäureamide (Ergin, Lysergsäurehydroxyethylamid, Ergometrin, Lysergsäurediemethylamid), Ergotamin, Ergovalin, α-Ergosin, β-Ergosin, Ergocristin, Ergocornin, α-Ergokryptin und β-Ergokryptin.

Vorteilhaft ermöglichen das hierzu vorgeschlagene Herstellungsverfahren, das vorgeschlagene Bestimmungsverfahren und der vorgeschlagen Kit, bzw. dessen/deren Verwendung, solche Agrarrohstoffe wie Getreide und Getreideprodukte, Mais, Nüsse und Hülsenfrüchte, Kaffee und Kakaobohnen, Ölpflanzen, Kräuter für arzneiliche Anwendungen, Futtermittel und Futtermittelprodukte, sowie anderes für die Verarbeitung vorgesehenes Pflanzenmaterial hinsichtlich der Einhaltung von Grenzwerten der bezeichneten Toxine zu überwachen. Die genannten Agrarrohstoffe bzw. Extrakte daraus können mithilfe der hierin beschriebenen Ausführungsformen simultan auf das Vorhandensein von verschiedenen Analyten, insbesondere von verschiedenen Toxinen, z.B. Mykotoxinen und/oder Pestiziden untersucht werden, was Rückschlüsse auf deren Gehalt in den entsprechendne Agrarrohstoffen zulässt. Besagte Toxine stellen eine erhebliche Gesundheitsgefahr dar, insbesondere, wenn sie durch die Verarbeitung Toxin-konatminierter Agrarrohstoffe in Nahrungs- oder Futtermittel gelangen. Die Agrarrohstoffe müssen daher, analog zu Umweltproben, kontinuierlich auf diese Substanzen untersucht werden und die Einhaltung von betreffenden Grenzwerten in Agrarrohstoffen überwacht werden.

DCF wurde in Konzentrationen bis zu 7,1 µg/L in eingeleiteten Abwässern von Abwasserbehandlungsanlagen gefunden, bei einer konventionellen Abwasserbehandlung wird es nur schlecht abgebaut und kann deshalb auch in Oberflächengewässer gelangen, wo es ökotoxikologische Schäden, u.a. an Fischen und Muscheln, verursacht. Der Anti-Epilepsie Wirkstoff Carbamazepin wurde mit bis zu 5,0 µg/L in Abwässern nachgewiesen und bereits zuvor als anthropogener Marker vorgeschlagen. Coffein, das in Abwasserbehandlungsanlagen problemlos nahezu vollständig abgebaut wird, kann zum Nachweis der Einleitung unbehandelter kommunaler Abwässer in Oberflächengewässer dienen. Auch die Gallensäure Isolithocholsäure wird in Abwasserbehandlungsanlagen kommunaler Abwässer, in denen es Konzentrationen von bis zu 70 µg/L erreicht, fast vollständig abgebaut und kann, wie auch andere Gallensäuren, zum Nachweis der Einleitung unbehandelter kommunaler Abwässer in Oberflächengewässer dienen.

Die oben genannten Mykotoxine, inbesondere OTA, DON, ZON, AFB1, FB1, T2 wurden in einer Konzentration von bis zu 5 mg/kg nachgewiesen. In der Verordnung 1881/200 der Europäischen Union sind Grenzwerte von typischerweise 2 µg/kg (AFB1) bis 1250 µg/kg (DON) z. B. für unverarbeitetes Getreide festgesetzt. Diese Grenzwerte müssen durch Analysen zum Schutz der öffentlichen Gesundheit (vgl. Absatz 2 EG 1881/2006) kontrolliert werden.

Gemäß einer Ausführungsform wird ein Verfahren zur Herstellung einer Mischung von Kern/Schale-Mikropartikeln zur simultanen Bestimmung verschiedener Analyte in einer Umweltprobe und/oder in einer Agrarrohstoffprobe oder einem Extrakt daraus vorgeschlagen. Das Verfahren umfasst die Schritte:
(1) Bereitstellen von zumindest zwei Kohorten von Kern/Schale-Mikropartikeln. Die Mikropartikel sind Kern/Schale-Partikel und umfassen einen Polymerkern, der einen Luminophor aufweist, und eine den Polymerkern umschließende Silikat-Schale, wobei sich die erste und die zweite Kohorte hinsichtlich einer Art und/oder einer Konzentration des Luminophors und/oder hinsichtlich eines mittleren Durchmessers und/oder hinsichtlich einer mittleren Dichte der Kern/Schale-Mikropartikel unterscheiden, bzw. im FACS oder einer anderen geeigneten Messanordnung eindeutig voneinander unterscheidbar sind.
   Der Polymerkern der Kern/Schale-Mikropartikel weist typischerweise einen mittleren arithmetischen Durchmesser von 0,1 - 7 µm, beispielsweise 0,2 bis 3 µm, 0,2 - 2 µm oder 0,5 - 1,5 µm; die Schale weist typischerweise eine Dicke auf im Bereich von 10 - 200 nm, beispielsweise 10, 20, 25, 30, 35, 40, 45, 50, 55, 75, 100, 125, 150, 175, 190, oder 200 nm; und das gesamte Kern/Schale-Mikropartikel weist einen mittleren arithmetischen Durchmesser von 0,1 - 7 µm, beispielsweise 0,5 µm bis 2,5 µm, bevorzugt 0,7 µm bis 2 µm auf.
   Der mittlere arithmetische Durchmesser dieser Größenbereiche kann für hinreichend monodisperse Partikel beispielsweise unter Verwendung bekannter Streulichttechniken oder z.B. mittels (Raster-) Elektronenmikroskopie bestimmt werden. Die Dicke der Schale kann, beispielsweise, mit einem Elektronenmikroskop bestimmt werden.
(2) Koppeln von zumindest zwei unterschiedlichen funktionellen Gruppen an die Silikat-Schale der Kern/Schale-Mikropartikel, wobei eine erste der zumindest zwei unterschiedlichen funktionellen Gruppen ausgewählt ist unter einer -NH₂, -COOH, -CH(O), -NC, -C-SH, -CS-OH, -SO₂-OH, -SCN, -NCS oder -NCO -Gruppe; und eine zweite der zumindest zwei unterschiedlichen funktionellen Gruppen ausgewählt ist unter einem Polyethylenglycol, einem Sulfobetain und einem Peptid umfassend eine Aminosäuresequenz, die zumindest eine Sequenz von fünf Aminosäuren der Aminosäuresequenzen CYSYSYS oder CRERERE umfasst (hierbei steht C für Cystein, Y für Tyrosin, S für Serin, R für Arginin und E für Glutaminsäure).
(3) Koppeln eines ersten anthropogenen Markers oder eines ersten Toxins, oder eines den ersten anthropogenen Marker oder das erste Toxins repräsentierenden oder zumindest teilweise repräsentierenden ersten Haptens an die erste funktionelle Gruppe von Kern/Schale-Mikropartikeln der ersten Kohorte.
(4) Koppeln eines zweiten anthropogenen Markers oder eines zweiten Toxins oder eines den zweiten anthropogenen Marker oder das zweite Toxin repräsentierenden zweiten Haptens an die erste funktionelle Gruppe von Kern/Schale-Mikropartikeln der zweiten Kohorte. Analoge Schritte werden mit weiteren anthropogenen Markern bzw. Toxinen bzw. Haptenen an den ersten funktionellen Gruppen weiterer Kohorten von Kern/Schale-Mikropartikeln vorgenommen, bis alle Kohorten jeweils eine Art der interessierenden Analyten oder eine Art des jenen repräsentierenden Haptens aufweisen.
(5) Vereinen und Mischen zumindest von Teilmengen der ersten und der zweiten Kohorte von Kern/Schale-Mikropartikeln.

Vorteilhaft ermöglicht die erfindungsgemäß bereitgestellte Mischung von Kern/Schale-Mikropartikeln unterschiedlicher Selektivität, d.h. der beschriebenen unterschiedlichen Kohorten, die sich durch ihre optisch auslesbare Kodierung unterscheiden, den simultanen Nachweis verschiedener Analyte in einer Probe. Die parallele Verwendung von zumindest zwei unterschiedlichen funktionellen Gruppen ermöglicht die kovalente Anbindung eines gewünschten Antigens und unabhängig davon die Ausrüstung der Mikropartikel mit einem anti-fouling-Molekül. Die für PEG-modifizierte Flächen bekannten anti-fouling-Eigenschaften können auch auf Kern/Schale-Mikropartikel übertragen werden. Die anti-fouling-Eigenschaften der beiden benannten Peptide sind bekannt aus Ye H, Wang L, Huang R, et al. (2015): Superior Antifouling Performance of a Zwitterionic Peptide Compared to an Amphiphilic, Non-Ionic Peptide. Applied Materials & Interfaces 7(40):22448-57 (doi: 10.1021/acsami.5b06500). Auch für Peptide, deren Sequenz lediglich fünf der benannten sieben aufeinanderfolgenden Aminosäuren umfasst, sind hinreichende anti-fouling-Eigenschaften zu erwarten. Dabei ist es gleichgültig, ob Aminosäurereste am C-, am N-, oder am C- und am N-Ende der Peptide fehlen.

Unter "anti-fouling" wird in diesem Zusammenhang das Verhindern einer unerwünschten unspezifischen Bindung eines Proteins, beispielsweise eines Antikörpers, und damit das mögliche Zustandekommen eines falsch-positiven Messsignals verstanden.

Gemäß einer Modifizierung des Schritts (3) "Koppeln eines ersten anthropogenen Markers oder eines ersten Toxins oder eines diesen/dieses repräsentierenden ersten Haptens...." kann das Koppeln auch über die Anbindung eines Protein-Konjugats des anthropogenen Markers bzw. des Toxins mit einem Trägerprotein (z.B. Rinderserumalbumin (BSA), Ovalbumin (OVA), Peroxidase (POD), Alkalischer Phosphatase (ALP), Thyroglobulin (TG), Keyhole Limpet Hemocyanin (KLH)) bzw. eines Hapten-Konjugates mit einem solchen Trägerprotein erfolgen. Vorteilhaft sind solche Konjugate kommerziell erhältlich.

Gemäß einer Ausführungsform sind die anthropogenen Marker ausgewählt unter: Carbamazepin (CBZ), Coffein (CAF), Coprostanol, Cholesterol, Diclofenac (DCF), Isolithocholsäure (ILA), Kreatinin, Cotinin, Atenolol, Bisoprolol, Celiprolol, Clofibrinsäure, Diazepam, Dihydrocodein, Doxepin, Estron, Estradiol, Ethinylöstradiol, Galaxolid, Ibuprofen, Iopromid, Methadon, Metoprolol, Morphin, Norfloxacin, Oxazepam, Benzotriazol und Methyl-Benzotriazolderivate, Mecoprop, einem Östradiol, Primidon, Propranolol, Roxithromycin, Sulfonamid-Antiinfektiva, Penicillin- und Penicillin-analoge Antibiotika, Tetracyclin, Gentamicin, Streptomycin, Sotalol und Tonalid, Kokain, Benzoylecgonin, THC, MDMA, Glyphosat, Atrazin, Metolachlor, Carbofuran, Carbaryl, Imidacloprid, Fenitrothion, Chlorpyrifos-methyl, Triazophos, Chloramphenicol, Clenbuterol, Tylosin, Phthalate, Nicht-Phthalat Weichmacher, eine poly- oder einer perfluorierten Alkyl substanz (PFAS), insbesondere Perfluoroktansulfonsäure (PFOS); und einem Bisphenol.

Gemäß einer Ausführungsform sind das erste, das zweite und gegebenenfalls weitere Toxine unabhängig voneinander ausgewählt unter: Mykotoxinen: Aflatoxine, Alternariol und Alternariol-monomethlyether, Cephalosporin, Chaetemin, Citrinin, Deoxynivalenol, Fumagilin, Fumonisine, Fusarin C, Fusarinsäure, Gilotoxin, Griseofulvin, Kojisäure, Moniliformin, Mutterkornalkaloide (Ergotalkaloide), Mykophenolsäure, Nivalenol, Ochratoxin A, Patulin, Penicillin, Penitrem A, Roquefortin, Satratoxin, Sterigmatocystin, Tenuazonsäure, Trichotecenen, (H)T-2-Toxin, Viomellein, Verrucosidin, Verruculogen, Xanthomegnin und Zearalenon; Algentoxinen, wie Domoinsäure, Ciguatoxine und Maitotoxine, Okadasäure und deren oben genannten Derivaten; Brevetoxinen; Saxitoxinen; und Palytoxinen.

Vorteilhaft sind die zuerst benannten Substanzen als anthropogene Marker bekannt oder als solche geeignet. Typischerweise sollen diese Substanzen durch Abwasserbehandlungsschritte völlig entfernt oder ihre Konzentration auf einen maximal zulässigen Wert reduziert werden, um Gefährdungen für den Menschen und/oder die Umwelt auszuschließen. Folglich ist die Kenntnis ihrer Anwesenheit und/oder Konzentration von unmittelbarer praktischer Bedeutung. Analog gilt dies für die oben nachfolgend benannten Toxine, die potentiell in den Agrarrohstoffen enthalten sein können und somit eine potentielle Gefahr für den Verbraucher, insbesondere bei deren Verarbeitung und beim Verzehr daraus gewonnener Produkte darstellen. Daher ist die Kenntnis über den Toxingehalt einer Agrarrohstoffprobe, bzw. die Gewissheit der Einhaltung vorgegebener Grenzwerte bedeutsam und somit vorteilhaft.

Gemäß einer Ausführungsform ist die erste der zumindest zwei unterschiedlichen funktionellen Gruppen eine NH₂-Gruppe und die zweite der zumindest zwei unterschiedlichen funktionellen Gruppen ist ein PEG-Rest, der 2 bis 20, bevorzugt 3 bis 15, weiter bevorzugt 6 bis 9 Ethylenglycol-Blöcke, also konstitutionelle Repetiereinheiten bzw. Wiederholeinheiten aufweist.

Vorteilhaft wird die für PEG bekannte "Ab schirm-Wirkung" gegenüber einer unspezifischen Proteinadsorption an der Partikeloberfläche unter Beibehaltung der spezifischen Wechselwirkung mit dem jeweiligen Bindungspartner (Analyt, bzw. gegen den Analyten gerichteten primären Antikörper (Antiserum) oder sekundärem Antikörper (Antiserum)) wirksam.

Gemäß einer Ausführungsform wird die NH₂-Gruppe durch Reaktion der Silikatschale mit 3-Aminopropyltriethoxysilan (APTES), und der PEG-Rest mit (3-[methoxy(polyethylenoxy)propyl]trimethoxysilan, beispielsweise umfassend sechs bis neun Ethylenglycol-Einheiten (PEG₆₋₉-PTMS) erzeugt. Bevorzugt erfolgt die Funktionalisierung der Silikatschale mit 3-Aminopropyltriethoxysilan (APTES) zur Verankerung von Aminogruppen für die Kopplung von Haptenen und mit Polyethylenglycol(3-[methoxy(polyethylenoxy)propyl]trimethoxysilane, 90%, 6-9 PEG Einheiten; PEG₆₋₉-PTMS).

Wie experimentell belegt werden konnte, sind gerade für die bezeichnete Kettenlänge des PEG die Abschirmwirkung gegenüber einer unerwünschten unspezifischen Wechselwirkung und die Beibehaltung der spezifischen Wechselwirkung der Partikel (der betreffenden Kohorte) mit den ihnen zugedachten Analyten gut ausgewogen.

Gemäß einer Ausführungsform werden DCF sowie ILA und OTA direkt, und CAF sowie CBZ über ihre Derivate 7-(5-carboxypentyl)-1,3-di-methylxanthin (CAF-(CH₂)₅-COOH) und N-(dibenz[b,f]azepin-5-yl-carbonyl)glycylglycylglycin(CBZ-Gly₃-COOH), jeweils durch Aktivierung ihrer Carbonsäuregruppen an die NH₂-Gruppe gekoppelt.

Vorteilhaft ermöglicht die bekannte DCC/NHS - Chemie eine einfache und unkomplizierte Anbindung der Analyten bzw. Antigene an die Partikeloberfläche.

Gemäß einer Ausführungsform ist der Luminophor ausgewählt unter: Azulen, Benzindol, Benzofuran, Benzoxadiazol, Benzoxazol, BODIPY, Chlorin, Coumarin, Cyanin, DAPI, Diphenylacetylen, Dipyrromethen, Fluorescein, Naphthalimid, Oxazin, Perylen, Phenazin, Phthalocyanin, Phycoerythrin, Polymethin, Porphyrin, einem porphyrinoiden Makrozyklus, Pyridin, Pyridinium, Pyrromethen, Pyrylium, Rhodamin, Rubyrin, Squaraine, Stilben, Styryl, Thiopyrylium und deren Derivaten.

Vorteilhaft bieten die bezeichneten Farbstoffe und ihre Derivate hinreichend große Möglichkeiten der parallelen optischen Kodierung unterschiedlicher Mikropartikel-Kohorten. Auf Grund ihrer großen Verbreitung gehören mittlerweile für kommerzielle Scannersysteme die erforderlichen optischen Filtersätze typischerweise bereits zur Standardausstattung.

Gemäß einer Ausführungsform weist der Polymerkern einen Luminophor und die Schale einen anderen Luminophor auf, wobei der erste Luminophor ausgewählt ist unter: Azulen, Benzindol, Benzofuran, Benzoxadiazol, Benzoxazol, BODIPY, Chlorin, Coumarin, DAPI, Diphenylacetylen, Dipyrromethen, Naphthalimid, Oxazin, Perylen, Phenazin, Phthalocyanin, Phycoerythrin, Porphyrin, Porphyrinoiden, Pyridin, Pyrromethen, Rubyrin, Squaraine, Stilben und deren Derivaten und der zweite Luminophor ausgewählt ist unter: Cyanin, Fluorescein, Polymethin, Pyridinium, Pyrylium, Rhodamin, Styryl, Thiopyrylium und deren Derivaten.

Vorteilhaft sind diese Substanzen und verfügbare Derivate hinreichend gut charakterisiert, kommerziell verfügbar und auch als reaktive Spezies zur kovalenten Verankerung verfügbar.

Gemäß einer Ausführungsform wird ein Verfahren zur simultanen Bestimmung verschiedener Analyte, z.B. verschiedener anthropogener Marker, in einer Umweltprobe oder Toxine in einer Agrarrohstoffprobe vorgeschlagen, das die folgenden Schritte umfasst:
(a) Bereitstellen einer Mischung von Kern/Schale-Mikropartikeln mit einem Polymerkern, der einen Luminophor und eine den Polymerkern umschließende Silikat-Schale aufweist, wobei die Mischung verschiedene Kohorten, bzw. Populationen von Kern/Schale-Mikropartikeln umfasst, die sich hinsichtlich einer Art und/oder einer Konzentration des Luminophors und/oder hinsichtlich eines mittleren Durchmessers und/oder hinsichtlich einer mittleren Dichte der Kern/Schale-Mikropartikel voneinander unterscheiden und so, beispielsweise an Hand optischer Messtechniken, eindeutig unterscheidbar sind, wobei die Silikat-Schale der Kern/Schale-Mikropartikel aller Kohorten Reste eines Polyethylenglycols und/oder eines Sulfobetains, und/oder ein Peptid umfassend eine Aminosäuresequenz, insbesondere die Aminosäuresequenz CYSYSYS oder die Aminosäuresequenz CRERERE, trägt, wobei die Silikat-Schale der Kern/Schale-Mikropartikel der verschiedenen Kohorten unterschiedliche, jeweils kovalent verankerte, anthropogene Marker oder Toxine oder die anthropogenen Marker oder Toxine zumindest teilweise repräsentierende Haptene trägt;
(b) in-Kontakt-Bringen der Mischung von Kern/Schale-Mikropartikeln mit einem wässrigen Aliquot oder einem wässrigen oder organischen Auszug der Umweltprobe oder Agrarrohstoffprobe und Erhalten einer ersten wässrigen Suspension der Kern/Schale-Mikropartikel;
(c) Zusetzen zumindest eines primären Antikörpers (ob nun als monoklonaler Antikörper oder in Form eines polyklonalen Antiserums), aufweisend jeweils eine Selektivität gegenüber den anthropogenen Markern oder gegenüber den anthropogene Marker repräsentierenden Haptenen zu der ersten wässrigen Suspension und Erhalten einer zweiten wässrigen Suspension der Kern/Schale-Mikropartikel;
(d) Zusetzen von sekundären Antikörpern und/oder Antiseren, die jeweils eine Selektivität gegenüber den primären Antikörpern oder Antiseren aufweisen und eine Markierung tragen, zu der zweiten wässrigen Suspension und Erhalten einer dritten Suspension der Kern/Schale-Mikropartikel;
   Anmerkung: An Stelle des sekundären Antikörpers bzw. Antiserums können auch andere - antikörperfreie - Markierungsmethoden, z.B. entweder chemische (Click-Chemie o.ä.) oder andere Bindungsproteine (Biotin/(Strept-)Avidin-System, Protein A, Protein G usw.) eingesetzt werden.
(e) Zusetzen eines Fixierungs- und Quervernetzungsmittels zu der dritten Suspension der Kern/Schale-Mikropartikel, sodass am primären Antikörper gebundene(r) sekundäre(r) Antikörper - ob nun monoklonal oder aus einem Antiserum stammend - mit dem primären Antikörper vernetzt und in der dritten Suspension vorliegende, noch ungebundene sekundäre Antikörper denaturiert werden;
(f) Auslesen einer Kombination von optischen, bspw. fluoreszenzoptischen, Charakteristika der Kern/Schale-Mikropartikel in der dritten wässrigen Suspension;
   Anmerkung: Hierbei umfasst die Kombination optischer, bspw. fluoreszenzoptischer, Charakteristika optisch erfassbare Messgrößen der Kerne der Kern/Schale- Mikropartikel und/oder der Markierung (Label) sekundärer Antikörper, welche durch die Reaktion mit dem Fixierungs- und Quervernetzungsmittel, z.B. Formaldehyd, auf den Kern/Schale-Mikropartikeln denaturiert wurden.
(g) Zuordnen ausgelesener Kombinationen optischer, bspw. fluoreszenzoptischer, Charakteristika zu einer Konzentration der Analyten, z.B. verschiedener anthropogener Marker, in der Umweltprobe oder Toxine in einem Agrarrohstoff. Das erfolgt typischerweise an Hand von Kalibrierkurven.

Mit dem vorgeschlagenen Verfahren können, in Abhängigkeit von der Anzahl eingesetzter Kohorten, mehrere Analyte simultan in einer Probe mit einem Ansatz erfasst werden. Die Kern/Schale-Mikropartikel der unterschiedlichen Kohorten vermischen sich während der beschriebenen Verfahrensschritte problemlos miteinander, sodass für alle Mikropartikeln, respektive alle Analyten identische Reaktionsbedingungen vorliegen.

Damit umfassen die Vorteile des vorstehend beschriebenen Verfahrens: (1) Multiplex-Fähigkeit bei (2) Mix-and-Read (3) ohne Waschschritte auf Grund von (4) anti-fouling-Oberfläche und (5) Fixierung und Quervernetzung (bspw. mit Formaldehyd); (6) Verwendung vergleichsweise kleiner Partikel und damit (7) schnellerer Kinetik; (8) höher auflösende Größenkodierungen (im Vergleich zu vorbekannten Assays, die einfache, nicht von einer Schale umgebene Partikel verwenden); (9) mehr Partikel pro Volumen bzw. Gewichteinheit und damit mehr Assays für gleichen Materialeinsatz/Materialkosten bzw. ökologischer Vorteil; (10) Verwendung von Kern/Schale-Partikeln und damit Möglichkeit der Einstellung der Dichte [Anmerkung: mit reinen Polystyrol-Partikeln von 1 µm Durchmesser kann man nicht vernünftig arbeiten, da diese auf wässrigen Lösungen "floaten" / aufschwimmen]; (11) direkte Kopplung von Hapten auf Partikeloberfläche ohne zusätzliche Linker; was (12) eine vereinfachte, d.h. kostengünstigere Partikelpräparation gestattet; (13) Vorteile von Fluoreszenzimmunoassays: insbesondere (14) keine Inhibition von Enzyme-Labeln (Störeffekt) möglich, da keine Enzyme verwendet werden; (15) kontrollierbare, definierte Labeldichte; (16) Vorteile einer kompetitiven Signalerzeugung: insbesondere (17) selektive Erkennung ohne Auftrennung der Probe, (18) keine Probenanreicherung erforderlich; und (19) nur ein analytselektiver Antikörper wird benötigt (statt zweien wie bei Sandwich-Assays).

Gemäß einer Ausführungsform sind die anthropogenen Marker ausgewählt unter: Carbamazepin (CBZ), Benzotriazol (und Methyl-Benzotriazolderivate), Mecoprop, Coffein (CAF), Coprostanol, Cholesterol, Diclofenac (DCF), Isolithocholsäure (ILA), Kreatinin, Cotinin, Atenolol, Bisoprolol, Celiprolol, Clofibrinsäure, Diazepam, Dihydrocodein, Doxepin, Estron, Estradiol, Ethinylöstradiol, Galaxolid, Ibuprofen, Iopromid, Methadon, Metoprolol, Morphin, Norfloxacin, Oxazepam, einem Östradiol, Primidon, Propranolol, Roxithromycin, Sulfonamid-Antiinfektiva, Penicillin- und Penicillin-analoge Antibiotika, Tetracyclin, Gentamicin, Streptomycin, Sotalol und Tonalid, Kokain, Benzoylecgonin, THC, MDMA, Glyphosat, Atrazin, Metolachlor, Carbofuran, Carbaryl, Imidacloprid, Fenitrothion, Chlorpyrifos-methyl, Triazophos, Chloramphenicol, Clenbuterol, Tylosin, Phthalate, Nicht-Phthalat Weichmachern, einer poly- oder einer perfluorierten Alkylsubstanz (PFAS), insbesondere einer Perfluoroktansulfonsäure (PFOS); und einem Bisphenol.

Die bezeichneten Substanzen (anthropogenen Marker) sind typischerweise unfehlbare Indikatoren unterschiedlicher zivilisatorischer Aktivität. Deshalb kann das Monitoring der Konzentration(en) einer Kombination verschiedener Marker einen verlässlichen Hinweis auf die Anwendung eines Abwasserbehandlungsverfahrens bzw. über dessen Effektivität geben oder die nicht-statthafte Einleitung eines unbehandelten kommunalen Abwassers in ein Oberflächengewässer belegen. Analog sind die oben genannten Toxine unfehlbare Indikatoren für einen mikrobiellen Befall von Agrarrohstoffen durch z. B. Schimmelpilze und/oder Cyanobakterien und oder Algen wie Dinoflagellaten.

Gemäß einer Ausführungsform sind die Toxine ausgewählt unter: den Mykotoxinen: Aflatoxine, Alternariol und dessen Monomethlyether, Cephalosporin, Chaetemin, Citrinin, Deoxynivalenol, Fumagilin, Fumonisine, Fusarin C, Fusarinsäure, Gilotoxin, Griseofulvin, Kojisäure, Moniliformin, Mutterkornalkaloide (Ergotalkaloide), Mykophenolsäure, Nivalenol, Ochratoxin A, Patulin, Penicillin, Penitrem A, Roquefortin, Satratoxin, Sterigmatocystin, Tenuazonsäure, Trichotecenen, (H)T-2-Toxin, Viomellein, Verrucosidin, Verruculogen, Xanthomegnin und Zearalenon; Algentoxinen, wie beispielsweise Domoinsäure; Ciguatoxinen und Maitotoxinen; Okadasäure und deren Derivaten;, Brevetoxinen; Saxitoxinen und Palytoxinen.

Die Kenntnis des Gehalts der bezeichneten Toxine in Agrarrohstoffproben ist auf Grund der gesundheitsschädlichen Wirkungen der Substanzen im menschlichen und tierischen Organismus bedeutsam. Insbesondere ist die Einhaltung entsprechender gesetzlich vorgegebener Grenzwerte zu überwachen. So bietet die gemäß einer erfindungsgemäßen Ausführungsform durchgeführte simultane Bestimmung der Toxine Aflatoxin B1, Ochratoxin A, T2/HT2-Toxin, Deoxynivalenol, Zearalenon und Fumonisin B1 eine erhebliche Zeit- und Kostenersparnis gegenüber einem Nachweis der genannten Toxine in gesondert voneinander durchgeführten Analysen. Die bezeichnete Kombination der Toxine Aflatoxin B1, Ochratoxin A, T2/HT2-Toxin, Deoxynivalenol, Zearalenon und Fumonisin B1 kann potentiell im Agrarrohstoff Getreide auftreten. Die Vermeidung einer Kontamination mit den bezeichneten Ochratoxin A, Deoxynivalenol, Zearalenon, Fumonisin B1 und B2 sowie T-2- und HT-2-Toxin ist Gegenstand des gemäß EG-Verordnung Nr. 1881/2006 durchzuführenden Monitoring.

Gemäß einer Ausführungsform umfasst die bereitgestellte Mischung von Kern/Schale-Mikropartikeln unterschiedliche Kohorten von Kern/Schale-Mikropartikeln, wobei die unterschiedlichen Kohorten der Kern/Schale-Mikropartikel jeweils kovalent verankertes DCF, ILA, CAF oder CBZ als anthropogene Marker umfassen.

Gemäß einer Ausführungsform umfasst die bereitgestellte Mischung von Kern/Schale-Mikropartikeln Kohorten von Kern/Schale-Mikropartikeln, die jeweils eines von Aflatoxin B1, Ochratoxin A, T-2-Toxin, HT-2-Toxin, Deoxynivalenol, Zearalenon und Fumonisin B1 oder ein die entsprechende Substanz zumindest teilweise repräsentierendes Hapten kovalent verankert tragen.

Vorteilhaft erlaubt es ein Gemisch der unterschiedlichen Kern/Schale-Mikropartikel (d.h. der vier Kohorten), alle vier anthropogenen Marker bzw. alle sieben Toxine simultan in einer untersuchten Probe zu bestimmen.

Gemäß einer Ausführungsform trägt die Silikat-Schale der Kern/Schale-Mikropartikel aller Kohorten Reste eines Polyethylenglycols, wobei das Polyethylenglycol typischerweise 6 bis 9 Ethylenglycol-Einheiten umfasst. Derartige Reste lassen sich, beispielsweise, durch kovalente Bindung von PEG₆₋₉-PTMS an die Silikatschale binden.

Vorteilhaft vermindert eine PEG-Dekoration der Partikeloberfläche die unspezifische Bindung von Antikörpern und verringert somit das Auftreten falsch-positiver Befunde. Mithin wird sowohl die Selektivität als auch die Sensitivität (Verbesserung des Signal/Rausch-Verhältnisses) des partikelbasierten Assays verbessert.

Gemäß einer Ausführungsform wird ein Kit zur simultanen Bestimmung verschiedener Analyte in einer Probe, beispielsweise einer Umweltprobe und/oder in einer Agrarrohstoffprobe, vorgeschlagen. Das Kit umfasst:
- eine Spritze, beispielsweise eine Plastik-Einmalspritze oder Refill-Spritze, zur Aufnahme einer flüssigen Umweltprobe oder zur Aufnahme eines Extrakts der Agrarrohstoffprobe;
- ein Spritzenvorsatzfilter zur Filtration der flüssigen Umweltprobe oder des Extrakts der Agrarrohstoffprobe;
- zumindest zwei Kohorten von Kern/Schale-Mikropartikeln mit unterschiedlichen, jeweils kovalent verankerten anthropogenen Markern oder jeweils kovalent verankerten Toxinen oder die anthropogenen Marker oder die Toxine zumindest teilweise repräsentierenden Haptenen, wobei bevorzugt jede Kohorte weiterhin kovalent gebundene PEG-Reste, typischerweise umfassend 6-9 Ethylenglycol-Einheiten aufweist;
- ein Behältnis zum Mischen der zumindest zwei Kohorten mit der flüssigen Umweltprobe bzw. mit dem Extrakt der Agrarrohstoffprobe; und
- eine Lösung, umfassend ein Fixierungs- und Quervernetzungsmittel.

Hierbei versteht es sich von selbst, dass sowohl im beschriebenen Herstellungsverfahren, als auch im beschriebenen Bestimmungsverfahren sowie auch beim beschriebenen Kit und dessen Verwendung die zumindest zwei Kohorten Kern/Schale-Mikropartikel unabhängig voneinander jeweils solche Kern/Schale-Mikropartikel umfassen, die:
- kovalent verankerte anthropogene Marker tragen, oder
- kovalent verankerte Haptene tragen, wobei die kovalent verankerten Haptene jeweils anthropogene Marker repräsentieren oder zumindest teilweise repräsentieren, oder
- kovalent verankerte Toxine tragen, oder
- kovalent verankerte Haptene tragen, wobei die kovalent verankerten Haptene jeweils Toxine repräsentieren oder zumindest teilweise repräsentieren.

Typischerweise werden die zumindest zwei Kohorten von Kern/Schale-Mikropartikeln des Kits, also Kern/Schale-Mikropartikel mit unterschiedlichen, jeweils kovalent verankerten anthropogenen Markern oder Toxinen oder die anthropogenen Marker oder Toxine zumindest teilweise repräsentierenden Haptenen, gemäß dem eingangs beschriebenen Herstellungsverfahren bereitgestellt, in einem erfindungsgemäßen Bestimmungsverfahren eingesetzt und/oder zur Komplettierung eines erfindungsgemäßen Kits verwendet.

Damit ist der beschriebene Kit angepasst zur Durchführung des vorstehend beschriebenen Verfahrens zur simultanen Bestimmung unterschiedlicher Analyte, insbesondere zur Bestimmung unterschiedlicher anthropogener Marker in einer Umweltprobe und/oder Toxine in einer Agrarrohstoffprobe. Die Agrarrohstoffprobe wird typischerweise vor der Bestimmung der Toxine und/oder Pestizide mit einem wässrigen oder organischen oder Lösungsmittel oder Lösungsmittelgemisch extrahiert. Im Falle von Umweltproben ist das Erfordernis einer Extraktion abhängig von der Art der Probenmatrix und/oder der Assoziation des anthropogenen Markers mit derselben.

Während der Durchführung des Bestimmungsverfahrens bzw. bei Verwendung des Kits liegen die zumindest zwei Kohorten typischerweise als homogene Mischung vor. Der Kit selbst kann zweckmäßigerweise voneinander getrennt vorliegende unterschiedliche Kohorten umfassen, sodass erst unmittelbar vor Ort, je nach spezifischer Messsituation bzw. in Abhängigkeit von interessierenden oder erwarteten Analyten entschieden werden kann, welche Kohorten simultan im Bestimmungsverfahren bei Analyse der Umweltprobe oder des Extraktes der Agrarrohstoffprobe eingesetzt werden.

Optional kann das Kit noch einen Behälter mit einem wässrigen Puffer (Phosphatpuffer, Borat-Puffer, Tris/Tris·HCl - Puffer, Acetat-Puffer, HEPES-Puffer, oder ein anderes auf einen physiologischen pH-Wert einstellbares Puffersystem) umfassen und/oder eine Flasche mit destilliertem Wasser. Vorteilhaft umfasst das Kit damit alle notwendigen Utensilien, um auch unter widrigsten Feldbedingungen einen Assay durchführen zu können. Mobile tragbare Messgeräte, selbst mikrofluidische Systeme, die in Kombination mit einer Anwendungssoftware und einem Smartphone betreibbar sind, können für das automatisierte Auslesen der Analyseergebnisse genutzt bzw. entsprechend angepasst werden.

Gemäß einer Ausführungsform umfasst das Kit weiter eine Festphasen-Extraktionseinheit oder Adsorber-Kartusche zur Extraktion der flüssigen Umweltprobe und/oder einen Schütteltrichter bzw. einen Behälter zur Extraktion einer festen Umweltprobe, z.B. einer Bodenprobe, oder der jeweiligen Agrarrohstoffprobe mit einem flüssigen Extraktionsmittel (z.B. einer wässrigen Lösung oder einem organischen Lösungsmittel oder mit einem Gemisch aus solchen.). Die Extraktionseinheit bzw. die Adsorber-Kartusche ist angepasst zur selektiven Adsorption eines bestimmten Analyten. Sie kann, beispielsweise, ein Ionenaustauscher-Harz oder eine in Hinsicht auf ihre Polarität angepasste Silikagel-Packung (Umkehrphase, z.B. C18) o.ä. enthalten. Ebenso kann das Kit eine Palette verschiedener Kartuschen umfassen. Das verbessert die Anwendbarkeit des vorgeschlagenen BestimmungsVerfahrens bzw. des Kits und macht diese - bei Vorliegen eines entsprechenden Messgerätes - weitestgehend unabhängig von einem Labor. Betreffende Messgeräte sind dem Fachmann bekannt, beispielsweise tragbare, batteriebetriebene und LED basierende Reader.

Gemäß einer Ausführungsform sind die mit Hilfe des Kits bestimmbaren anthropogenen Marker ausgewählt unter: Carbamazepin (CBZ), Benzotriazol (und Methyl-Benzotriazolderivate), Mecoprop, Coffein (CAF), Coprostanol, Cholesterol, Diclofenac (DCF), Isolithocholsäure (ILA), Kreatinin, Cotinin, Atenolol, Bisoprolol, Celiprolol, Clofibrinsäure, Diazepam, Dihydrocodein, Doxepin, Estron, Estradiol, Ethinylöstradiol, Galaxolid, Ibuprofen, Iopromid, Methadon, Metoprolol, Morphin, Norfloxacin, Oxazepam, einem Östradiol, Primidon, Propranolol, Roxithromycin, Sulfonamid-Antiinfektiva, Penicillin- und Penicillin-analoge Antibiotika, Tetracyclin, Gentamicin, Streptomycin, Sotalol und Tonalid, Kokain, Benzoylecgonin, THC, MDMA, Glyphosat, Atrazin, Metolachlor, Carbofuran, Carbaryl, Imidacloprid, Fenitrothion, Chlorpyrifos-methyl, Triazophos, Chloramphenicol, Clenbuterol, Tylosin, Phthalate, Nicht-Phthalat Weichmacher, eine polyoder einer perfluorierten Alkylsubstanz (PFAS), insbesondere Perfluoroktansulfonsäure (PFOS); und einem Bisphenol. Weiterhin kommen die bereits oben benannten Verbindungen wie diverse Pestizide, Futtermittelzusatzstoffe sowie materialbürtige Stoffe als Analyte (mit dem entsprechend angepassten Kit nachweisbare Marker) in Betracht.

Gemäß einer Ausführungsform sind die mithilfe des Kits bestimmbaren Toxine ausgewählt unter den Mykotoxinen: Aflatoxine, Alternariol und Alternariol-monomethlyether, Cephalosporin, Chaetemin, Citrinin, Deoxynivalenol, Fumagilin, Fumonisine, Fusarin C, Fusarinsäure, Gilotoxin, Griseofulvin, Kojisäure, Moniliformin, Mutterkornalkaloide (Ergotalkaloide), Mykophenolsäure, Nivalenol, Ochratoxin A, Patulin, Penicillin, Penitrem A, Roquefortin, Satratoxin, Sterigmatocystin, Tenuazonsäure, Trichotecenen, (H)T-2-Toxin, Viomellein, Verrucosidin, Verruculogen, Xanthomegnin und Zearalenon; unter Algentoxinen, wie Domoinsäure; Ciguatoxinen und Maitotoxinen; Okadasäure und deren Derivaten; Brevetoxinen, Saxitoxinen und Palytoxinen.

Die Vorteile der Nachweisbarkeit der angeführten Substanzen mit Hilfe des bezeichneten Kits entsprechen den zuvor genannten. Selbstredend kann ein entsprechendes Kit auch zum simultanen Nachweis anderer Analyte konfiguriert werden. Entsprechende Mikropartikel-Kohorten müssen lediglich / z.B. im Falle des hier beschriebenen kompetitiven Assay-Formats / die jeweiligen Antigene tragende Kern/Schale-Mikropartikel umfassen und die für deren Nachweis spezifischen Antikörper (Antiseren) müssen vorliegen.

Gemäß einer Ausführungsform ist das Kit angepasst zum Nachweis der anthropogenen Marker DCF, ILA, CAF, und/oder CBZ.

DCF, ILA, CAF, und CBZ sind als anthropogene Marker generell anerkannt. Allein ihr Nachweis gestattet es, einen fundierten Befund über das Vorhandensein/ ggf. die unstatthafte Einleitung eines Siedlungsabwassers zu erheben/festzustellen.

Gemäß einer Ausführungsform ist das Kit angepasst zum simultanen Nachweis der Toxine Ochratoxin A, Deoxynivalenol, Zearalenon, Fumonisin B1 und B2 sowie T-2- und HT-2-Toxin in einer Agrarrohstoffprobe und somit besonders zum Monitoring praxisrelevanter Toxine in unverarbeitetem Getreide geeignet.

Gemäß einer Ausführungsform wird die Verwendung des oben beschriebenen Verfahrens und/oder des oben beschriebenen Kits zur Überwachung eines Grenzwertes und oder zum Nachweis einer Einleitung eines Abwassers oder Klärschlamms, ober einer Abschwemmung eines behandelten Bodenanteils, in ein Oberflächengewässer vorgeschlagen. Für die bezeichnete Abschwemmung kommen hier insbesondere pestizidbehandelte Bodenanteile, also Bodenanteile landwirtschaftlicher Nutzflächen in Betracht. Ebenso wird die Verwendung des oben beschriebenen Verfahrens und/oder des oben beschriebenen Kits zur Überwachung des Vorkommens von Toxinen in Agrarrohstoffen oder in Trinkwasser vorgeschlagen. Die vorstehend beschriebenen Ausführungsformen können beliebig miteinander kombiniert werden. Die Erfindung ist jedoch nicht auf die konkret beschriebenen Ausführungsformen beschränkt, sondern kann durch die Kombination einzelner Merkmale und ausgewählter Merkmalskombinationen einer Ausführungsform mit Merkmalen und Merkmalskombinationen einer anderen Ausführungsform modifiziert werden, um zu weiteren erfindungsgemäßen Ausführungsformen zu gelangen.

Bevor nachfolgend die Ausführungsbeispiele anhand der Figuren näher erläutert werden, wird daraufhingewiesen, dass die Figuren nicht notwendigerweise maßstabsgerecht sind, der Schwerpunkt liegt vielmehr auf der Erläuterung des Grundprinzips der Erfindung.

### Kurzdarstellung der Figuren

**Figur** 1 zeigt in schematischer Weise ein Kern/Schale-Mikropartikel
**Figur 2** zeigt das Prinzip des hier vorgeschlagenen Verfahrens zur simultanen Bestimmung verschiedener Analyte in einer Umweltprobe / bzw. des vorgeschlagenen Immunoassays.
**Figur 3** ist eine alternative Darstellung des in Fig. 2 gezeigten Verfahrens
**Figur 4** ist eine weitere alternative Darstellung des in Fig. 2 und Fig. 3 gezeigten Verfahrens.
**Figur 5** illustriert die Herstellung hier genutzter Kern/Schale-Mikropartikel mit gefärbtem Polystyrolkern und Siliziumdioxidschale.
**Figur 6** illustriert schematisch die Hapten- bzw. Antigen-Dekoration farbkodierter Kern/Schale-Mikropartikel.
**Figur 7** zeigt Ergebnisse zum Spezifizitäts-Test des Mikropartikelassays.
**Figur** 8 zeigt Wiederfindungsraten sowie intra- und inter-Assay-Variationskoeffizienten für den simultanen Nachweis von CBZ, CAF, DCF und ILA in 5 "gespikten" Wasserproben.
**Figur** 9 zeigt den Signalverlauf während des zytometrischen Auslesens des Partikelstromes ohne (A) und mit (B) Zusatz von Fixationslösung.
**Figur 10** zeigt den Verlauf der Bindung eines FITC-markierten anti-CAF-Antikörpers an CAF-funktionalisierte Mikropartikel.
**Figur 11** illustriert den Einfluss der Fließgeschwindigkeit auf die Anzahl erfasster Partikel (linke Ordinate, Quadrate) und den Partikelanteil (rechte Ordinate, Dreiecke) im Gate "Size" als Funktion der verwendeten Partikelkonzentration.
**Figur 12** zeigt Kalibrierkurven für die anthropogenen Marker CBZ, CAF, DCF und ILA und die beim Kurvenfitting ermittelten Parameter.
**Figur 13** zeigt Kalibrierkurven für die Toxine Ochratoxin A (OTA) und Zearalenon (ZON), sowie die beim Kurvenfitting ermittelten Parameter.
**Figur 14** illustriert schematisch die Hapten- bzw. Antigen-Dekoration farbkodierter Kern/Schale-Mikropartikel mit einem ZON-Hapten-Derivat und OTA.
**Figur 15****.** zeigt Ergebnisse zum Spezifizitäts-Test eines erfindungsgemäßen Mikropartikelassays mit ZON, OTA und CAF.

### Detaillierte Beschreibung

Wie eingangs erläutert, ist die simultane Bestimmung der jeweiligen Analyten gemäß dem hier vorgeschlagenen SAT-Verfahren auf optisch kodierte Kern/Schale-Mikropartikel gestützt. Typischerweise handelt es sich dabei um lumineszierend markierte Polymerkerne, beispielsweise um monodispers vorliegende, ideal sphärische Polystyrol-Mikrokugeln. Der hier bevorzugte mittlere arithmetische Partikeldurchmesser der Kern/Schale Mikropartikel liegt in einem Größenbereich von 200 nm bis 7 µm, insbesondere im Bereich von 500 nm bis 5 µm, besonders bevorzugt im Bereich von 700 nm bis 2 µm. Bei kleinen Partikeln ist (a) die Kinetik schneller, (b) können höher auflösende Größenkodierungen erreicht werden, (c) stehen mehr Partikel pro Volumen bzw. Gewichteinheit zur Verfügung (ökologischer Vorteil). Zudem erreicht man erst durch Schalenaufwachsen bei kleinen Partikeln eine Dichte oberhalb jener wässriger Medien, so dass die Kern/Schale-Partikel im Vergleich zu reinen Polystyrol-Partikeln gleicher Größe nicht mehr floaten. Die Polymerpartikel werden nach bekannten Verfahren der Dispersionspolymerisation erhalten. Die jeweilige Lumineszenzmarkierung der Polymerkerne erfolgt, beispielsweise, mittels Diffusion des gelösten Luminophors in die in einem Gemisch eines organischen Lösungsmittels mit Wasser gequollenen, aber nicht gelösten Kerne (Beispielsweise ein Gemisch aus 1 Teil Tetrahydrofuran und 10 Teilen Wasser). Nach entsprechenden Waschschritten mit Wasser ist der eingesetzte Luminophor typischerweise homogen im Polymerkern verteilt. Die Umhüllung der Polymerkerne erfolgt mit einer Silikatschale, die in Anlehnung an die bekannte Stöber-Technik, beispielsweise in Ethanol: Wasser (50:1 v/v) mit Tetraethoxysilan (TEOS) als Precursor und Ammoniumhydroxid oder APTES als Katalysator erzeugt wird. Typischerweise, aber nicht ausschließlich, wird die Silikatschale in Gegenwart von Polyvinylpyrrolidon (PVP) ausgebildet. Das mittlere Molekulargewicht des dabei verwendeten PVP ist darauf angepasst, eine gewünschte Oberflächenrauheit der Silikatschale zu erzielen. Ein bevorzugtes mittleres Molekulargewicht beträgt beispielsweise 40 kD (PVP 40). Die entsprechenden Polymere sind typischerweise nicht monodispers, der angegebene Wert stellt einen Mittelwert dar. Bevorzugte Bereiche der mittleren arithmetischen Partikeldurchmesser liegen im Bereich 100 - 8000 nm, insbesondere im Bereich 200 - 6000 nm, besonders bevorzugt im Bereich 500 - 2500 nm. Die Messung mittels modernen Streulicht-Messgeräten gestattet eine zuverlässige Ermittlung der mittleren arithmetischen Partikeldurchmesser sogar unterhalb 100 nm. Im praktischen Test waren sowohl Kern/Schale-Mikropartikel mit 500 nm mittlerem arithmetischen Durchmesser, als auch Kern/Schale-Mikropartikel mit 2500 nm mittlerem arithmetischen Durchmesser geeignet für das beschriebene Analyseverfahren. Insbesondere die kleineren Beads (Kern/Schale-Mikropartikel) bieten die hierin benannten Vorteile.

Typischerweise, aber nicht ausschließlich, lässt sich über die Größe der Polymerkerne die Größe der Kern/Schale-Mikropartikel einstellen. Typischerweise, aber nicht ausschließlich, lässt sich über die Dicke der Silikatschale im Verhältnis zum Durchmesser der Polymerkerne eine mittlere Dichte der Kern/Schale-Mikropartikel einstellen. Kleinere Silikatpartikel, zugänglich z.B. über Aminosäure-katalysierte Stöber-Synthese, können auch als Schale verwendet werden. Diese lassen sich z.B. mit Re-Growth-Methoden auf Schichtdicken von bis zu 200 nm anwachsen. Solche Silikatpartikel können auch sterisch mit Farbstoff gedopt werden. Typischerweise, aber nicht ausschließlich, lässt sich über die benannten Parameter eine optisch messbare mittlere (winkelabhängige) Streulichtintensität der Kern/Schale-Mikropartikel einstellen. Unter Zuhilfenahme bekannter Messverfahren sind einzelne Populationen (Kohorten) von Kern/Schale-Mikropartikeln allein auf der Grundlage der genannten optisch messbaren Parameter zuverlässig voneinander unterscheidbar. Über die Auswahl der Luminophore hinsichtlich Anregungswellenlängen (Absorptionsspektrum), Emissionswellenlängenmaximum (Emissionsspektrum), Fluoreszenzintensität und/oder Fluoreszenzabklingzeit lassen sich einzelne Populationen (Kohorten) von Kern/Schale-Mikropartikeln ebenso zuverlässig voneinander unterscheiden. Eine Kombination entsprechender Messverfahren, die beispielsweise durch bekannte optisch aktivierte Zell/Partikel-Sortiervorrichtungen, wie z.B. FACS oder mikrofluidische Imaging Systeme ohne Hüllstrom (Abfrage per Laserscan) erfolgen, gestatten es, komplexe Mischungen von entsprechend kodierten Partikeln zu analysieren. Auf diesem Prinzip beruhen SAT-Verfahren (SAT = suspension array technology).

In der nachfolgenden Tabelle 1 sind einige für das hier beschriebene SAT-Verfahren geeignete Luminophore angeführt.

**Tabelle 1**

| Klasse #1 | Klasse #2 |
|---|---|
| Cyanin und Polymethin Derivate | Azulen und Derivate |
| Fluorescein und Derivate | Benzindol und Derivate |
| Pyridinium Derivate | Benzofuran und Derivate |
| Pyrylium und Thiopyrylium Derivate | Benzoxadiazol und Derivate |
| Rhodamin und Derivate | Benzoxazol und Derivate |
| Styryl Derivate | Chlorin und Derivate |
| | Coumarin und Derivate |
| | DAPI und Derivate |
| | Diphenylacetylen und Derivate |
| | Dipyrromethen oder BODIPY und Derivate |
| | Naphthalimid und Derivate |
| | Oxazin und Derivate |
| | Perylen und Derivate |
| | Phenazin und Derivate |
| | Phthalocyanin und Derivate |
| | Phycoerythrin und Derivate |
| | Porphyrin und Derivate (Porphyrinoide) |
| | porphyrinoide Makrocyclen |
| | Pyridin Derivate |
| | Pyrromethen und Derivate |
| | Rubyrin und Derivate (Porphyrinoide) |
| | Squaraine und Derivate |
| | Stilben und Derivate |

Die in Klasse #2 genannten Porphyrinoide, vor allem wasserlösliche Corrole, Porphyrine, Rubyrine, Sapphyrrine und Hexaphyrine sind für das vorliegende Assayformat ebenso geeignet. Beispielsweise kann das wasserlösliche Rubyrin ([26]hexaphyrin(l. 1.0.1.1.0)) und dessen Derivate als NIR-Farbstoff sowohl zur Markierung des Kerns, als auch der Schale der beschriebenen Kern/Schale-Mikropartikel verwendet werden. Diese Farbstoffe lassen sich u.a. auch wegen ihrer großen Stokes'schen Verschiebung gut mit herkömmlichen Lumineszenzfarbstoffen kombinieren und kommen deshalb für die Markierung der beschriebenen Kern/Schale-Mikropartikel in Betracht. Selbst wenn nicht alle in Klasse #2 angeführten Verbindungen eine große Stokes'sche Verschiebung aufweisen, wie z.B. Rubyrin, so weist Rubyrin und alle anderen Porphyrionoide eine breitbandige Absorption auf, so dass die Wellenlängenbereiche (optische Fenster), die für Anregung und Emission gewählt werden können, hinreichend weit voneinander getrennt sind, um einen optischen Cross-Talk zu vermeiden.

Zum homogenen Färben des Polymerkerns mit nur einem einzigen Luminophor können bevorzugt die Verbindungen von Klasse #2 verwendet werden. Die ionischen Farbstoffe der Klasse #1 dringen vermutlich nur mit betont lipophilen Gegenionen in das unpolare Partikel. Soll hingegen, eine Doppelmarkierung der Partikel vorgenommen werden, so werden zum Färben des Polymerkerns und der Silikat-Schale sinnvollerweise die Luminophore jeweils der anderen Klasse oder unterschiedliche Derivate verwendet. Die Luminophore der Klasse #1 lassen sich bedeutend besser in SiO₂-Partikel einbauen, wobei hier wiederum die kationischen deutlich besser als die anionischen Luminophore geeignet sind, da Silikat-Oberflächen in der Regel netto negativ geladen sind. Selbstredend werden die zur Markierung der sekundären Antikörper eingesetzten Fluorophore und die zur Kodierung der Kern/Schale-Mikropartikel eingesetzten Fluorophore, insbesondere hinsichtlich ihrer Emissionswellenlängen, aneinander angepasst.

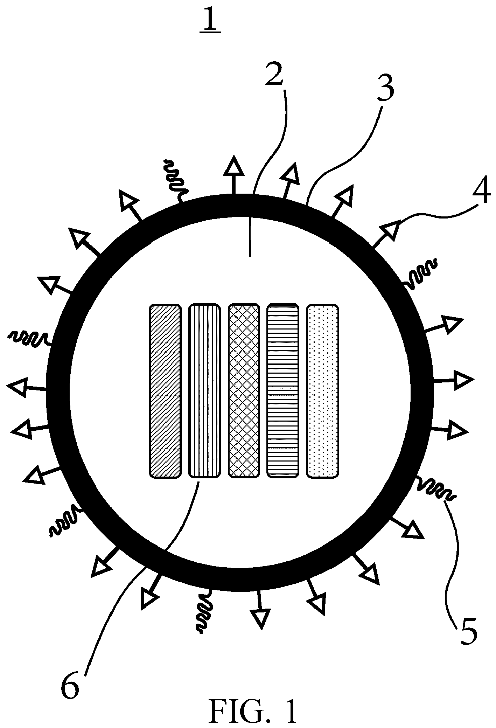

**Fig. 1** zeigt schematisch ein Kern/Schale-Mikropartikel, das die Grundlage des erfindungsgemäß vorgeschlagenen SAT-Assays bietet.

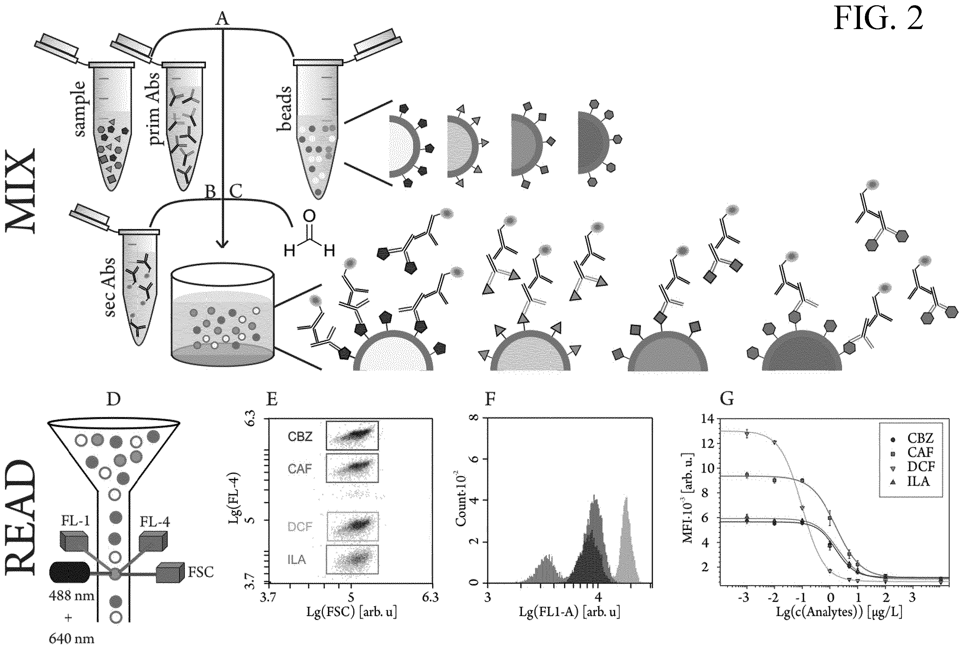

**Fig.2** stellt schematisch das Prinzip des vorgeschlagenen Immunoassay-Formats dar: A) Kompetitive Bindung der primären Antikörper; B) Färben der partikelgebundenen primären Antikörpermit fluorophor-markierten sekundären Antikörpern; C) Stoppen der Reaktion und Vernetzen der Antigen-Antikörper-Komplexe; D) Zytometrisches Auslesen. Die Prozedur des Messwert-Behandlung ist schematisch in den Teilbildern E-G gezeigt:
E) Prä-Gating (Filtern) an der optischen Messvorrichtung / "Scanner" (Vorwärtsstreusignal / Seitwärtsstreusignal, bzw. FSC/SSC) zum Ausschluss von Partikel-Aggregaten;
F) Decodieren in betreffende Code-bezogene Fluoreszenzkanäle durch das Festlegen von vier gates / hier gezeigt für »dye 2 red» - kodierte Mikropartikel auf Kanal FSC/FL-4;
G) Resultierendes Histogram der auf den sekundären Antikörper bezogenen Fluoreszenz für jeder individuelle Mikropartikel-Population (Kohorte), hier gezeigt für Alexa Fluor™488 Fluoreszenz in FL-1. Die mittlere Fluoreszenz Intensität wird aus diesem Plot berechnet.

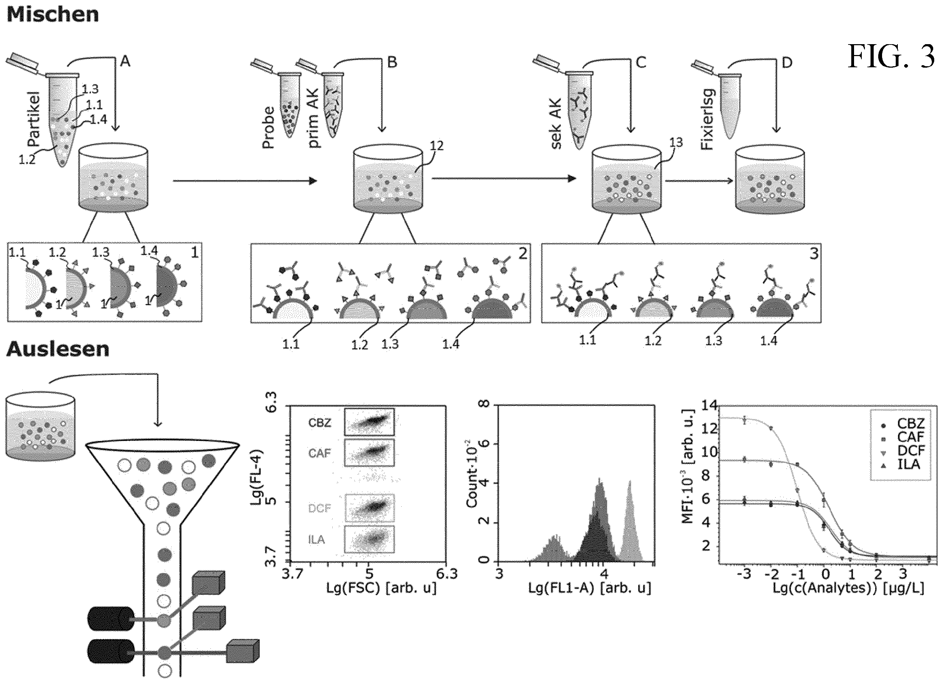

**Fig.** 3 verdeutlicht im oberen Bildteil die Abfolge von Schritten des Mischens der Kern/Schale Mikropartikel von vier verschiedenen Kohorten 1.1, 1.2, 1.3, und 1.4 (A) mit einer Probe unbekannter Zusammensetzung (z.B.wässriger Aliquot einer Umweltprobe) und verschiedenen primären (monoklonalen) Antikörpern / prim AK /, die jeweils eine Spezifität gegenüber einem von vier interessierenden Analyten der Probe aufweisen (B). Die vier Analyten sind CBZ, CAF, DCF und ILA. In Kasten 1 des Schemas ist gezeigt, dass sich die vier Kohorten 1.1, 1.2, 1.3 und 1.4 einerseits hinsichtlich der Farbstoffmarkierung (im Schema durch unterschiedlich intensive Blautöne verdeutlicht) und andererseits durch oberflächlich, d.h. an einer Art funktionellen Gruppe der Silikatschale gebundene, jeweils einem interessierenden Analyten entsprechende Substanzen (z.B. CBZ, CAF, DCF und ILA) unterscheiden. Auf die Darstellung der Anti-fouling-Moleküle an der Oberfläche der Kern/Schale Mikropartikel wurde verzichtet. Der Zusatz der Probe zu den geeignet dispergierten Kohorten führt zur "ersten wässrigen Suspension" 11, mit Zusatz der primären Antikörper wird die "zweite wässrige Suspension" 12 erhalten. Optional kann der Zusatz der Probe und des primären Antikörpers gleichzeitig in einem Schritt erfolgen. Wie im Kasten 2 vereinfacht dargestellt, binden die zugesetzten primären Antikörper in der dritten wässrigen Dispersion 13 sowohl an jeweilige Probenbestandteile / z.B. CBZ, CAF, DCF und ILA/, als auch an die betreffenden Kern/Schale Mikropartikel der vorliegenden Kohorten 1.1, 1.2, 1.3 und 1.4. Nachfolgend (C) wird der Mischung ein für alle eingesetzten (monoklonalen) Antikörper universeller, fluoreszenzmarkierter sekundärer Antikörper / sek AK / zugesetzt und so die "dritte Suspension" 13 erhalten. Durch Zusatz (D) der Fixierlösung / Fixierlsg /, bspw. Formaldehyd oder ein anderes Aldehyd, werden ungebundene Reaktionspartner am weiteren Reagieren gehindert (denaturiert). Nun folgt, wohlgemerkt ohne zwischengeschalteten Wasschritt, das Auslesen (vgl. unterer Bildteil) mit Hilfe einer geeignet eingerichteten Messanordnung. Der linke Kasten im unteren Bildteil zeigt das Setzen der für die jeweiligen Analyte angepassten Gates, der mittlere die jeweils gezählten individuellen Partikel / counts / und der rechte Kasten zeigt den konzentrationsabhängigen Verlauf entsprechender Kalibrierkurven für die einzelnen Analyte. Deren Linearbereiche bieten hinlänglich weite Bereiche, in denen die betreffenden Analyten reproduzierbar nachweisbar sind.

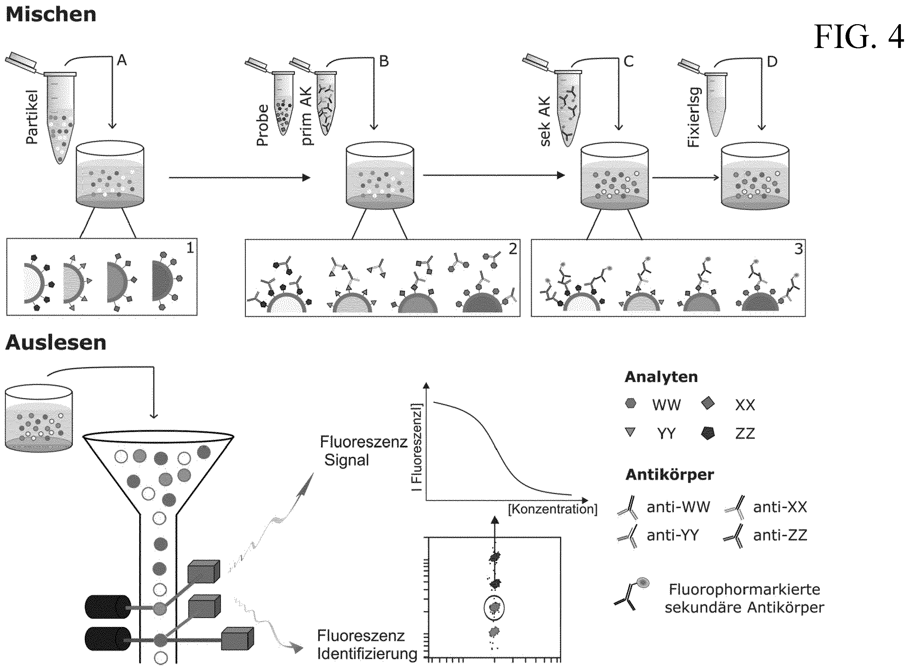

**Fig. 4** zeigt die zu den Fign. 2 und 3 beschriebenen Verfahrensschritte nochmals auf andere Art. Es soll verdeutlicht werden, dass die zuvor benannten Analyte CBZ, CAF, DCF und ILA selbstredend nur beispielhaft hier angeführt bzw. schematisch dargestellt sind. Mit dem beschriebenen Verfahren können auch jegliche andere der im Text genannten Analyten, unabhängig davon, ob es nun 2, 3, 4, 5, 6, 7, 8 oder mehr unterschiedliche Substanzen sind, simultan in einer Probe bestimmt werden. Aus Gründen der Übersichtlichkeit wurde auf Bezugszeichen verzichtet.

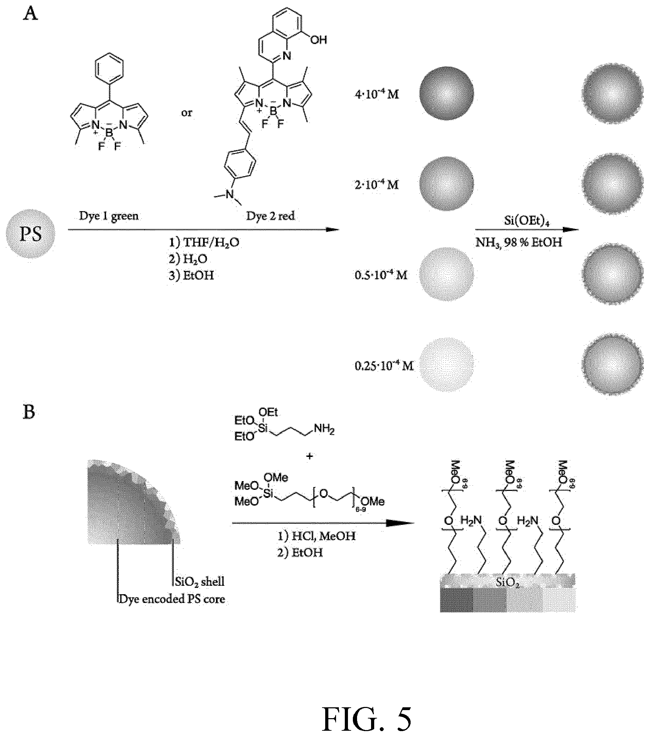

**Fig. 5** zeigt schematisch die Herstellung von Kern/Schale-Mikropartikeln mit farbstoffmarkiertem Polystyrolkern und Silikatschale. A: Die hydrophoben BODIPY Farbstoffe "1 green" und "2 red" werden mit vier verschiedenen Konzentrationen in Polystyrolkerne eingebracht, um einen vierfach Assay zu ermöglichen. Danach wird eine SiO₂ Hülle in einem Sol-Gel Verfahren unter Verwendung von Tetraethoxysilan (TEOS) und Ammonik als Katalysator erzeugt. B: Oberflächenfunktionalisierung mit Amino- und PEG-Gruppen unter Verwendung von 3-Aminopropyltriethoxysilan (APTES) und 3-[methoxy(polyethylenoxy)propyl]trimethoxysilan (6-9 PEG Einheiten).

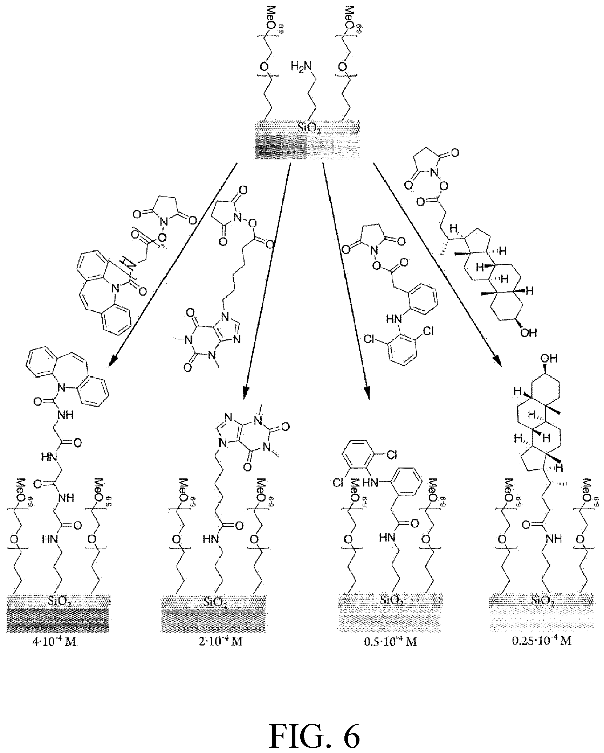

**Fig. 6** zeigt die Kopplung von NHS-aktivierten Haptenen (N-(Dibenz[b,f]azepin-5-ylcarbonyl)glycylglycylglycin (CBZfGly₃-COOH), 7-(5-Carboxypentyl)-1,3-dimethylxanthin (CAF-(CH₂)₅-COOH), DCF und ILA) an Aminogruppen auf der Oberfläche der PS-Kern/SiO₂-Schalen-Mikropartikel. Die Konzentration der Farbstoffe zur Kodierung der jeweiligen Partikelpopulation ist in der Box unterhalb der schematischen Darstellung der Oberfläche der Mikropartikel angegeben.

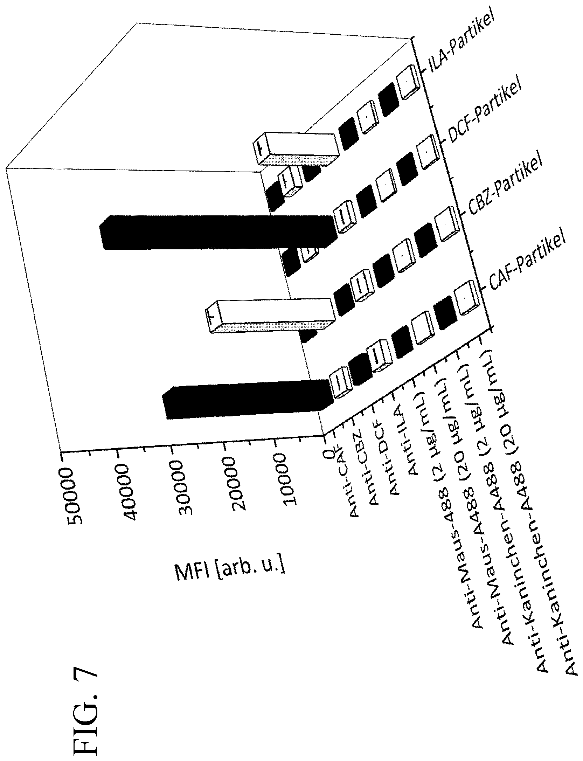

**Fig. 7** zeigt Ergebnisse zum Spezifizitäts-Test des Mikropartikelassays. Jede Partikelpopulation (Kohorte) wurde individuell mit den primären und sekundären Antikörpern /Antiserum behandelt. Antikörper-Konzentrationen betrugen 300 µg/L für monoklonale Antikörper, wohingegen die Anti-ILA-Serumverdünnung 1:20.000 betrug und die Konzentration der sekundären Antikörper 2 µg/mL in Kombination mit primären Antikörpern und 2 µg/mL (oder 2000 µg/L) und 20 µg/mL für unabhängige Messungen war. Während des Assays wurden keine Waschschritte durchgeführt. Mit Farbstoff 2 rot (dye 2 red) kodierte Mikropartikel wurden in Kombination mit Alexa Fluor™ 488 (A488) markierten Antikörpern verwendet.

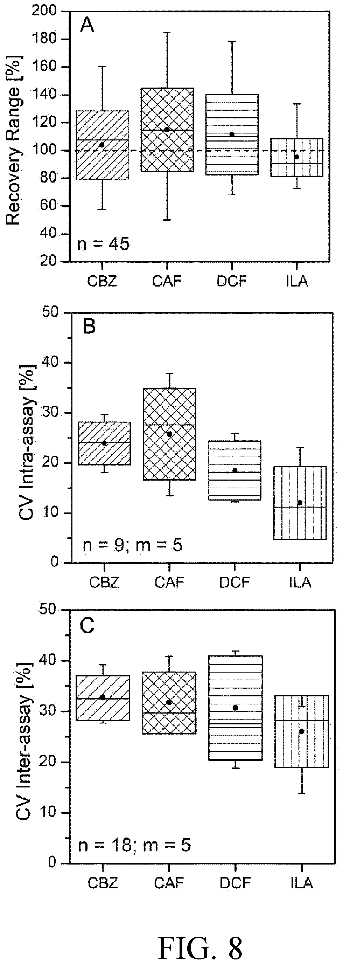

**Fig. 8** zeigt Wiederfindungsraten sowie intra- und inter-Assay-Variationskoeffizienten für den simultanen Nachweis von CBZ, CAF, DCF und ILA in 5, künstlich mit den betreffenden Analyten versetzten Wasserproben. Insbesondere zeigt die Fig. 6A die Wiederfindungsraten; Fig. 6B zeigt die Intra-Assay-Variationskoeffizienten und Fig. 6C zeigt die Inter-Assay-Variationskoeffizienten. Jede Probe enthielt eine zufällig ausgewählte und unterschiedliche Menge der Analyte und wurde mittels SAFIA in neun (n) Replikaten analysiert. Die eingezeichneten Intervalle betreffen 1-99% aller Daten, Punkte bezeichnen jeweils das arithmetische Mittel.

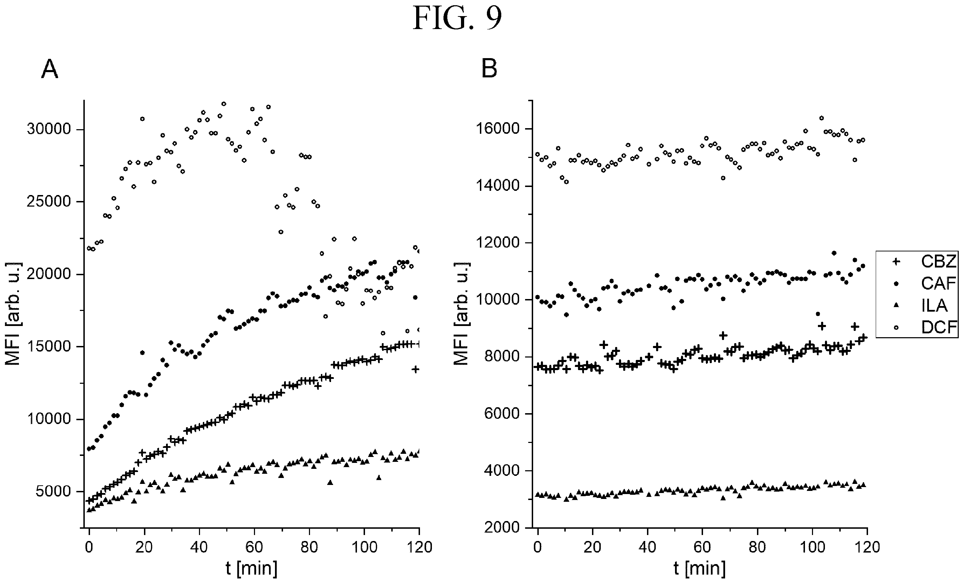

**Fig. 9** zeigt den Signalverlauf in Abhängigkeit vom Zeitpunkt der Messung: A: Ohne Fixierung der Antikörper, d.h. ohne Zusatz von Fixierlösung, umfassend ein Mittel zur Fixierung und Quervernetzung; B: Mit Fixierung unter Verwendung des beschriebenen Formaldehyds als Fixationsmittel. Jeder Punkt stellt die Messung einer Kavität eines sequentiellen, automatisierten Auslesesens einer Mikrotiterplatte dar. Der Assay wurde - um die maximale Signalintensität zu erreichen - mit Reinstwasser durchgeführt, d.h. es war kein Konkurrenzanalyt im Assay vorhanden. Die Zeitverzögerung zwischen den Messungen der Kavitäten betrug 1,5 Minuten.

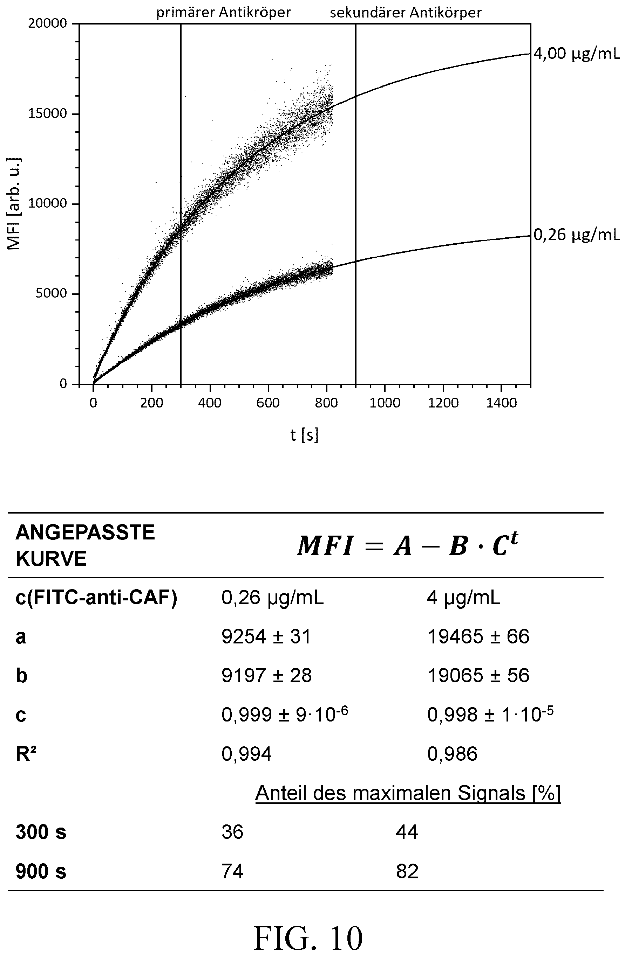

**Fig. 10** zeigt den Verlauf der Bindung eines FITC-markierten anti-CAF-Antikörpers an CAF-funktionalisierte Mikropartikel. Jeder Punkt repräsentiert die Fluoreszenzintensität eines einzelnen Kern/Schale-Mikropartikels.

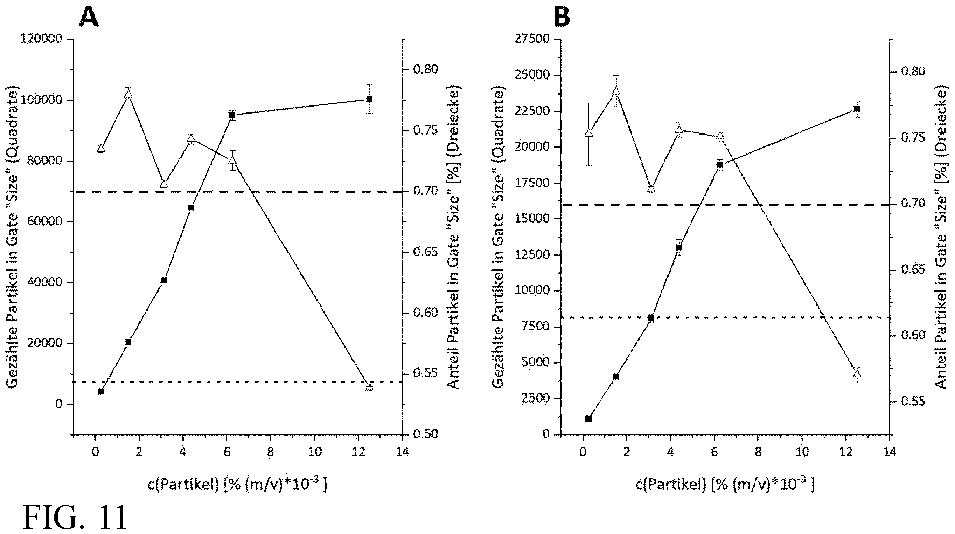

**Fig. 11** zeigt die gezählten Partikel (linke Ordinate, Quadrate) und den Partikelanteil (rechte Ordinate, Dreiecke) im Gate "Size" in Abhängigkeit von der verwendeten Partikelkonzentration. A: Durchflussrate 66 (µL/min und B: Durchflussrate 14 µL/min. Linie, Quadrate verbindend: Minimale Anzahl von Partikeln (8000), die in FSC/SSC gezählt werden müssen. Linie, Dreiecke verbindend: Minimaler Anteil der Partikel in R-1 (70 %)

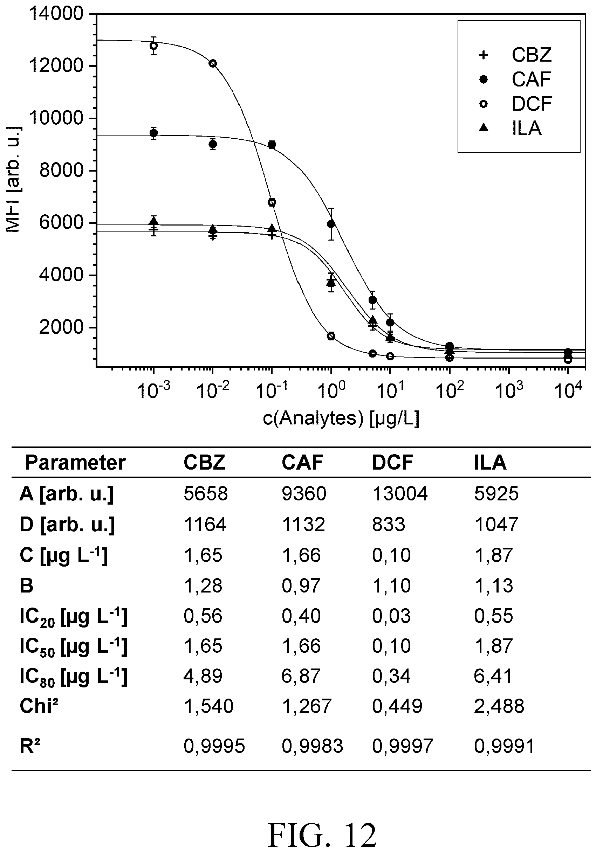

**Fig. 12** zeigt Kalibrierkurven für die mittleren Fluoreszenzwerte auf logarithmischer Skala abgetragen gegen die Konzentration der Analyte CBZ, CAF, DCF und ILA (oben). Die Fehlerbalken resultieren aus vier Messungen. Eine vier-Parameter Logit-Funktion wurde zur Kurvenanpassung genutzt. Die dabei ermittelten Parameter des optimalen Kurvenfitting sind in der Tabelle (unten) dargestellt. Es sind ausgeprägte Linearbereiche erkennbar, die sich für den Nachweis der betreffenden Analyte anbieten.

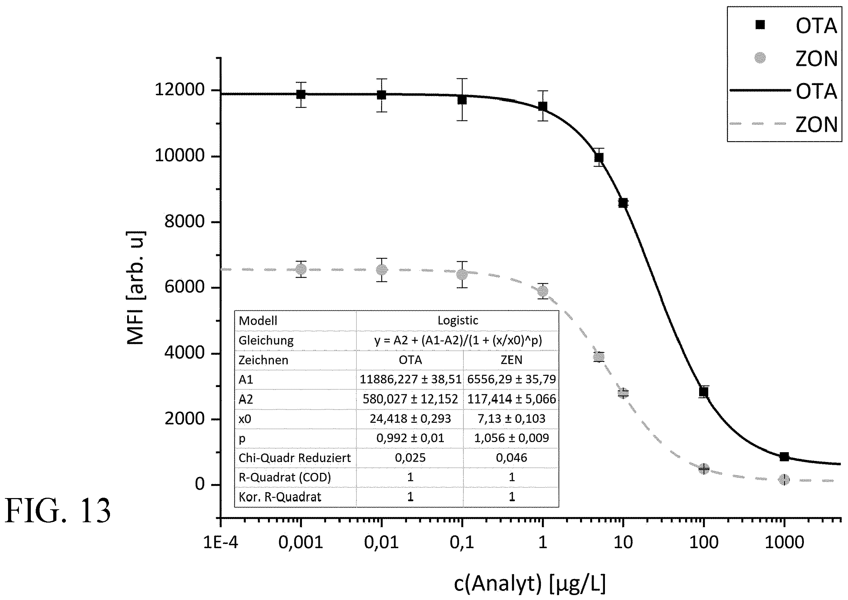

**Fig. 13** zeigt Kalibrierkurven für die mittleren Fluoreszenzwerte auf logarithmischer Skala abgetragen gegen die Konzentration der Analyte OTA und ZON. Die Fehlerbalken resultieren aus drei Messungen. Eine vier-Parameter Logit-Funktion wurde zur Kurvenanpassung genutzt. Die dabei ermittelten Parameter des optimalen Kurvenfitting sind in der Tabelle dargestellt. Es ist ein ausgeprägter Linearbereich erkennbar, der für den Nachweis dieser Toxine in Agrarrohstoffen (bzw. deren Extrakten) geeignet ist.

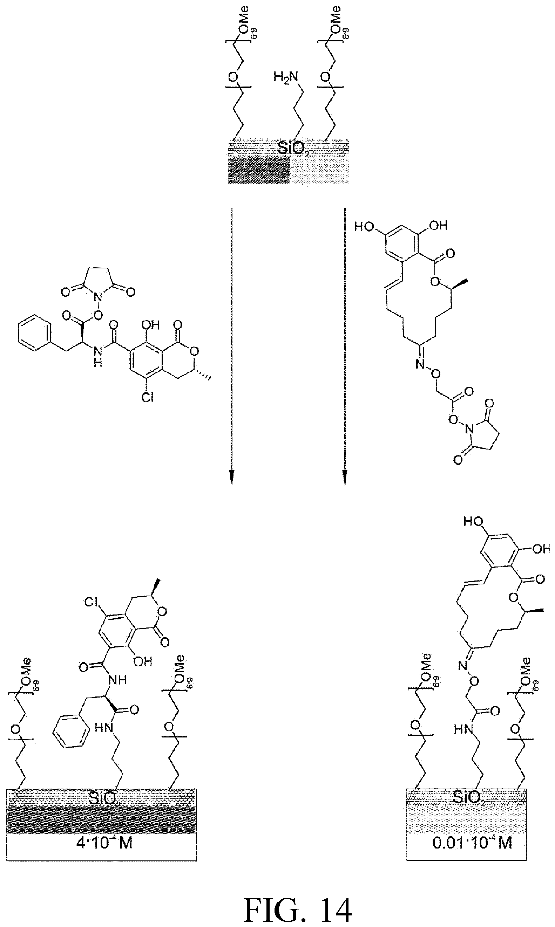

**Fig 14****.** zeigt die Kopplung von NHS-aktivierten Haptenen Zearalenon-7-(O-Carboxymethyl)oxim (ZON-COOH) und OTA an Aminogruppen auf der Oberfläche von PS-Kern/SiO₂-Schalen-Mikropartikeln und damit schematisch die Herstellung von zwei unterschiedlichen Kohorten der zur simultanen Bestimmung von OTA und ZON (vgl. Fig. 13) eingesetzten Kern/Schale-Mikropartikel. Die Konzentration der Farbstoffe zur Kodierung der jeweiligen Partikelpopulation ist in der Box unterhalb der schematischen Darstellung der Oberfläche der Mikropartikel angegeben.

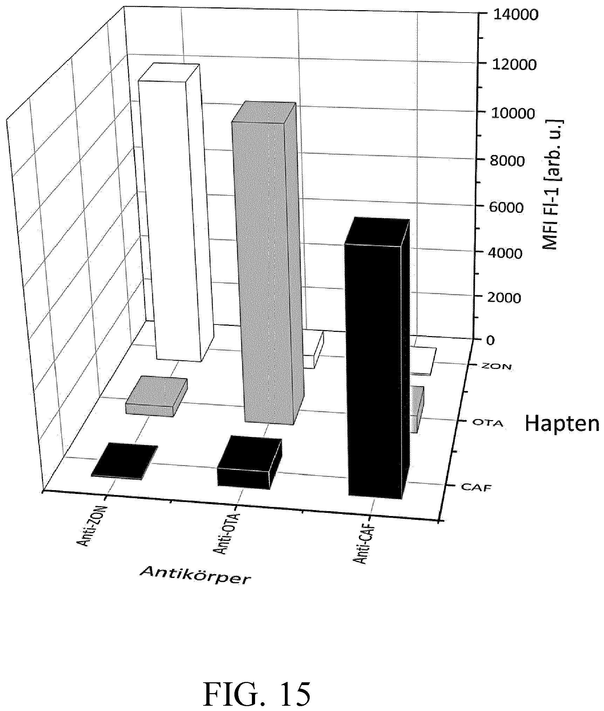

**Fig. 15** zeigt Ergebnisse zum Spezifizitäts-Test des Mikropartikelassays für die Toxine ZON und OTA im Vergleich zum anthropogenen Marker CAF. Jede Partikelpopulation (Kohorte) wurde individuell mit den primären und sekundären Antikörpern /Antiserum behandelt. Die Antikörper-Konzentrationen betrugen 500 µg/L für anti-OTA und Anti-ZON Antikörper. Die Konzentration der Anti-CAF-Antikörper betrug 272 µg/L. Die Konzentration der sekundären Antikörper betrug 2 µg/mL in Kombination mit primären Antikörpern. Während des Assays wurden keine Waschschritte durchgeführt. Mit Farbstoff 2 rot (dye 2 red) kodierte Mikropartikel wurden in Kombination mit Alexa Fluor™488 (A488) markierten Antikörpern verwendet.

### Ausführungsbeispiele

Nachfolgend wird beispielhaft die Entwicklung eines vierfachen (CBZ, CAF, DCF und ILA) und eines zweifachen Immunoassays (ZON und OTA) auf Polystyrol (PS) Kern/Silica (SiO₂) Schale Mikropartikeln beschrieben. Zur Kodierung der Kern/Schale-Mikropartikel dienten unterschiedliche Anteile eines grün ("dye 1 green") und eines rot fluoreszierenden ("dye 2 red") BODIPY Farbstoffs. Die bezeichneten Partikel lassen sich leicht mit Hilfe von auf der Partikelschale angebrachten Amino-Gruppen funktionalisieren, sodass entsprechende NHS-aktivierte Haptene kovalent an der Silikatschale verankert werden können (vgl. **Fig. 5****,** **6**). Mittels der bereits beschriebenen Silan-Chemie können ebenso PEG-Gruppen verankert werden, um eine unspezifische Proteinbindung ("fouling") zu minimieren.

Materialien: Die verwendeten Reagenzien wurden von Merck KGaA, Sigma-Aldrich, Fluka, Steraloids, Chemsolute oder Serva in der jeweils verfügbaren höchsten Reinheitsstufe bezogen. Der monoklonale anti-CAF Antikörper (mAb) (Maus, IgG2b, clone 1.BB.877, lot L2051502M) wurde von US Biological (Swampscott, MA, USA), der anti-DCF mAb (Maus, IgG2κ, clone 12G5) von dessen Herstellern bezogen. Der anti-CBZ mAb (Maus, IgGl, "clone-1" / Bestellnummer BAM-mab 01 (CBZ) und das anti-ILA Antiserum (Kaninchen, polyklonal, "rabbit 2", dritte Serumentnahme) wurden von uns selbst erzeugt. Die anti-OTA mAB (Clone G213-BC10) und anti-ZON mAB (Clone: G190-AE9) wurde von der Hybrotec GmbH (Potsdam, Deutschland) zur Verfügung gestellt. Alexa Fluor™ 488 und Alexa Fluor™ 647 markierte Ziege anti-Maus Antikörper (IgG(H+L), RRID: AB_2534088 und AB_2633277) und Ziege anti-Kaninchen Antikörper (IgG (H+L), RRID: AB_143165 und AB_2633282) wurden von Thermo Fisher Scientific erworben. Es wurde durchgehend ultrareines Wasser eines Milli-QReference (Millipore) Wasserreinigungssystems genutzt.

Die Herstellung der Kern/Schale-Mikropartikel erfolgte wie zuvor beschrieben (Sarma, D., Gawlitza, K., Rurack, K. Langmuir 2016, 32, 3717-3727; Sarma, D., Carl, P., Climent, E., Schneider, R.J., Rurack, K. ACS Appl. Mater. Interfaces 2019, 11, 1321-1334): Polystyrol-Kerne wurden mittels Dispersionspolymerisation mit ACVA als Initiator und PVP 40 als Stabilisator synthetisiert. Die Polymerpartikel wurden mit unterschiedlichen Mengen des entsprechenden hydrophoben BODIPY-Farbstoffs ("dye 2 red" und "dye 1 green", vgl. Fig. 3) mittels einer Aufquelltechnik präpariert um die vier verschiedenen Partikel-Kohorten zu erhalten. Nach dieser Kodierung wurde die Beschichtung mit SiO₂ zur Ausbildung der Silikatschale gemäß einer Stöber Sol-Gel Synthese unter Verwendung von Tetraethoxysilan (TEOS) als Präcursor und Ammoniumhydroxid als Katalysator vorgenommen. Abschließend wurden die Kern/Schale-Mikropartikel mit 3-Aminopropyltriethoxysilan (APTES) funktionalisiert, um die zur kovalenten Verankerung der Haptene erforderlichen Aminogruppen zu erzeugen und synchron zur Reaktion mit APTES mit Polyethylenglycol (3-[methoxy(polyethylenoxy)propyl]trimethoxysilan, 90%, 6-9 PEG Reste) (PEG₆₋₉-PTMS) zur Verminderung unspezifischer Proteinbindung modifiziert.

Die Kopplung der Haptene erfolgte durch kovalente Bindung mittels DCC/NHS Verknüpfung. DCF und ILA wurden direkt nach Aktivierung ihrer Carboxylgruppen gebunden. Für CAF und CBZ wurden die Hapten-Derivate 7-(5-Carboxypentyl)-1,3-di-methylxanthin (CAF-(CH₂)₅-COOH)14'15 und N-(Dibenz[b,f]azepin-S-ylcarbonyl) glycylglycylglycin (CBZ-Gly₃-COOH) verwendet, deren Carboxylgruppen vor der Kopplung entsprechend aktiviert wurden (vgl. **Fig. 6**). OTA wurde ebenfalls direkt durch Aktivierung der Carbonsäure an die Partikel gekoppelt. Für ZON wurde das Hapten-Derivat Zearalenon-7-(O-Carboxymethyl)oxim verwendet. Dieses wurde nach der Vorschrift von D. Thouvenot et al. (D. Thouvenot and R. F. Morfinn, Appl. Environ. Microbiol., 1983, 45, 16-23.) synthetisiert. 1,5 µmol ZON wurden in 0,49 mL Pyridin gelöst und in einem Braunglas-Schraubdeckelgefäß (5 mL) vorgelegt. Dazu wurden 2,3 µmol O-(Carboxymethyl)hydroxylamine hemihydrochloride in 0,05 mL Pyridin gegeben. Die Reaktion wurde für 5 h unter Rühren durchgeführt. Nach der Reaktion wurde Pyridin im Stickstoffstrom entfernt. Der Erfolg der Synthese wurde mittels HPLC-MS bestätigt (m/z 390).

Insbesondere wird die Präparation der NHS-Ester der Antigene und deren Kopplung an die Partikel wie folgt vorgenommen: In einem 0,5 mL Mikrozentrifugenröhrchen wurden zur Aktivierung der Carbonsäuren jeweils 12 µmol Hapten in 72 µL trockenem DMF gelöst. Dazu wurden 14,4 µmol NHS (1.2 eq.), eine Spatelspitze von ca. 1 mg *N,N'*-Disuccinimidylcarbonat und 14,4 µmol DCC in dieser Reihenfolge gegeben. Dafür wurden 0,5 M Stammlösungen von NHS und DCC in DMF frisch vor der Reaktion hergestellt. Der Ansatz wurde im Dunkeln bei RT über Nacht im Eppendorf ThermoMixer C bei 800 u/min geschüttelt, dann bei mindestens 10.000 g zentrifugiert, um den entstandenen Dicyclohexylharnstoff abzutrennen. Die erhaltenen Überstände wurden direkt ohne weitere Aufreinigung verwendet. Zur Kopplung der Haptene wurden 50 µL der erhaltenen Überstände der NHS-Ester zu 1500 µL einer 1 mg/mL Suspension der amino- oder amino-/PEG-funktionalisierten PS/SiO₂-Kern-Schale-Partikel in abs. EtOH gegeben. Die Reaktion wurde in einem 2 mL Mikrozentrifugenröhrchen durchgeführt. Jedes NHS-aktivierte Hapten wurde dabei zu einer mit BODIPY Farbstoff 1 ("dye 1 green") grün oder BODIPY Farbstoff 2 ("dye 2 red") rot codierten Partikelpopulation gegeben.

Der Reaktionsansatz wurde bei RT 20 h geschüttelt. Danach wurden die Partikel dreimal mittels Zentrifugations- (maximal mit 10.000 g) und Redispersionszyklen mit abs. EtOH gewaschen. Nach dem letzten Zentrifugationsschritt und Entfernen des Lösemittels wurden die Partikel in 150 µLabs. Ethanol redispergiert und bis zur Verwendung in der Dunkelheit und bei 4 °C gelagert. Die Herstellung der Partikel ist schematisch in den Figuren 5, 6 und 14 gezeigt.

Das anti-CAF FITC-Konjugat wurde wie folgt präpariert: Mittels Gelpermationschromatographie wurde zunächst der Puffer von monoklonalen anti-CAF-Antikörpern, IgG, clone 1.BB.877 (US Biological, 1,36 mg mL⁻¹) ausgetauscht: Dazu wurden 150 µL des Antikörpers auf eine GE PD Spin Trap G-25 Zentrifugationssäule gegeben und der Antikörper wurde mit 0,1 M Na₂CO₃ Lösung (pH = 9,00) eluiert. Die Lösung wurde in ein 1,5 mL Mikrozentrifugenröhrchen überführt. Zu dieser Lösung wurde dreimal 10 µL frisch angesetzte FITC-Lösung (0,4 mg/mL in wasserfreiem DMSO) langsam hinzugegeben und der Ansatz wurde bei 4 °C über Nacht im Eppendorf ThermoMixer C geschüttelt. Das entstandene Konjugat wurde anschließend mittels Gelpermationschromatographie gereinigt, analog zum Pufferaustausch, wobei PBS als Elutionspuffer verwendet wurde. Das isolierte Konjugat wurde mittels Nanodrop 1000 spektralphotometrisch charakterisiert. Ein mittleres Fluorophor-zu-Protein-Verhältnis (F/P) wurde bestimmt zu 3,80 und die Konzentration wurde bestimmt mit 0,21 mg/mL.

Die Charakterisierung der sekundären Antikörper erfolgte wie praxisüblich: Die Bestimmung der Markierungsdichte (Fluorophor zu Protein-Verhältnis) von Alexa Fluor® 488 und Alexa Fluor® 647-markierten Ziege-anti-Maus-IgG(H+L) und Ziege-anti-Kaninchen-IgG(H+L)-Antikörpern wurde hier durch UV/VIS-Absorptionsspektroskopie mithilfe eines Spektralphotometers Nano-drop 1000 (Thermo Scientific) bestimmt. Die Extinktionswerte bei 280 nm und 495 nm (Alexa Fluor® 488) und bei 650 nm (Alexa Fluor® 647) wurden für die Berechnung verwendet. Die Antikörper wurden unverdünnt vermessen.

Die LC-MS/MS-Analyse wurde wie nachfolgend beschrieben vorgenommen: LC-MS/MS Experimente wurden mit einem Agilent 1260 Infinity LC System (Agilent Technologies), ausgestattet mit Binärpumpe, Autosampler, Säulenofen und UV-Detektor, gekoppelt an einen Triple Quad™ 6500 (AB Sciex) Massenspektrometer, ausgeführt. Eine Kinetex C18 Vorsäule (Phenomenex) und eine Kinetex XB-C18 core/shell Säule (150 mm x 3 mm, 2,6 µm) wurden zur Separation verwendet. 10 µL Probe wurden für die Bestimmung von CBZ, CAF und DCF und 20 µL für die Bestimmung von ILA injiziert. Der Säulenofen wurde mit einer Temperatur von 55 °C betrieben und die Flussrate auf 350 µL/min eingestellt. Ein binärer Gradient aus Wasser und Methanol wurde zur Elution verwendet. Beide enthielten 10 mM Ammoniumacetat und 0,1 % (vol/vol) Essigsäure. Der Gradient wurde nach folgendem Schema verwendet: 30 % Methanol, isokratisch für 3 min, lineare Zunahme auf 95 % Methanol in 9 min, isokratisch mit 95 % MeOH für weitere 6 min. Dann wurde zu 30 % Methanol in 0,5 min zurückgekehrt, das Verhältnis wurde weitere 5,5 min beibehalten. CBZ, CAF und DCF wurden im positiven Elektrospray-Modus (ESI+) mit einer Quelltemperatur von 400 °C und einer Ionisierungsspannung von 4500 V betrieben. Zur Quantifizierung wurden folgende Masseübergänge im selected reaction monitoring (*engl.* für ausgewählter Reaktionsüberwachungs-) Modus (SRM) gewählt: SRM1 (CBZ): *m*/*z* 237→194; Kollisionsenergie (CE) 30 V; Zellenausgangspotenzial (CXP) 14 V, Entclusterungspotential 90 V, Verweilzeit 50 ms. SRM2 (CAF): *m*/*z* 195→138; Kollisionsenergie (CE) 27 V; Zellenausgangspotenzial (CXP) 12 V, Entclusterungspotential 60 V, Verweilzeit 50 ms. SRM3 (DCF): *m*/*z* 296→214; Kollisionsenergie (CE) 30 V; Zellenausgangspotenzial (CXP) 15 V, Entclusterungspotential 60 V, Verweilzeit 50 ms .Zur Bestimmung von ILA, wurde der negative Elektrospray Ionisierungsmodus (ESI-), mit einer Quelltemperatur von 400 °C und einer Ionisierungsspannung von 4500 V, gewählt. SRM4 (ILA): *m*/*z* 375→375; Kollisionsenergie (CE) -45 V; Zellenausgangspotenzial (CXP) -10 V, Entclusterungspotential -60 V, Verweilzeit 50 ms. Die Datenaufnahme und -analyse erfolgte mit der Software Analyst® 1.6.2 (AB Sciex).

Die Analyse von Proben aus Abwasserbehandlungsanlagen erfolgte mittels ELISA mit transparenten 96-Well, "high binding" Mikrotiterplatten (Greiner Bio-One) Währen der Inkubation wurden die Platten auf einem Orbitalschüttler bei 750 u/min geschüttelt, dabei wurden sie mit Parafilm® M versiegelt. Zum Waschen der Platten wurden ein automatisches 96-Kanal Mikrotiterplattenwaschgerät und PBS-Waschpuffer verwendet. Die Platten wurden in einem Waschprogramm, bestehend aus drei einzelnen Waschzyklen gewaschen und danach auf Laborpapier ausgeklopft. Für alle Immunoassays wurde die HRP-Substratlösung nach *Frey et al* (Frey, A; Meckelein, B., Externest, D.; Schmidt, M. A. I. Immunol. Methods 2000, 233, 47-56), verwendet und zu 200 µL pro Kavität aufgegeben. Sie wurde unmittelbar vor dem Signal generierenden Schritt aus Citratpuffer (22 mL), Wasserstoffperoxid (8.5 µL einer 30 %(vol/vol)igen Lösung) und TMB-Stammlösung (550 µL) hergestellt. Die Signalentwicklung wurde durch Zugaben von 100 µL 1 M H₂SO₄ nach 30 min gestoppt. Die Extinktion bei 450 nm und bei 620 nm (Referenz) wurde entweder mit dem Mikortiterplattenspektralphotometer Eon oder SpektraMax Plus 384 gemessen. Sofern nicht anders angegeben, wurden die Standards in Milli-Q-Wasser durch serielle Verdünnung der Stammlösung des Analyten in MeOH verdünnt. Eine Acht-Punkte Kalibrationskurve mit den Analytkonzentrationen 10.000 (1000) µg/L, 100 µg/L, 10 µg/L, 5 µg/L, 1 µg/L, 0,1 (0.5) µg/L, 0,01 (0.05) µg/L und 0,001 µg/L, wurde je nach Anforderung an die zu bestimmenden Proben oder analytischen Fragestellungen gewählt. Proben und Standards wurden vollständig randomisiert auf den Platten aufgetragen, um Platteneffekte zu vermeiden. Standards wurden in Triplikaten und Proben in Quadruplikaten vermessen, sofern nicht anders angegeben. Diese wurden dazu entweder in 96-Well non-Binding Mikrotiterplatten (Greiner Bio-One) oder 96-Deep-Wellplatten aus Glas vorgelegt und das für den ELISA benötigte Probenvolumen wurde daraus mittels einer Achtkanalpipette oder des Liquidator entnommen. Auf jeder einzelnen Mikrotiterplatte wurden jeweils acht verschiedene Kalibratoren vermessen, um eine Kalibrationskurve zu generieren. Kalibratoren und Standards wurden einem Ausreißertest nach Grubbs mit einem Signifikanzlevel von α = 0,05 unterzogen; als Ausreißer identifizierte Messwerte wurden in nachfolgenden Berechnungen ausgeschlossen. Zur Generierung der Kalibrationskurve wurde die Differenz der Extinktionswerte von 450 nm und 620 nm gegen die Konzentration des Analyten auf einer logarithmischen Skala aufgetragen und die vier-parametrische Kalibrationsfunktion wurde in Origin 9.0 mithilfe eines *"Least-Squares"-*Algorithmus unter Verwendung von instrumenteller Gewichtung angepasst.

Die Bestimmung von CBZ, CAF und ILA mittels direktem ELISA erfolgte nach folgendem Protokoll: Zunächst wurden die Kavitäten der Mikrotiterplatten mit polyklonalen sekundären Antikörpern beschichtet, diese wurden in einer Konzentration von 1 mg/L in PBS und einem Volumen von 200 µL/Well für mindestens 18 h inkubiert. Nach einem Waschschritt wurde in jede Kavität mit 200 µL des entsprechenden primären Antikörpers in PBS (Maus-anti-CBZ IgG: 7,5 µg/L; Maus-anti-CAF IgG: 27,2 µg/L; Verdünnung des Kaninchen-anti-ILA-Serums 1:50.000) gefüllt und eine Stunde inkubiert. Nicht gebundene Antikörper wurden durch einen weiteren Waschschritt entfernt. Danach wurden 150 µL der Probe oder des Standards und sofort danach 50 µL des entsprechenden Tracers in TRIS-Puffer (CAF-C5-Alkylspacerspacer-HRP, 4.1 ng/L) oder Probenpuffer (CBZ-Triglycin-HRP, 8,3 ng/L und ILA-HRP 1: 100.000 verdünnt) aufgegeben und 30 min (CAF, CBZ) oder 60 min (ILA) inkubiert. Nach einem finalen Waschritt wurde die Signalentwicklung und Auswertung wie oben beschrieben durchgeführt.

Für die Bestimmung von DCF mittels indirektem ELISA wurde folgendes Protokoll verwendet: Die Kavitäten der Mikrotiterplatten wurden zunächst mit 200 µL 2-[2-[(2,6-Dichlorphenyl)amino]phenyl]essigsäure-N-(5-carboxypentyl)amid gekoppelt an Apo-Transferrin (DCF-NH-(CH₂)₅-APO) in einer Konzentration von 30 µg/L in PBS für mindestens 18 h beschichtet. Nach einem Waschschritt wurden die Kavitäten mit Casein blockiert, dieses wurde als 0,1 %ige (m/m) Lösung in PBS verwendet und 60 min inkubiert. Die Platte wurde erneut gewaschen. Danach wurden 150 µL Standard oder Probe gefolgt von 50 µL des primären anti-DCF-Antikörpers, in einer Konzentration von 20 µg/L in TRIS-Puffer, in jede Kavität gegeben und für weitere 60 min inkubiert. Die Platte wurde erneut gewaschen und jede Kavität wurde mit 200 µL eines polyklonalen Ziege-anti-Maus-HRP-Antikörper befüllt. Dieser wurde dazu 1:20.000 in TRIS-Puffer verdünnt. Nach 60 min Inkubationszeit wurde die Platte zum letzten Mal gewaschen und das Signal wurde wie oben beschrieben generiert.

Indirekt kompetitiver partikelbasierter Immunoassay: Alle Immunoassays wurden in schwarzen, nicht-bindenden Mikrotiterplatten mit flachem Boden (Greiner Bio-One) durchgeführt. Zum Verdünnen der Suspension der Mikropartikel (Beads) und der biochemischen Reagenzien wurde TRIS-gepufferte Salzlösung (10 mM Tris (hydroxymethyl)aminomethan, 150mM NaCl, pH = 8,5) verwendet. Zunächst wurden 50 µL der Probe oder des Standards und 50 µl einer Mischung der primären (prim.) Antikörper anti-CAF (272 µg/L), anti-CBZ (350 µg/L), anti-DCF (20 µg/L), anti-OTA (50 ng/L), anti-ZON (250µg/L) und anti-ILA (Verdünnung des (polyklonalen) Antiserums: 1:10.000) während 5 Minuten auf einem Kreisschüttler (Titra-max 101, Heidolph) bei 750 U/min geschüttelt. Zur Kalibrierung wurde eine 8-Punkt Verdünnungsserie eines Mischstandards jedes Analyten umfassend Konzentrationen von 10.000 µg/L bis 1 ng/L verwendet, der durch serielle Verdünnung einer methanolischen Stammlösung von 1 mg/mL jedes einzelnen Analyten mit ultrareinem Wasser hergestellt worden war. Danach wurden 20 µL einer Mischung der fluoreszenzkodierten, Hapten-gekoppelten Mikropartikel (Mikropartikel) zugesetzt und für weitere 5 min geschüttelt. Um ein Messsignal der gebundenen Antikörper zu erhalten, wurden 100 µL Alexa Fluor™488 Ziege anti-mouse (und anti-Kaninchen) IgG(H+L), je 2 µg/mL Antikörper (dye 2 red-kodierte Partikel), oder Alexa Fluor™647 Ziege anti-Maus (und anti-Kaninchen) IgG(H+L), je 2 µg/ml Antikörper (dye 1 green kodierte Partikel) den Kern/Schale-Partikeln zugesetzt und für 15 min inkubiert.

Abschließend wurden, um den Assay zu stoppen, 100 µL einer 6%-igen Formaldehyd-Lösung (aus 37% HCHO/10-15% MeOH Stammlösung, (w/w)), 2% MeOH und 0.01% SDS in PBS zugesetzt und für mindestens 10 min inkubiert. Die Mischung jedes Kavität wurde in einem BD Accuri C6 oder BD AccuriC6 plus Durchflusszytometer bei einer Flussrate von 66 µL/min während 30 s analysiert, wobei ein Schwellwert von 80.000 a.u. am FSC Detektor und ein Probeninjektionsport (SIP)- Reinigungszyklus zwischen den einzelnen Durchläufen eingeschoben wurde, um ein Verschleppen (Übertrag) zu verhindern. Standards und Proben wurden zufällig über die Mikrotiterplatte verteilt angeordnet, um einen Einfluss von Signaldrift und Randeffekte möglichst auszuschließen. Zum Testen der Bindungs-Selektivität wurde jeder Antikörper separat mit jedem haptenmodifizierten (d.h. Haptengekoppeltem) Set der verwendeten Kern/Schale-Mikropartikel inkubiert. Die hier als Fixierlösung verwendete Lösung umfasst bevorzugt Formaldehyd im Konzentrationsbereich 1-10 % vol/vol), Methanol (1-10 % vol/vol) und Natriumdodecylsulfat (0,001-10 % m/m). Statt Formaldehyd können auch andere Aldehyde mit quervernetzenden Eigenschaften (z.B. Glyoxal, Glutarladehyd, Malondialdehyd, Dipalaldehyd) eingesetzt werden. Statt Natriumdodecylsulfat können andere Detergenzien (z.B. Tween20, TitronX, Genapol, CTAB) verwendet werden.

Datenbehandlung: Alle zytometrischen Daten wurden zunächst nach Streuwerten gegated (FSC/SSC (gate "size": vgl. **Fig. 2E**) um Partikelaggregate von der Berechnung auszuschließen. Danach wurden vier Gates am FSC/FL-4 (dye 2 red kodierte Partikel, Fig. 2F) oder FSC/FL-1 (dye 1 green kodierte Partikel) verwendet, um die Partikel zu dekodieren und die Analyt-bezogene mittlere Fuoreszenzintensität zu berechnen (vgl. Fig. 2G). Mittelwerte und Standardabweichungen von Wiederholungsmessungen wurden gegen die Konzentration der entsprechenden Analyte auf einer logarithmischen Skale abgetragen. Die von Rodbard eingeführte (Rodbard, D. Biometrics 1975, 31, 599-600) Vier-Parameter-Eichkurve wurde nach der Methode der kleinsten Fehlerquadrate mittels Origin® ver. 9 Grafik-Software gefittet. Ausreißer wurden mittels Grubbs Test bei einem SignifikanzNiveau α = 0,05 identifiziert und eliminiert. Welch's t-Test wurde mit einem Signifikanzniveau von α = 0,05 unter Zuhilfenahme von Origin® 9 verwendet.

Bestimmung der molaren Kreuzreaktivitäten: Die molaren Kreuzreaktivitäten (CR-%), die von den Antikörpern im Assay angezeigt werden, wurden nach der Methode von Abraham bestimmt (Abraham, G. E. J. Clin. Endocrinol. Metab. 1969, 29, 866-870). Die in dem vorliegend beschriebenen SAFIA erhaltenen CR-% werden mit denen von ELISA in der nachfolgenden Tabelle 2 verglichen.

**Tabelle 2 Molare Kreuzreaktivität (CR-%) bei SAFIA im Vergleich zum ELISA**

| Substanz | Molare Kreuzreaktivität [%] | | | | | | | |
|---|---|---|---|---|---|---|---|---|
| | CBZ (SAFIA) | CBZ (ELISA) | CAF (SAFIA) | CAF (ELISA) | DCF (SAFIA) | DCF (ELISA) | ILA (SAFIA) | ILA (ELISA) |
| CBZ | 100 | 100 | <0,1 | - | <0,1 | - | < 0,1 | - |
| CAF | <0,1 | - | 100 | 100 | <0,1 | - | < 0,1 | - |
| DCF | <0,1 | - | <0,1 | - | 100 | 100 | < 0,1 | - |
| ILA | <0,1 | - | <0,1 | - | <0,1 | - | 100 | 100 |
| 3-Hydroxycarbamazepin | 20,2 | 5,1 | <0,1 | - | <0,1 | - | < 0,1 | - |
| 10,11-Dihydroxy-carbamazepin | 0,3 | 0,1 | <0,1 | - | < 0,1 | - | - | - |
| 10,11-Epoxycarbamazepin | 117 | 140 | <0,1 | - | <0,1 | - | < 0,1 | - |
| Theophyllin | <0,1 | - | 5,8 | 12,0 | <0,1 | - | <0,1 | - |
| Paraxanthin | <0,1 | - | <0,1 | <0,1 | <0,1 | - | <0,1 | - |
| Theobromin | <0,1 | - | 2,0 | 0,13 | <0,1 | - | <0,1 | - |
| 4'-Hydroxydiclofenac | <0,1 | - | <0,1 | - | <0,1 | 11 | <0,1 | - |
| 5'-Hydroxydiclofenac | <0,1 | - | <0,1 | - | 9,8 | 13 | <0,1 | - |
| Aceclofenac | <0,1 | - | <0,1 | - | 16,6 | - | <0,1 | - |
| Cholsäure | <0,1 | - | <0,1 | - | <0,1 | - | <0,1 | <0,1 |
| Lithocholsäure | <0,1 | - | <0,1 | - | <0,1 | - | 1,9 | 0,3 |
| Taurolithocholsäure | <0,1 | - | <0,1 | - | <0,1 | - | <0,1 | 1,2 |

Leitungswasser- und Abwasserproben mit Analytzusatz: Zur Bestimmung der Wiederfindungsraten und der Genauigkeit wurden fünf Proben mit CBZ, CAF, DCF und ILA in verschiedenen, zufällig gewählten Konzentrationen Leitungswasser (Berlin-Adlershof) zugesetzt. Die Analytzusatz-Konzentrationen sind in Tabelle 3 angegeben.

Analyse von Maismehlproben unter Analytzusatz: 1 g Maismehl wurde mit 10 mL Methanol (70 %) 10 min extrahiert. Dann wurde der Extrakt zentrifugiert, und 5 mL des Überstandes wurden 1:5 mit MilliQ-Wasser verdünnt. Die Verdünnung wurde mit einer OTA und ZON Stocklösung dotiert, sodass die in Tab. 8 angegebenen Konzentrationen von OTA und ZON erreicht wurden.

**Tabelle 3 Nominelle Konzentration von 5 "bespikten" Trinkwasserproben und mittels SAFIA ermittelte Konzentrationen. Die Analyse wurde in neun Replikaten ausgeführt.**

| Probe | Nominale Konzentration [µn/L] | | | | Bestimmte Konzentration [µg/L] | | | |
|---|---|---|---|---|---|---|---|---|
| Analyt | CBZ | CAF | DCF | ILA | CBZ | CAF | DCF | ILA |
| #1 | 0,53 | 2,23 | 0,42 | 3,71 | 0,53 ± 0,12 | 2,58 ± 0,35 | 0,55 ± 0,07 | 3,50 ± 0,23 |
| #2 | 5,80 | 0,85 | 0,08 | 0,53 | 6,18± 1,11 | 0,97 ± 0,27 | 0,09 ± 0,02 | 0,61 ± 0,14 |
| #3 | 1,27 | 1,70 | 0,05 | 4,77 | 1,33 ± 0,40 | 1,96 ± 0,74 | 0,05 ± 0,01 | 4,15 ± 0,60 |
| #4 | 0,84 | 4,77 | 0,21 | 8,48 | 0,94 ± 0,23 | 5,58 ± 1,60 | 0,31 ± 0,04 | 7,31 ± 0,34 |
| #5 | 9,50 | 8,69 | 0,04 | 2,65 | 9,15 ± 2,34 | 9,68 ± 2,05 | 0,04 ± 0,01 | 2,67 ± 0,30 |

Die Proben wurden in neun Replikaten analysiert, um die Genauigkeit und Reproduzierbarkeit des Tests zu beurteilen. Zur Beurteilung der Inter-Assay-Präzision wurden die gleichen Proben an zwei aufeinanderfolgenden Tagen auf zwei verschiedenen Mikrotiterplatten analysiert. Stammlösungen von Reagenzien wurden für jeden Durchlauf frisch zubereitet. Proben der mechanisch vorbehandelten Kläranlagen- Zu- und -Abläufe wurden am 16. Oktober 2017 in drei Kläranlagen in Berlin gesammelt. Die Proben wurden vor der Analyse filtriert mit einem 0,45 µm Spritzenvorsatzfilter (PE, 25 mm Durchmesser, LLG). Neben der Messung der unverdünnten Proben wurden sie in Verdünnung gemäß den Tabellen 4 bis 7 analysiert.

**Tabelle 4**

| CBZ | LC-MS/MS [µg/L] | Verdünnung | SAFIA [µg/L] | Verdünnung | ELISA [µg/L] | Verdünnung |
|---|---|---|---|---|---|---|
| Klärwerk-1 Zulauf | 0,70 ± 0,03 | - | 0,99 ± 0,24 | 1:5 | 1,21 ± 0,07 | - |
| Klärwerk-1 Ablauf | 0,84 ± 0,05 | - | 0,86 ± 0,63 | - | 1,03 ± 0,14 | - |
| Klärwerk-2 Zulauf | 1,19 ± 0,04 | - | 1,52 ± 0,61 | 1:5 | 2,09 ± 0,25 | - |
| Klärwerk-2 Ablauf | 1,94 ± 0,11 | - | 1,41 ± 0,35 | - | 2,31 ± 0,23 | - |
| Klärwerk-3 Zulauf | 0,88 ± 0,03 | - | 1,55 ± 0,94 | 1:5 | 1,80 ± 0,24 | - |
| Klärwerk-3 Ablauf | 1,14 ± 0,06 | - | 0,90 ± 0,23 | - | 1,39 ± 0,18 | - |

**Tabelle 5**

| CAF | LC-MS/MS [µg/L] | Verdünnung | SAFIA [µg/L] | Verdünnung | ELISA [µg/L] | Verdünnung |
|---|---|---|---|---|---|---|
| Klärwerk-1 Zulauf | 123 ± 13 | 1:100 | 119 ± 50 | 1:5 | 232 ± 15,2 | 1:1000 |
| Klärwerk-1 Ablauf | 0,01 ± 0,002 | - | 0,50 ± 0,22 | - | 0,45 ± 0,02 | 0 |
| Klärwerk-2 Zulauf | 102 ± 9 | 1:100 | 144 ± 34 | 1:5 | 172 ± 4,8 | 1:1000 |
| Klärwerk-2 Ablauf | 0,05 ± 0,01 | - | 0,55 ± 0,08 | - | 0,63 ± 0,05 | 0 |
| Klärwerk-3 Zulauf | 158 ± 14 | 1:100 | 165 ± 43 | 1:5 | 237 ± 44 | 1:1000 |
| Klärwerk-3 Ablauf | 0,11 ± 0,02 | - | 0,54 ± 0,04 | - | 0,67 ± 0,04 | 0 |

**Tabelle 6**

| DCF | LC-MS/MS [µg/L] | Verdünnung | SAFIA [µg/L] | Verdünnung | ELISA [µg/L] | Verdünnung |
|---|---|---|---|---|---|---|
| Klärwerk-1 Zulauf | 2,12 ± 0,07 | - | 1,58 ± 0,19 | 1:100 | 3,22 ± 1,34 | 1:100 |
| Klärwerk-1 Ablauf | 2,39 ± 0,02 | - | 2,98 ± 0,57 | 1:10 | 2,79 ± 0,63 | 1:100 |
| Klärwerk-2 Zulauf | 4,49 ± 0,14 | - | 3,29 ± 1,03 | 1:100 | 5,37 ± 2,38 | 1:100 |
| Klärwerk-2 Ablauf | 3,29 ± 0,06 | - | 3,47 ± 0,004 | 1:10 | 4,32 ± 1,38 | 1:100 |
| Klärwerk-3 Zulauf | 4,45 ± 0,05 | - | 3,20 ± 0,95 | 1:100 | 6,46 ± 3,35 | 1:100 |
| Klärwerk-3 Ablauf | 4,28 ± 0,02 | - | 6,95 ± 3,24 | 1:10 | 5,48 ± 1,91 | 1:100 |

**Tabelle 7**

| ILA | LC-MS/MS [µg/L] | Verdünnung | SAFIA [µg/L] | Verdünnung | ELISA [µg/L] | Verdünnung |
|---|---|---|---|---|---|---|
| Klärwerk-1 Zulauf | 6,37 | 1:100 | 5,93 ± 1,83 | 1:5 | 6,68 ± 0,25 | 1:5 |
| Klärwerk-1 Ablauf | < LOD | - | 0,01 ± 0,02 | - | 0,25 ± 0,04 | - |
| Klärwerk-2 Zulauf | 21,0 | 1:100 | 29,7 ± 2,25 | 1:5 | 23,6 ± 1,09 | 1:5 |
| Klärwerk-2 Ablauf | <LOD | - | 0,02 ± 0,04 | - | 0,21 ± 0,04 | - |
| Klärwerk-3 Zulauf | 11,8 | 1:100 | 28,8 ± 2,72 | 1:5 | 22,1 ± 1,29 | 1:5 |
| Klärwerk-3 Ablauf | <LOD | - | 0,07 ± 0,06 | - | 0,47 ± 0,03 | - |

Die Tabelle 8 zeigt die Konzentration von OTA und ZON in damit dotiertem Maismehl und die mit dem beschriebenen Assay jeweils ermittelte Konzentration. Maismehl wurden auf drei Leveln sowohl mit Ochratoxin A (OTA), als auch mit ZON dotiert. Die Proben wurden mit einem Puffer (70 % Methanol) extrahiert und mit dem partikelbasierten Immunoassay analysiert. Die Nachweisgrenze betrug 6 (µg/kg für OTA und 0,7 µg/L für ZON. Angegeben sind ebenfalls die ermittelten Wiederfindungsraten und die Variationskoeffizienten der Messung und eine Interpretation der Ergebnisse.

**Tabelle 8**

| **Probe** | **Nominale Konzentration [µg/L]** | | **Bestimmte Konzentration [µg/L]** | |
|---|---|---|---|---|
| | **OTA** | **ZON** | **OTA** | **ZON** |
| #1 | 0,3 | 0,3 | <LOD | <LOD |
| #2 | 3 | 3 | 0,6 ± 0,8 | 1,6 ± 0,4 |
| #3 | 15 | 15 | 7,1 ± 1,0 | 9,4 ± 0,6 |
| #4 | 75 | 75 | 39,6 ± 1,4 | 53,1 ± 2,2 |
| #5 | 20 | 110 | 10,7 ± 0,9 | 67,6 ± 3,2 |
| #6 | 110 | 20 | 57,1 ± 1,0 | 14,6 ± 0,3 |
| #7 | 0 | 0 | <LOD | <LOD |
| | | | | |

| **Probe** | **Wiederfindungsrate [%]** | | **CV [%]** | |
|---|---|---|---|---|
| | **OTA** | **ZON** | **OTA** | **ZON** |
| #1 | <LOD | <LOD | <LOD | <LOD |
| #2 | <LOD | 53 | <LOD | 26,7 |
| #3 | 48 | 63 | 14,7 | 6,6 |
| #4 | 53 | 71 | 3,6 | 4,1 |
| #5 | 54 | 61 | 8,3 | 4,7 |
| #6 | 52 | 73 | 1,7 | 2,4 |
| #7 | <LOD | <LOD | <LOD | <LOD |
| | | | | |

| **Probe** | **Interpretation** | | | |
|---|---|---|---|---|
| | **OTA** | **ZON** | | |
| #1 | Richtig negativ | Richtig negativ | | |
| #2 | Richtig negativ | Richtig positiv | | |
| #3 | Richtig positiv | Richtig positiv | | |
| #4 | Richtig positiv | | Richtig positiv | |
| #5 | Richtig positiv | | Richtig positiv | |
| #6 | Richtig positiv | | Richtig positiv | |
| #7 | Richtig negativ | | Richtig negativ | |

Alle Proben wurden vierfach / in Quadruplikaten analysiert. Für die Referenzanalyse wurden LC-MS/MS und ELISA verwendet. Ausführliche Methodenbeschreibungen sind nachfolgenden nochmals angegeben und aus der einschlägigen Fachliteratur (Carvalho, J. J., Weller, M. G., Panne, U., Schneider, R. J. Anal. Bioanal. Chem. 2010, 396, 2617-2628; Huebner, M., Weber, E., Niessner, R., Boujday, S., Knopp, D. Anal. Bioanal. Chem. 2015, 407, 8873-8882; Oberleitner, L., Dahmen-Levison, U., Garbe, L.-A., Schneider, R. J. Anal. Methods 2016, 8, 6883-6894; Gärtner, S., Carvalho, J.J., Emmerling, F., Garbe, L.-A., Schneider, R.J. J. Immunoassay Immunochem. 2015, 36, 233-252) bekannt.

Bestimmung von CBZ, CAF, DF und ILA mittels ELISA: Allgemeines Vorgehen: Transparente 96-Well "high binding" Mikrotiterplatten (Greiner Bio-One) wurden für alle ELISAs verwendet. Währen der Inkubation wurden die Platten auf einem Orbitalschüttler bei 750 u/min geschüttelt, dabei wurden sie mit Parafilm® M versiegelt. Zum Waschen der Platten wurden ein automatisches 96-Kanal Mikrotiterplattenwaschgerät und PBS-Waschpuffer verwendet. Die Platten wurden in einem Waschprogramm, bestehend aus drei einzelnen Waschzyklen gewaschen und danach auf Laborpapier ausgeklopft. Für alle Immunoassays wurde die HRP-Substratlösung nach Frey et al. verwendet und zu 200 µL pro Kavität aufgegeben. Sie wurde unmittelbar vor dem Signal generierenden Schritt aus Citratpuffer (22 mL), Wasserstoffperoxid (8.5 µL einer 30 %(vol/vol)igen Lösung) und TMB-Stammlösung (550 µL) hergestellt. Die Signalentwicklung wurde durch Zugaben von 100 µL 1 M H₂SO₄ nach 30 min gestoppt. Die Extinktion bei 450 nm und bei 620 nm (Referenz) wurde entweder mit dem Mikortiterplattenspektralphotometer Eon oder SpektraMax Plus 384 gemessen. Sofern nicht anders angegeben, wurden die Standards in Milli-Q-Wasser durch serielle Verdünnung der Stammlösung des Analyten in MeOH verdünnt. Eine Acht-Punkte Kalibrationskurve mit den Analytkonzentrationen 10.000 (1000) µg/L, 100 µg/L, 10 µg/L, 5 µg/L, 1 µg/L, 0,1 (0.5) µg/L, 0,01 (0,05) µg/L und 0,001 µg/L, wurde je nach Anforderung an die zu bestimmenden Proben oder analytischen Fragestellungen gewählt. Proben und Standards wurden vollständig randomisiert auf den Platten aufgetragen, um Platteneffekte zu vermeiden. Standards wurden in Triplikaten und Proben in Quadruplikaten vermessen, sofern nicht anders angegeben. Diese wurden dazu entweder in 96-Well non-Binding Mikrotiterplatten (Greiner Bio-One) oder 96-Deep-Wellplatten aus Glas vorgelegt und das für den ELISA benötigte Probenvolumen wurde daraus mittels einer Achtkanalpipette oder des Liquidator entnommen. Auf jeder einzelnen Mikrotiterplatte wurden jeweils acht verschiedene Kalibratoren vermessen, um eine Kalibrationskurve zu generieren. Kalibratoren und Standards wurden einem Ausreißertest nach Grubbs mit einem Signifikanzlevel von α = 0,05 unterzogen; als Ausreißer identifizierte Messwerte wurden in nachfolgenden Berechnungen ausgeschlossen. Zur Generierung der Kalibrationskurve wurde die Differenz der Extinktionswerte von 450 nm und 620 nm gegen die Konzentration des Analyten auf einer logarithmischen Skala aufgetragen und die vier-parametrische Kalibrationsfunktion wurde in Origin 9.0 mithilfe eines *"Least-Squares"-* Algorithmus unter Verwendung von instrumenteller Gewichtung angepasst.

Direkte kompetitive ELISAs zur Bestimmung von CBZ, CAF und ILA: Die ELISAs wurden in Anlehnung an Oberleitner et al., Carvalho et al. und Baldofski et al. durchgeführt. Zunächst wurden die Kavitäten der Mikrotiterplatten mit polyklonalen sekundären Antikörpern beschichtet, diese wurden in einer Konzentration von 1 mg/L in PBS und einem Volumen von 200 µL/Well für mindestens 18h inkubiert. Nach einem Waschschritt wurde in jede Kavität mit 200 µL des entsprechenden primären Antikörpers in PBS (Maus-anti-CBZ IgG: 7,5 µg/L; Maus-anti-CAF IgG: 27,2 µg/L; Verdünnung des Kaninchen-anti-ILA-Serums 1:50.000) gefüllt und eine Stunde inkubiert. Nicht gebundene Antikörper wurden durch einen weiteren Waschschritt entfernt. Danach wurden 150 µL der Probe oder des Standards und sofort danach 50 µL des entsprechenden Tracers in TRIS-Puffer (1,3-Dimethyl-7-(carboxypentyl)xanthin gekoppelt an Meerrettichperoxidase (HRP) 4,1 ng/L) oder Probenpuffer (Carbamazepintriglycin gekoppelt an HRP, 8,3 ng/L und ILA-HRP 1:100.000 verdünnt) aufgegeben und 30 min (CAF, CBZ) oder 60 min (ILA) inkubiert. Nach einem finalen Waschritt wurde die Signalentwicklung und Auswertung wie oben beschrieben durchgeführt.

Indirekt kompetitiver ELISA zur Bestimmung von DCF: Die Kavitäten der Mikrotiterplatten wurden zunächst mit 200 µL 2-[2-[(2,6-Dichlorphenyl)amino]phenyl]essigsäure-N-(5-carboxypentyl)amid gekoppelt an Apo-Transferrin (DCF-NH-(CH₂)₅-APO) in einer Konzentration von 30 µg/L in PBS für mindestens 18 h beschichtet. Nach einem Waschschritt wurden die Kavitäten mit Casein blockiert, dieses wurde als 0,1 %ige (m/m) Lösung in PBS verwendet und 60 min inkubiert. Die Platte wurde erneut gewaschen. Danach wurden 150 µL Standard oder Probe gefolgt von 50 µL des primären anti-DCF-Antikörpers, in einer Konzentration von 20 µg/L in TRIS-Puffer, in jede Kavität gegeben und für weitere 60 min inkubiert. Die Platte wurde erneut gewaschen und jede Kavität wurde mit 200 µL eines polyklonalen Ziege-anti-Maus-HRP-Antikörper befüllt. Dieser wurde dazu 1:20.000 in TRIS-Puffer verdünnt. Nach 60 min Inkubationszeit wurde die Platte zum letzten Mal gewaschen und das Signal wurde wie oben beschrieben generiert.

Bestimmung von CBZ, CAF, DCF und ILA mittels LC-MS/MS: LC-MS/MS Experimente wurden mit einem Agilent 1260 Infinity LC System (Agilent Technologies), ausgestattet mit Binärpumpe, Autosampler, Säulenofen und UV-Detektor, gekoppelt an einen Triple Quad™ 6500 (AB Sciex) Massenspektrometer, ausgeführt. Eine Kinetex C18 Vorsäule (Phenomenex) und eine Kinetex XB-C18 core/shell Säule (150 mm x 3 mm, 2,6 µm) wurden zur Separation verwendet. 10 µL Probe wurden für die Bestimmung von CBZ, CAF und DCF und 20 µL für die Bestimmung von ILA injiziert. Der Säulenofen wurde mit einer Temperatur von 55 °C betrieben und die Flussrate auf 350 µL/min eingestellt. Ein binärer Gradient aus Wasser und Methanol wurde zur Elution verwendet. Beide enthielten 10 mM Ammoniumacetat und 0.1 %(vol/vol) Essigsäure. Der Gradient wurde nach folgendem Schema verwendet: 30 % Methanol, isokratisch für 3 min, lineare Zunahme auf95 % Methanol in 9 min, isokratisch mit 95 % MeOH für weitere 6 min. Dann wurde zu 30 % Methanol in 0.5 min zurückgekehrt, das Verhältnis wurde weitere 5.5 min beibehalten.

CBZ, CAF und DCF wurden im positiven Elektrospray-Modus (ESI+) mit einer Quelltemperatur von 400 °C und einer Ionisierungsspannung von 4500 V betrieben. Zur Quantifizierung wurden folgende Massenübergänge im selected reaction monitoring (engl. für ausgewählter Reaktionsüberwachungs-) Modus (SRM) gewählt:
SRM1 (CBZ): m/z 237-7194; Kollisionsenergie (CE) 30 V; Zellenausgangspotenzial (CXP) 14 V, Entclusterungspotential 90 V, Verweilzeit 50 ms
SRM2 (CAF): m/z 195-7138; Kollisionsenergie (CE) 27 V; Zellenausgangspotenzial (CXP) 12 V, Entclusterungspotential 60 V, Verweilzeit 50 ms
SRM3 (DCF): m/z 296-7214; Kollisionsenergie (CE) 30V; Zellenausgangspotenzial (CXP) 15 V, Entclusterungspotential 60 V, Verweilzeit 50 ms
   Zur Bestimmung von ILA, wurde der negative Elektrospray Ionisierungsmodus (ESI-), mit einer Quelltemperatur von 400 °C und einer Ionisierungsspannung von 4500 V, gewählt.
SRM4 (ILA): m/z 375-7375; Kollisionsenergie (CE) -45 V; Zellenausgangspotenzial (CXP) -10 V, Entclusterungspotential -60 V, Verweilzeit 50 ms

Insbesondere wurden dabei folgende allgemeine Parameter verwendet:

| Parameter | ESI+ | ESI- |
|---|---|---|
| Curtain Gas | 35 psi | 35 psi |
| Collision Gas | 8 psi | 8 psi |
| Nebulizer Gas | 62 psi | 62 psi |
| Turbo Gas | 62 psi | 62 psi |
| Entrance Potenzial | 10V | -10 V |

Für die Bestimmung der Wiederfindungsraten der beispielhaft ausgewählten anthropogenen Marker CAF und ILA in Ablaufwasser wurden drei Ablauf-Wasserproben jeweils auf 2 verschiedenen Niveaus mit CAF und ILA versetzt, da diese Analyten nicht oder nur in geringer Menge im Ablauf-Wasser vorhanden sind (mittels LC-MS /MS bestätigt). Die durch Analytzusatz eingestellten Konzentrationen sind in **Tabelle 9** und **10** angegeben.

**Tabelle 9**

| Probe | Nominelle CAF Konzentration [µg/L] | SAFIA [µg/L] | ELISA [µ/L] | LC-MS/MS [µg/L] | Wiederfindungsrate SAFIA [%] | Wiederfindungsrate ELISA [%] | Wiederfindungsrate LC-MS/MS [%] |
|---|---|---|---|---|---|---|---|
| Klärwerk 1 Ablauf #1 | 0,40 | 0,76 ± 0,18 | 0,75 ± 0,17 | 0,34 ± 0,01 | 191 | 188 | 84 |
| Klärwerk 1 Ablauf #2 | 2,70 | 1,94 ± 0,40 | 1,91 ±0,17 | 2,31 ± 0,22 | 72 | 71 | 86 |
| Klärwerk 2 Ablauf #1 | 1,80 | 1,85 ± 0,47 | 1,81 ± 0,21 | 1,67 ± 0,13 | 103 | 100 | 93 |
| Klärwerk 2 Ablauf #2 | 0,70 | 1,02 ± 0,14 | 1,14 ± 0,15 | 0,56 ± 0,05 | 146 | 163 | 80 |
| Klärwerk 3 Ablauf #1 | 0,90 | 1,49 ± 0,23 | 1,35 ± 0,30 | 0,82 ± 0,06 | 166 | 150 | 91 |
| Klärwerk 3 Ablauf #2 | 4,50 | 4,51 ± 1,22 | 4,08 ± 1,11 | 4,20 ± 0,37 | 100 | 91 | 93 |

**Tabelle 10**

| Probe | Nominelle ILA Konzentration [µg/L] | SAFIA [µg/L] | ELISA [µ/L] | LC-MS/MS [µg/L] | Wiederfindungsrate SAFIA [%] | Wiederfindungsrate ELISA [%] | Wiederfindungsrate LC-MS/MS [%] |
|---|---|---|---|---|---|---|---|
| Klärwerk 1 Ablauf #1 | 0,80 | 1,03 ± 0,35 | 0,70 ± 0,26 | 0,86 | 129 | 87 | 108 |
| Klärwerk 1 Ablauf #2 | 6,80 | 5,47 ± 2,86 | 2,62 ± 1,67 | 20,36 | 81 | 38 | 299 |
| Klärwerk 2 Ablauf #1 | 2,30 | 1,81 ± 1,00 | 1,12 ± 0,34 | 5,05 | 79 | 49 | 219 |
| Klärwerk 2 Ablauf #2 | 1,20 | 1,12 ± 0,34 | 0,94 ± 0,12 | 2,16 | 93 | 79 | 180 |
| Klärwerk 3 Ablauf #1 | 1,50 | 1,71 ± 0,62 | 1,59 ± 0,46 | 2,63 | 114 | 106 | 176 |
| Klärwerk 3 Ablauf #2 | 0,90 | 0,66 ± 0,28 | 0,69 ± 0,04 | 1,90 | 74 | 76 | 211 |

Der gelöste organische Kohlenstoff (DOC) wurde nach DIN EN 1484 mit einem TOC VCPH-Analysator (Shimadzu, Duisburg, Deutschland) bestimmt, wobei der gelöste anorganische Kohlenstoff (TIC) vom Gesamtkohlenstoff (TC) abgezogen wurde. Alle Proben mit Analytzusatz wurden unter Verwendung einer methanolischen Stammlösung der einzelnen Analyte hergestellt.

Ergebnisse und Diskussion: Suspensions Array Immunoassay Design: Das Messprinzip des hier vorgeschlagenen SAFIA ist in Fig. 2 dargestellt. Zuerst wird eine Mischung aus zielspezifischem primärem Antikörper mit der Probe inkubiert und eine Mischung aus kodierten, mit Analyten dekorierten Mikropartikeln. Die primären Antikörper binden entweder den Analyten in Lösung oder sie binden sich an die Oberfläche der Mikropartikel. Ein Überschuss an markierten (Alexa Fluor™488 und Alexa Fluor™647, je nach Code) sekundären Antikörpern wird dann der Mischung hinzugefügt, um die partikelgebundenen primären Antikörper zu quantifizieren. Um die weitere Antikörper-Reaktion beim Auslesen zu unterdrücken, wird erfindungsgemäß eine Stopp-Lösung zugesetzt, die Formaldehyd als Vernetzungs- und Denaturierungsmittel enthält. Der gesamte Prozess findet in 96-Well-Mikrotiterplatten statt und nutzt den hohen Probendurchsatz der Batch-Verarbeitung. Die abschließende sequentielle Auslesung der einzelnen Kavitäten erfolgt in einem Durchflusszytometer: Ein Satz von zwei Lasern (488 nm und 640 nm), zwei Lichtstreuungsdetektoren in 90° (SSC) und 0° (FSC) Geometrie und zwei Fluoreszenzdetektoren (FL-1: 488 nm Anregung, 533 ± 30 nm Emission; FL-4: 640 nm Anregung, 675 ± 25 nm Emission) werden zur Dekodierung der Mikropartikel und zur Bestimmung der Fluoreszenzintensität der sekundären Antikörper Marker verwendet, die indirekt mit der Analyt-Konzentration verknüpft ist.

Selektivität des SAFIA: Die spezifische Erkennung jedes Targets (Analyts) durch seinen Antikörper ist selbstredend ein Schlüsselfaktor in multiplexen Assays. Unspezifische Bindung von Antikörpern oder eine Kreuzreaktivität der Antikörper kann zu falsch positiven Ergebnissen oder hohen Hintergrundsignalen führen. Wir konnten feststellen, dass aminofunktionalisierte Oberflächen eine hohe unspezifische Bindung von Antikörpern aufweisen, während gemischte Oberflächen, die PEG und Aminogruppen enthalten, eine unspezifische Bindung von Antikörpern an entsprechend modifizierte Silica-Oberflächen verhindern, wobei das CAF-Hapten und der Anti-CAF Antikörper als Modell verwendet wurde. Wir fanden nun heraus, dass diese gemischten Oberflächen eine spezifische Erkennung aller verwendeten Antikörper an ihrem jeweiligen Hapten ermöglichen. Die primären Antikörper binden nur dann an ihre jeweilige Hapten-modifizierten Mikropartikel, wenn sie in jeder Kombination separat inkubiert werden, wie die jeweils höheren Fluoreszenzen belegen (vgl. **Fig. 7** und **Fig. 15**).

Die Selektivität der Bindung wird auch durch eine sehr geringe Kreuzreaktivität (CR< 0,1%) für jeden Antikörper angezeigt, wenn die anderen jeweiligen Analyten als Einzelkalibratoren verwendet werden (vgl. Tabelle 2 Die sekundären Antikörper zeigen selbst bei einer hohen Konzentration von 20 µg/mL keine unspezifische Bindung an die Haptenmodifizierten Mikropartikel.

Signalstabilität während der durchflusszytometrischen Analyse: Bei herkömmlichen Immunoassays wie ELISA wird der Signalerzeugungsschritt typischerweise chemisch gestoppt, beispielsweise durch 1 mol/L H₂SO₄, um eine konstante Reaktionszeit während oder vor der photometrischen Messung einer Multiwellplatte zu erreichen. Im Gegensatz dazu werden SAFIAs in der Regel gewaschen, um überschüssige Reagenzien zu entfernen und ein stabiles Signal bei der sequentiellen Einzelkavität-Messung mittels Durchflusszytometer zu gewährleisten. Allerdings sind die Waschschritte arbeitsaufwendig und Partikelverluste sind nicht völlig auszuschließen.

Um diese Einschränkung zu überwinden, haben wir eine Stoppmethode für die SAFIA entwickelt. Das Mittel zur Fixierung und Quervernetzung (Stoppreagenz) muss zwei Anforderungen erfüllen: Erstens sollte es ungebundene Antikörper in der Lösung denaturieren, um zu verhindern, dass sie nach einer bestimmten Inkubationszeit an die Oberfläche der Mikropartikel binden. Zweitens sollte es die bereits spezifisch gebundenen Antikörper auf der Oberfläche der Mikropartikel stabilisieren oder vernetzen, um eine Dissoziation der Antikörper während der Denaturierung und des Auslesens zu verhindern. Im Allgemeinen können Aldehyde in Kombination mit einem organischen Lösungsmittel eingesetzt werden. Wir haben Formaldehyd als Stopplösung verwendet. Wie der Fig. 9 A zu entnehmen ist, wächst das Signal von SAFIA ohne Stopp-Mittel mit der Messzeit nach Start der durchflusszytometrischen Analyse allmählich an, da sich auf Grund der zeitlichen Verzögerung zwischen dem Auslesen jeder Mikrotiterplatten-Kavität von etwa 1,5 min überschüssige Antikörper an die Partikel binden. Bei Verwendung von Formaldehyd als Fixierlösung (Fig. 9B) liefern alle Messkavitäten hingegen ein konstantes Signal. Das belegt, dass überschüssige Antikörper denaturiert sind, d.h. nicht mehr an die Oberfläche binden können und gebundene Antikörper vernetzt werden, so dass eine Dissoziation verhindert wird. Während unserer Messungen ohne Formaldehydzusatz stellten wir zunächst einen Signalverlust im Zusammenhang mit einer Messung von Diclofenac während des Auslesens fest, was darauf hindeutet, dass der betreffende Antikörper extrem instabil ist. Bei Verwendung der Fixierlösungs Formaldehyd ist das Signal von Diclofenac jedoch über die Zeit stabil, was wiederum mit der Vernetzung der Antikörper auf der Oberfläche der Mikropartikel zusammenhängen kann.

Optimierung des Immunoassays und Bestimmung der Nachweisgrenzen: Um einen schnellen "Mix-and-Read-Assay" zu erzielen, haben wir die Bindungskinetik der Antikörper abgeschätzt, um möglichst kurze Inkubationszeiten zu nutzen. Darüber hinaus ist die Empfindlichkeit eines kompetitiven Immunoassays höher, wenn kurze Inkubationszeiten für die kompetitive Reaktion genutzt werden. Um die erforderliche Bindungszeit der Antikörper an die Mikropartikel abzuschätzen, inkubierten wir CAF-modifizierte Mikropartikel mit FITC-markierten Anti-CAF-Antikörpern in verschiedenen Konzentrationen und verfolgten die Bindungskinetik über die Fluoreszenz im FITC-bezogenen Kanal (FL-1) während einer kontinuierlichen Messung im Durchflusszytometer. In **Fig. 10** ist zu sehen, dass auch nach 800 s die Bindung der Antikörper noch nicht vollständig abgeschlossen ist, da die Signalintensität kein asymptotisches Verhalten zeigt. Die steilste Steigung der Kurve wird jedoch nach ca. 300 Sekunden sowie ca. 40-60 % der maximalen Signalintensität erreicht, abhängig von der Konzentration des FITC-anti-CAF Antikörpers. Unter der Annahme, dass die Bindungskinetik, d.h. die Reaktionsgeschwindigkeit der Assoziation, aller verwendeten Antikörper in der gleichen Größenordnung liegt, haben wir für den kompetitiven Reaktionsschritt eine Zeitdauer von fünf Minuten gewählt. Dies ist der beste Kompromiss zwischen einem schnellen Assay und der Signalintensität.

Auch die manuelle Handhabungszeit ist ausreichend, da lediglich etwa drei Minuten benötigt werden, um auf der gesamten 96-Well-Mikrotiterplatte einen Pipettierschritt durchzuführen. Um ein hohes Fluoreszenz-Signal aus der Bindung des sekundären Antikörpers zu erhalten, stellen wir die Inkubationszeit auf 15 Minuten ein. Bemerkenswert ist, dass mit unseren Mikropartikeln ein relativ schneller Immunoassay durchgeführt werden kann, verglichen mit handelsüblichen, z.B. Luminex™ Beads oder klassischem ELISA, bei welchen Inkubationszeiten von ca. 30 min bis zu einer Stunde pro Schritt erforderlich sind. Die Konzentrationen von Mikropartikel, sowie primären und sekundären Antikörpern sind jeweils zu optimieren. Im Allgemeinen sollte bei kompetitiven Immunoassays die Konzentration des Antigens, d.h. des Haptens auf der Oberfläche der Mikropartikel und der primären Antikörper so niedrig wie möglich sein, um eine möglichst hohe Empfindlichkeit zu gewährleisten.

Das resultierende maximale Signal sollte jedoch mindestens drei, besser fünf bis zehn Mal höher als der Hintergrund sein (S/N ≥ 3), um eine auswertbare Kalibrierkurve zu erstellen. Zuerst haben wir die Konzentrationen der Mikropartikel und die Fließgeschwindigkeit des Instruments in einem Schritt optimiert (siehe **Fig. 11**). Die Fließgeschwindigkeit im Instrument hat einen Einfluss über die hydrodynamische Fokussierung der Mikropartikel: Eine niedrigere Fließgeschwindigkeit ermöglicht es, kleinere Partikel unabhängig voneinander zu trennen und zu messen, wodurch der innere Stromdurchmesser in der Kapillare verringert wird (die sogenannte "core-size"). Ursprünglich haben wir die Einstellung 14 µL/min verwendet, um zu verhindern, dass mehr als ein Partikel gleichzeitig den Detektor passiert. Dies kann aber auch mit einer schnelleren Fließgeschwindigkeit und einer höheren Verdünnung der Mikropartikel erreicht werden, wie in Fig. 10 zu sehen ist. Dies erhöht auch die Empfindlichkeit des Immunoassays. Wir haben die Konzentration der Mikropartikel angepasst, um mindestens 2000 Mikropartikel pro Analyt (insgesamt 8000 Partikel) in 30 s zu messen, um eine statistisch robuste Anzahl von Partikeln in angemessener Analysezeit vermessen zu können. Die relative Anzahl der aggregierten Partikel in den Streudiagrammen "FSC/SSC" (**Fig. 2E**), angegeben durch den "Anteil im Gate "Size", ist bei Verwendung einer hohen Verdünnung und einer hohen Fließgeschwindigkeit von 66 µL/min gegenüber der niedrigen Fließgeschwindigkeit bis zu einer Partikelkonzentration von 6·10⁻³ % (m/v) nicht verringert (siehe **Fig. 11**). Darüber hinaus beträgt die niedrigste mögliche Partikelkonzentration 1,5 ·10⁻³ % (m/v), um insgesamt 8000 Partikel zu zählen, verglichen mit der notwendigen Konzentration von mindestens 4·10⁻³ % (m/v), wenn man eine Fließgeschwindigkeit von 14 µL/min verwendet. Abschließend haben wir eine Fließgeschwindigkeit von 66 µL/min und eine Partikelkonzentration von 1,5·10⁻³ % (m/v) gewählt, um eine möglichst hohe Verdünnung zu erreichen. Schließlich wurden die Konzentrationen der Antikörper angepasst, bis das Signal-Rausch-Verhältnis zwischen 5 und 10 lag. Für den sekundären Antikörper wählten wir eine Konzentration von 2 µg/mL, wie vom Lieferanten vorgeschlagen. Eine weitere Verdünnung dieses Antikörpers führt zu weniger intensiven Signalen (Daten nicht gezeigt).

Typische Kalibrierkurven des SAFIA für die vier Marker nach einer Optimierung sind in **Fig. 1** dargestellt. Für die Schätzung der Nachweisgrenzen (LOD) haben wir das durch Ekins (Ekins, R. Ligand Quart. 1981, 4, 33-44) eingeführte Präzisionsprofil verwendet, welches 30% relativen Konzentrationsfehler zulässt, wobei die LOD 140 ± 40 ng/L für CBZ beträgt, was dreimal mehr als die LOD für den ELISA (50 ng/L) ist. Für den OTA- und ZON-SAFIA wurden nach dem gleichen Verfahren Nachweißgrenzen von 6 µg/L (OTA) und 0,7 µg/L (ZON) bestimmt.

Er ist jedoch niedriger als der LOD des waschfreien homogenen Fluoreszenzpolarisations-Immunoassays (660 ng/L), was einem Faktor von fünf entspricht. Die CAF LOD von 180 ± 110 ng/L ist vergleichbar mit indirekten CAF-ELISAs: Je nach Markierung und Auslesung wurden LODs zwischen 83 ng/L und 233 ng/L beobachtet (Grandke, J., Oberleitner, L., Resch-Genger, U., Garbe, L.-A., Schneider, R.J. Anal. Bioanal. Chem. 2013, 405, 1601-1611) Mit dem DCF SAFIA wurde eine Nachweisgrenze von 4 ± 3 ng/L erreicht, die auch mit dem ELISA (7,8 ng/L) (Huebner, M., Weber, E., Niessner, R., Boujday, S., Knopp, D. Anal. Bioanal. Chem. 2015, 407, 8873-8882). vergleichbar ist und um einen Faktor von zehn höher empfindlich ist als ein Aufwärtskonversions-ULISA (50 ng/L) (Hlaváček, A., Farka, Z., Hübner, M., Horňáková, V., Němeček, D., Niessner, R., Sklädal, P., Knopp, D., Gorris, H. H. Anal. Chem. 2016, 88, 6011-6017) und ein mikropartikelgestützter Chemilumineszenztest (50 ng/L) (Shi, J., Xu, M., Tang, Q, Zhao, K., Deng, A., Li, J Spectrochim. Acta, Part A 2018, 191, 1-7). Die ILA SAFIA zeigt eine LOD von 310 ± 70 ng/L, was um einen Faktor von 3 weniger empfindlich ist als der ILA-ELISA (LOD: 90 ng/L) (Gärtner, S., Carvalho, J.J., Emmerling, F., Garbe, L.-A., Schneider, R.J. J. Immunoassay Immunochem. 2015, 36, 233-252). Die etwas höhere Sensitivität in CBZ und ILA-ELISA könnte durch das direkte kompetitive Format verursacht sein, bei dem die die Antikörper direkt an der Festphase immobilisiert sind. So fanden Grandke et al. (Grandke, J., Oberleitner, L., Resch-Genger, U., Garbe, L.-A., Schneider, R. J. Anal. Bioanal. Chem. 2013, 405, 1601-1611) heraus, dass für CAF das indirekte Immunoassay-Format niedrigere LODs hatte als das direkte Format. Dennoch ist die Sensitivität für die Abwasseranalyse mehr als ausreichend im Vergleich zu den Konzentrationen der üblicherweise gefundenen Marker (Bahlmann, A., Carvalho, J. J., Weller, M. G., Panne, U., Schneider, R. J. Chemosphere 2012, 89, 1278-1286; Vieno, N., Sillanpaa, M. Environ. Int. 2014, 69, 28-39; Baldofski, S., Hoffmann, H., Lehmann, A., Breitfeld, S., Garbe, L.-A., Schneider, R.J. J. Environ. Manage. 2016, 182, 612-619).

Auswahl an Farbstoffen für die Kodierung der Mikropartikel und sekundäre Antikörper. Es ist möglich, die Fluoreszenz-Kodierung von Mikropartikel mit mindestens zwei verschiedenen Farbstoffen zu erreichen: "dye 1 green", der mit dem blauen Laser des Durchflusszytometers (488 nm) angeregt werden kann, wobei die Emission im grünen Kanal F1-1 (533 ± 30 nm) erfasst wird und "dye 2 red", der durch den roten Laser (640 nm) angeregt wird, wobei die Emission im Kanal bei 675 ± 25 nm (F1-4) erfasst wird. Wir haben Alexa Fluor™647 (Exc.: 640 nm, Em.: 675±25 nm, FL-4) als Signalfarbstoff für "dye 1 green" kodierte Mikropartikel und Alexa Fluor™488 (Exc: 488 nm, Em.: 533 ± 30 nm, FL-1) als Signalfarbstoff für "dye 2 red" kodierte Mikropartikel verwendet. Zur Struktur der beiden BODIPY-Farbstoffe vgl. Fig. 6. Zu Absorptions- und Emissionsspektren wurde an anderer Stelle berichtet (Sarma, D., Carl, P., Climent, E., Schneider, R.J., Rurack, K. ACS Appl. Mater. Interfaces 2019, 11, 1321-1334).

Um den Einfluss des Signalfarbstoffs auf die Empfindlichkeit des Immunoassays zu vergleichen, wurden Kalibrierkurven unter den gleichen Bedingungen für beide sekundäre Antikörper aufgenommen. Die IC₅₀-Werte, eine Schätzung für die Sensitivität eines Immuntests **(Tabelle 11**) sind für ILA (|t| = 1,344; p = 0,242), DCF (|t| = 0,522; p = 0,618) und CAF (|t| = 2,286; p = 0,079) nicht signifikant verschieden. Die nachfolgende Tabelle 1 zeigt die IC₅₀-Werte der Kalibrierkurven für die vier Analyte unter Verwendung der jeweils entsprechenden unterschiedlichen für die Kodierung ("dye 2 red" und "dye 1 green") und Signalgebung (Alexa Fluor™488 und Alexa Fluor™647) genutzten Fluoreszenzfarbstoffe.

**Tabelle 11**

| **Analyt** | **IC₅₀ "Dye 1 Green" [µL/L]** | **IC₅₀ "Dye 2 Red" [µg/L]** |
|---|---|---|
| CBZ | 2,98 ± 0,08 | 1,13 ± 0,07 |
| CAF | 1,79 ± 0,46 | 1,72 ± 0,16 |
| DCF | 0,05 ± 0,02 | 0,05 ± 0,02 |
| ILA | 2,41 ± 1,13 | 1,39 ± 0,72 |

Der Marker des sekundären Antikörpers hat keinen Einfluss auf die Empfindlichkeit dieser Immunoassays. Nur für den CBZ-Assay wurde ein signifikanter Unterschied (|t| = 10.356; p= 0,0009) beobachtet. Der CBZ-Assay mit Alexa Fluor™488-markierte sekundäre Antikörper ist dreimal empfindlicher als der mit Alexa FluorTM647-markierte sekundäre Antikörper (vgl. Tabelle 1).

Schätzung von Kreuzreaktivitäten (CRs) und deren möglichen Auswirkungen auf die Abwasseranalyse durch SAFIA: Für alle verwendeten Antikörper wurden CRs in ELISA berichtet (Carvalho, J. J., Weller, M. G., Panne, U., Schneider, R. J. Anal. Bioanal. Chem. 2010, 396, 2617-2628; Huebner, M., Weber, E., Niessner, R., Boujday, S., Knopp, D. Anal. Bioanal. Chem. 2015, 407, 8873-8882; Gärtner, S., Carvalho, J. J., Emmerling, F., Garbe, L.-A., Schneider, R.J. J. Immunoassay Immunochem. 2015, 36, 233-252). Wir haben die drei Verbindungen mit dem höchsten CR-Wert im ELISA und/oder der höchsten Umweltrelevanz für Tests in SAFIA ausgewählt, da sie eine mögliche Überschätzung bei der Messung von realen Abwasserproben verursachen könnten. Im Allgemeinen sind die CRs im Vergleich zu ELISA in der gleichen Größenordnung, unterscheiden sich bekanntermaßen (Weller, M. G. Anal. Chem. Insights 2018, 13) aber leicht zwischen den verschiedenen Immunoassay-Formaten. Alle CRs sind in **Tabelle 2** zusammengefasst. Die Kreuzreaktivität (CR) lag bei Substanzen, die auf CAF, DCF und ILA getestet wurden, unter 10%. Überschätzungen bei den realen Stichproben sind daher nicht zu erwarten. Für CBZ wurde der höchste CR von 118% für Carbamazepin-10,1-L-epoxid bestimmt, ähnlich wie bei ELISA (Oberleitner, L., Dahmen-Levison, U., Garbe, L.-A., Schneider, R. J. Anal. Methods 2016, 8, 6883-6894). Diese Verbindung ist jedoch nur in geringer Menge im Abwasser enthalten, da es sich um einen kleinen Metaboliten von CBZ (Bahlmann, A., Brack, W., Schneider, R. J., Krauss, M. Wat. Res. 2014, 57, 104-114) handelt, so dass nur eine geringfügige Überschätzung zu erwarten ist.

Genauigkeits- und Präzisionsbewertung mit Hilfe von gespikten Leitungswasserproben: Für eine erste Beurteilung der Genauigkeit und Präzision wurden fünf künstliche Proben mit Leitungswasser als Matrix angesetzt. Die Proben wurden auf zufällig ausgewählten Konzentrationsniveaus, entsprechend dem Messbereich für jeden Analyten, gespikt und jede Probe wurde mittels SAFIA in neun Replikaten analysiert. Zur Beurteilung der Inter-Assay-Präzision wurde der Assay mit dem gleichen Probensatz auf zwei Mikrotiterplatten an zwei aufeinanderfolgenden Tagen durchgeführt. Die Spike-Konzentrationen, Messwerte und Wiederfindungsraten sind in **Tabelle 3** und **Tabelle 12** zu finden.

**Tabelle 12. Wiederfindungsrate (%) and intra- und inter-Assay Variationskoeffizient (CV) (%) der 5 bespikten Trinkwasserproben.**

| **Probe** | **Wiederfindungsrate [%]** | | | | **Intra-Assay CV[%]** | | | | **Inter-Assay CV [%]** | | | |
|---|---|---|---|---|---|---|---|---|---|---|---|---|
| Analyt | CBZ | CAF | DCF | ILA | CBZ | CAF | DCF | ILA | CBZ | CAF | DCF | ILA |
| #1 | 101 | 116 | 130 | 94 | 22 | 13 | 12 | 7 | 30 | 26 | 24 | 28 |
| #2 | 106 | 115 | 100 | 114 | 18 | 28 | 18 | 23 | 28 | 30 | 19 | 31 |
| #3 | 105 | 116 | 96 | 87 | 30 | 38 | 26 | 14 | 33 | 41 | 42 | 27 |
| #4 | 111 | 117 | 144 | 86 | 24 | 29 | 13 | 5 | 34 | 35 | 28 | 31 |
| #5 | 96 | 111 | 90 | 101 | 26 | 21 | 23 | 11 | 39 | 28 | 41 | 14 |
| **Mittelwert** | **104** | **115** | **112** | **96** | **24** | **26** | **18** | **12** | **33** | **32** | **31** | **26** |

Eine ungespikte Probe wurde mittels SAFIA erfolgreich als negativ bewertet. Ebenso wurde eine ungespikte Maismehlprobe erfolgreich als negativ bewertet. Die Wiederfindungsraten (recovery range) und CVs für die fünf Proben sind in
**Fig. 8** dargestellt. Es ist zu erkennen, dass sich die mittlere Wiederfindungsrate für jeden Analyten in einem akzeptablen Bereich zwischen 96% und 115% bewegt, was mit anderen haptenbasierten Suspensionsarrays unter Verwendung von Luminex™ oder verwandten kommerziellen Mikropartikel (Wang, Y., Ning, B., Peng, Y., Bai, J., Liu, M., Fan, X., Sun, Z., LV, Z., Zhou, C., Gao, Z. Biosens. Bioelectron. 2013, 41, 391-396; Fraga, M., Vilariño, N., Louzao, M. C., Rodriguez, P., Campbell, K., Elliott, C. T., Botana, L. M. Anal. Chem. 2013, 85, 7794-7802; Guo, Y., Tian, J., Liang, C., Zhu, G., Gui, W. Microchim. Acta 2013, 180, 387-395; Liu, N., Gao, Z., Ma, H., Su, P., Ma, X., Li, X., Ou, G. Biosens. Bioelectron. 2013, 41, 710-716 ; Tsoi, T.-H.; Wong, W.-T. Anal. Methods 2015, 7, 5989-5995) vergleichbar ist **(****Fig. 5-A****, Tab.11)** und eine hohe Genauigkeit des Tests belegt (vgl. Fig. 6A).

Die Intra-Assay-Präzision ist, abhängig vom gemessenen Analyten, sehr gut für ILA (CV des Mittelwertes: 13%), gut für DCF und CBZ (CV des Mittelwertes < 24%) und akzeptabel für CAF (CV = 28%, vgl. Tab. 11). Die Intra-Assay Präzision war ebenfalls sehr gut für OTA (Mittlerer CV = 7%) und ZON (Mittlerer CV = 9%). Die Inter-Assay-Präzision ist erwartungsgemäß etwas geringer, die mittleren Inter-Assay-CVs reichen von 26% (ILA) bis 33% (CBZ). Das passt zu den berichteten Messgenauigkeiten von etwa 30%, die bei anderen Hapten-Mikropartikel-basierten Assays beobachtet wurden. Eine vollständige Automatisierung (Flüssigkeitshandhabung und Auslesen) des Assays könnte diesen Wert verbessern (Jolley, M. E., Stroupe, S. D., Schwenzer, K. S., Wang, C. J., Lusteffes, M., Hill, H. D., Popelka, S. R., Holen, J. T., Kelso, D. M. Clin. Chem. 1981, 27, 1575-1579). Auch die multivariate Datenanalyse könnte die Präzision erhöhen, wie wir bereits zuvor für ein fluoreszenzbasiertes Microarray zur Detektion von CAF und CBZ berichtet haben (Carl, P., Schneider, R.J. Procedia Eng. 2015, 120, 501-506).

Analyse der Kläranlagen Zufluss- und Abfluss- Proben von SAFIA und Vergleich mit ELISA und LC-MS/MS: Für die Bestimmung der Markersubstanzen in realen Zuflussund Abwasserproben, die aus drei verschiedenen Kläranlagen in Berlin entnommen wurden, wurde die Anwendbarkeit des beschriebenen SAFIA untersucht. Zum Vergleich wurden die sechs Proben mittels SAFIA und ELISA analysiert. Als Referenzmethode wurde LC-MS/MS gewählt. Vor der Analyse der Proben waren keine weiteren Probenvorbereitungsschritte als Filtration und Verdünnung erforderlich. Die ermittelten Konzentrationen, die Wiederfindungsraten unter Verwendung des LC-MS/MS-Ergebnisses als Referenzkonzentration und die Variationskoeffizienten (CV) sind in den nachfolgenden Tabellen zusammengestellt.

**Tabelle 13. Wiederfindungsrate (%) and Variationskoeffizienten (CV) für den CBZ SAFIA und CBZ ELISA, bezogen auf die mittels LC-MS/MS ermittelten Werte.**

| CBZ | Wiederfindungsrate SAFIA | Wiederfindungsrate ELISA | CV SAFIA | CV ELISA |
|---|---|---|---|---|
| | [%] | [%] | [%] | [%] |
| Klärwerk-1-Zulauf | 141 | 173 | 25 | 6 |
| Klärwerk-1-Ablauf | 102 | 122 | 73 | 13 |
| Klärwerk-2-Zulauf | 127 | 175 | 40 | 12 |
| Klärwerk-2-Ablauf | 73 | 119 | 25 | 10 |
| Klärwerk-3-Zulauf | 177 | 205 | 60 | 13 |
| Klärwerk-3-Ablauf | 79 | 122 | 26 | 13 |

**Tabelle 14. Wiederfindungsrate (%) und Variationskoeffizienten für den CAF SAFIA und CAF ELISA, bezogen auf die mittels LC-MS/MS ermittelten Werte.**

| CAF | Wiederfindungsrate SAFIA | Wiederfindungsrate ELISA | CV SAFIA | CV ELISA |
|---|---|---|---|---|
| | [%] | [%] | [%] | [%] |
| Klärwerk-1-Zulauf | 96 | 188 | 42 | 7 |
| Klärwerk-2-Zulauf | 141 | 170 | 23 | 3 |
| Klärwerk-3-Zulauf | 105 | 150 | 26 | 19 |

**Tabelle 15. Wiederfindungsrate (%) und Variationskoeffizienten für den DCF SAFIA und DCF ELISA, bezogen auf die mittels LC-MS/MS ermittelten Werte.**

| DCF | Wiederfindungsrate SAFIA | Wiederfindungsrate ELISA | CV SAFIA | CV ELISA |
|---|---|---|---|---|
| | [%] | [%] | [%] | [%] |
| Klärwerk-1-Zulauf | 75 | 152 | 12 | 42 |
| Klärwerk-1-Ablauf | 124 | 117 | 19 | 23 |
| Klärwerk-2-Zulauf | 73 | 120 | 31 | 44 |
| Klärwerk-2-Ablauf | 106 | 131 | <1 | 32 |
| Klärwerk-3-Zulauf | 72 | 145 | 30 | 52 |
| Klärwerk-3-Ablauf | 162 | 128 | 47 | 35 |

**Tabelle 16. Wiederfindungsrate (%) und Variationskoeffizienten für den ILA SAFIA und ILA ELISA, bezogen auf die mittels LC-MS/MS ermittelten Werte.**

| ILA | Wiederfindungsrate SAFIA | Wiederfindungsrate ELISA | CV SAFIA | CV ELISA |
|---|---|---|---|---|
| | [%] | [%] | [%] | [%] |
| Klärwerk-1-Zulauf | 93 | 105 | 31 | 4 |
| Klärwerk-2-Zulauf | 141 | 112 | 8 | 5 |
| Klärwerk-3-Zulauf | 243 | 187 | 9 | 6 |

CBZ wurde in allen Proben nachgewiesen, was darauf hindeutet, dass CBZ nicht durch das Belebtschlammverfahren eliminiert wird. Die von SAFIA ermittelte mittlere Wiederfindungsrate für CBZ von 116 ±40% war viel besser als die im CBZ-ELISA beobachtete (153 ± 36%). Auch in der Kläranlage wurde DCF, wie erwartet gefunden, Hier stimmen die SAFIA-Ergebnisse mit den LC-MS/MS-Ergebnissen überein, was sich in einer besseren Wiederfindungsrate (102 ± 36%) als bei ELISA (132 ± 19%) zeigt. Während ELISA zu einer Überschätzung von DCF und CBZ neigt, weist SAFIA im Vergleich zu LC-MS/MS nur geringe Über- und Unterschätzungen auf.

CAF wurde in einer großen Menge von bis zu 158 pg/L nachgewiesen, wird aber - wie unsere Analysen zeigen - zu > 99% eliminiert. Die Analyse von Kläranlagenproben für CAF ergab auch für den hier beschriebenen SAFIA bessere Wiederfindungsraten als für ELISA (114 ± 24% im Vergleich zu 169 ± 19%). Da CAF nur in geringen Mengen (< 105 ng/L) im Abwasser der Kläranlage nachgewiesen wurde, wurde die CAF-Konzentration der Proben jeweils auf zwei unterschiedliche CAF-Niveaus eingestellt (vgl. Tabelle 8).

Beide Immunoassays detektierten CAF genau, mit einer mittleren Wiederfindungsrate von 130 ± 45% (SAFIA) und 127 ± 46% (ELISA). Dies zeigt, dass natürliche organische Stoffe mit einem DOC-Gehalt von 17,5 bis 26,8 mg/L (siehe **Tabelle 17**), die im Kläranlagen-Effluent vorkommen, die Immunoassay-Messung nicht beeinflussen.

**Tabelle 17.**

| Probe | Gesamter gelöster Kohlenstoffgehalt [ma/L] | Gelöster anorganischer Kohlenstoff [ma/L] | Gelöster organischer Kohlenstoff [ma/L] |
|---|---|---|---|
| Klärwerk-1-Zulauf | 203,1 ± 1,9 | 97,3 ± 1,9 | 105,7 ± 2,2 |
| Klärwerk-1-Ablauf | 75,8 ± 0,1 | 58,3 ± 0,8 | 17,5 ± 0,6 |
| Klärwerk-2-Zulauf | 211,4 ± 0,8 | 110,5 ±1,3 | 101,0 ± 1,3 |
| Klärwerk-2-Ablauf | 84,5 ± 0,3 | 57,7 ± 0,2 | 26,8 ± 0,3 |
| Klärwerk-3-Zulauf | 213,1 ± 2,5 | 102,5 ± 1,6 | 110,6 ± 2,4 |
| Klärwerk-3-Ablauf | 83,6 ± 0,9 | 58,8 ± 0,7 | 24,8 ± 0,9 |

Die bessere Genauigkeit von SAFIA könnte auch mit dem indirekt-kompetitiven Assay-Format erklärt werden: Im direkten ELISA-Format steht der Enzymtracer in Kontakt mit der Matrix und kann durch Matrixverbindungen deaktiviert werden. Vorteilhafterweise ist diese Deaktivierung bei dem hier vorgeschlagenen SAFIA nicht möglich, da zur Signalerzeugung Fluorophore verwendet werden und die Fluoreszenz-Eigenschaften nicht verändert werden, wie durch diese Ergebnisse belegt. Ähnliche Effekte wurden bereits berichtet (Grandke, J., Oberleitner, L., Resch-Genger, U., Garbe, L.-A., Schneider, R.J. Anal. Bioanal. Chem. 2013, 405, 1601-1611). Wie durch alle Methoden belegt, wurde ILA vollständig abgebaut. Für die Proben des Kläranlagen-Zustromes wurden Konzentrationen von 6,4 µg/L bis 21 µg/L gefunden. Während die beobachteten Konzentrationen von ILA im Zustrom der Kläranlage 1 für SAFIA, ELISA und LC-MS /MS sehr ähnlich waren, wurden Zustrom-Proben aus den Kläranlagen 2 und 3 von den Immunoassays mit einem Maximalfaktor von 2 überschätzt, wobei die Überschätzung durch ELISA etwas geringer ausfiel. Unterschiedliches Verhalten des ELISA mit diesem Antikörper in Zustrom-Proben verschiedener Kläranlagen wurde zuvor bereits berichtet (Baldofski, S., Hoffmann, H., Lehmann, A., Breitfeld, S., Garbe, L.-A., Schneider, R.J. J. Environ. Manage. 2016, 182, 612-619). Das kann auch durch eine Variation der Konzentration und Zusammensetzung von Matrixkomponenten und/oder unbekannte Kreuzreaktionen verursacht sein. Ähnliche Trends für beide Immunoassays unterstützen diese Theorie: Die polyklonalen anti-ILA Antikörper scheinen durch diese Probenmaterialien betroffen zu sein, während andere Antikörper (CBZ, CAF, DCF) nicht betroffen sind, was durch eine erhöhte Genauigkeit angezeigt wird. In Abfluss-Proben lagen die ILA-Konzentrationen für alle Methoden unter den LODs.

Um die Genauigkeit für beide Immunoassays beurteilen zu können, wurden deshalb die Abwasserproben auf verschiedenen Niveaus mit Analyt versetzt. Hierbei zeigte SAFIA im Vergleich zu ELISA (73 ±25%) überlegene (95 ± 22%) mittlere Wiederfindungsraten. Bemerkenswert ist, dass SAFIA und ELISA bei ILA in diesen Probentypen deutlich bessere Wiederfindungsraten ergaben als LC-MS/MS, für die zuvor eine "geringe Reproduzierbarkeit" berichtet wurde (Baldofski, S., Hoffmann, H., Lehmann, A., Breitfeld, S., Garbe, L.-A., Schneider, R.J. J. Environ. Manage. 2016, 182, 612-619).

Ziel der vorliegend beschriebenen Verfahren ist die Bereitstellung einer Multiplex-Detektionsmethode zur Bestimmung repräsentativer anthropogenen Marker und Toxine, hier belegt durch die beispielhaft ausgewählten Verbindungen Carbamazepin (CBZ), Coffein (CAF), Diclofenac (DCF) und Isolithocholsäure (ILA) im Abwasser. Unser Ansatz, Mikropartikel, umfassend einen Polystyrolkern und eine diesen umgebende Siliziumdioxid-Schale zur Verankerung von Haptenen zu verwenden, gestattet die Bereitstellung eines Suspension-Array-Fluoreszenz-Immunoassays (SAFIA).

In früheren Arbeiten konnte gezeigt werden, dass durchflusszytometrische Immunoassays bessere Signale liefern, wenn Haptene mit Trägerproteinen (z.B. BSA oder OVA) konjugiert wurden und solche, die auf z.B. Luminex® carboxylierten Mikropartikel (Anderson, G. P., Kowtha, V. A., Taitt, C. R. Toxins 2010, 2, 297-309) immobilisiert wurden. Wir fanden jedoch heraus, dass die direkte Immobilisierung von Haptenen (über eine NHS-Chemie) zu guten Signalintensitäten führt und damit empfindliche Immunoassays ermöglicht. Dies verkürzt auch die für die Materialvorbereitung erforderliche Zeit, da auf langwierige Biokonjugationsschritte verzichtet werden kann. Wie demonstriert, können erfindungsgemäß die Vorteile von Haptenen mit COOH-Anteil, die oft verfügbar sind, genutzt werden, da sie für die Herstellung der betreffenden Immunogene benötigt werden (Bahlmann, A., Weller, M. G., Panne, U., Schneider, R. J. Anal. Bioanal. Chem. 2009, 395, 1809-1820; Carvalho, J. J., Weller, M. G., Panne, U., Schneider, R. J. Anal. Bioanal. Chem. 2010, 396, 2617-2628). Vorteilhaft sind die vorgeschlagenen Mikropartikel vergleichsweise stabiler als Hapten-Protein-Konjugate.

Die vorgeschlagene Verwendung von PEGylierten Oberflächen erlaubt es, eine unspezifische Bindung von Antikörpern vermeiden und mit einem einfachen Protokoll ohne Waschschritte eine hohe Selektivität zu erreichen. Die erfindungsgemäß vorgeschlagene Verwendung von Formaldehyd in einer Fixierlösung führt zu stabilen Signalen und gestattet eine chargenweise Auslesung mit hoher Genauigkeit auf Mikrotiterplattenbasis. Der somit ermöglichte "Mix-and-Read" - Ansatz erlaubt es, eine kurze Zeit bis zum Ergebnis zu erzielen und erfordert lediglich grundlegende experimentelle Fähigkeiten und technische Ausrüstung. Der Einsatz einer automatisierten, mikrotiterplattenbasierten Auslesung mittels Durchflusszytometer ermöglicht den erwünscht hohen Probendurchsatz. Wir untersuchten auch den Einfluss des Labels auf die Empfindlichkeit unter Verwendung von Mikropartikeln, die in verschiedenen Spektralbereichen kodiert waren. Hierbei erwies sich, dass die Empfindlichkeit für die meisten unserer Analyten unabhängig von dem verwendeten Fluoreszenzmarker ist. Nur für CBZ wurde eine leichte Verbesserung der Empfindlichkeit beobachtet, wenn für den FITC-Kanal FL-1 (hier: Alexa Fluor™488) geeignete Label verwendet werden.

Im Allgemeinen erfüllen die Empfindlichkeitskriterien die Anforderungen an den Nachweis der eingangs bezeichneten anthropogenen Markersubstanzen im Abwasser. Die gute Genauigkeit wurde an Hand von mit Analyt versetzten Leitungswasserproben und durch die Analyse von realen Abwasserproben belegt, was darauf hindeutet, dass die Methode beispielsweise zur Überwachung der Schadstoffbeseitigungseffizienz von Kläranlagen in einem Prozessanalyseverfahren (PAT) oder zum Nachweis einer Abwassereinleitung in natürliche Gewässer einsetzbar ist. Der beschriebene SAFIA konnte alle vier Marker mit akzeptabler Präzision und Genauigkeit ohne eine vorausgehende Probenreinigung auch im Zustrom-Abwasser von Kläranlagen mit DOC-Gehalten von mehr als 100 mg/L bestimmen. Die Matrixstabilität und Genauigkeit des beschriebenen SAFIA ist bei der Analyse von realen Abwasserproben den klassischen ELISAs überlegen.

Analyse von mit OTA und ZON bespikten Proben: : Die Analyse der mit OTA und ZON dotierten Proben, also sowohl OTA als auch ZON enthaltenden Agrarrohstoffe zeigt beispielhaft, dass verschiedene Toxine in Extrakten daraus (hier: Mais) simultan nachgewiesen werden können. Proben, deren Gehalt an OTA mehr als 6 µg/L betrug, wurden richtig positiv getestet. Dies entspricht einem OTA Gehalt von 18 µg/kg in einer Agrarrohstoffprobe, wenn diese nach dem beschriebenen Verfahren extrahiert wird. Proben, deren Gehalt unter der Nachweißgrenze lagen wurden richtig negativ auf OTA getestet. Ebenso konnte für ZON festgestellt werden, dass Proben, die mit mehr als 0,7 µg/L (LOD) bespikt wurden richtig positiv getestet wurden. Dies entspricht einem ZON Gehalt von 2,1 µg/kg in einer Agrarrohstoffprobe, wenn diese nach dem beschriebenen Verfahren extrahiert wird. Proben mit kleinerem Gehalt wurden richtig negativ getestet. Eine nicht-dotierte Probe wurde auf OTA und ZON richtig negativ getestet, vgl. Tabelle 8.

Die vorstehende Offenbarung wird beispielhaft in Form der nachfolgend benannten Aspekte zusammengefasst:
1. Verfahren zur Herstellung einer Mischung von Kern/Schale-Mikropartikeln (1) zur simultanen Bestimmung verschiedener Analyte in einer Umweltprobe und/oder in einer Agrarrohstoffprobe, umfassend:
   - Bereitstellen von zwei Kohorten (1.1, 1.2) von Kern/Schale Mikropartikeln (1) umfassend: einen Polymerkern (2), der einen Luminophor (6) aufweist, und eine den Polymerkern (2) umschließende Silikat-Schale (3);
      wobei sich die erste und die zweite Kohorte (1.1, 1.2) hinsichtlich einer Art und/oder einer Konzentration des Luminophors (6) und/oder hinsichtlich eines mittleren Durchmessers und/oder hinsichtlich einer mittleren Dichte der Kern/Schale-Mikropartikel (1) unterscheiden;
   - Koppeln von zumindest zwei unterschiedlichen funktionellen Gruppen (4, 5) an die Silikat-Schale (3) der zwei Kohorten (1.1, 1.2) von Kern/Schale-Mikropartikeln (1),
      wobei eine erste der zumindest zwei unterschiedlichen funktionellen Gruppen (4, 5) ausgewählt ist unter einer -NH₂, -COOH, -CH(O), -NC, -C-SH, -CS-OH, -SO₂-OH, -SCN, -NCS oder -NCO - Gruppe (4); und eine zweite der zumindest zwei unterschiedlichen funktionellen Gruppen (4, 5) ausgewählt ist unter einem Polyethylenglycol (5), einem Sulfobetain (5) und einem Peptid (5) umfassend eine Aminosäuresequenz, die zumindest eine Sequenz von fünf Aminosäuren der Aminosäuresequenzen CYSYSYS oder CRERERE umfasst;
   - Koppeln eines ersten anthropogenen Markers oder eines ersten Toxins oder eines den ersten anthropogenen Marker oder das erste Toxin repräsentierenden ersten Haptens an die erste funktionelle Gruppe (1) von Kern/Schale-Mikropartikeln (1) der ersten Kohorte (1.1);
   - Koppeln eines zweiten anthropogenen Markers oder eines zweiten Toxins oder eines den zweiten anthropogenen Marker oder das zweite Toxin repräsentierenden zweiten Haptens an die erste funktionelle Gruppe (1) von Kern/Schale-Mikropartikeln (1) der zweiten Kohorte (1.2); und
   - Vereinen und Mischen zumindest von Teilmengen der ersten (1.1) und der zweiten Kohorte (1.2) von Kern/Schale-Mikropartikeln (1).
2. Verfahren nach Aspekt 1, wobei die anthropogenen Marker ausgewählt sind unter: Carbamazepin (CBZ), Coffein (CAF), Coprostanol, Cholesterol, Diclofenac (DCF), Isolithocholsäure (ILA), Kreatinin, Cotinin, Atenolol, Bisoprolol, Celiprolol, Clofibrinsäure, Diazepam, Dihydrocodein, Doxepin, Estron, Estradiol, Ethinylöstradiol, Galaxolid, Ibuprofen, Iopromid, Methadon, Metoprolol, Morphin, Norfloxacin, Oxazepam, einem Östradiol, Primidon, Propranolol, Roxithromycin, Sulfonamid-Antiinfektiva, Penicillin- und Penicillin-analoge Antibiotika, Tetracyclin, Gentamicin, Streptomycin, Sotalol und Tonalid, Kokain, Benzoylecgonin, THC, MDMA, Glyphosat, Atrazin, Metolachlor, Carbofuran, Carbaryl, Imidacloprid, Fenitrothion, Chlorpyrifos-methyl, Triazophos, Chloramphenicol, Clenbuterol, Tylosin, Phthalate, Nicht-Phthalat Weichmacher, eine poly- oder einer perfluorierten Alkylsubstanz (PFAS), insbesondere Perfluoroktansulfonsäure (PFOS); und einem Bisphenol.
3. Verfahren nach Aspekt 1, wobei das erste Toxin, das zweite Toxin und/oder weitere Toxine ausgewählt sind unter: einem Mykotoxin, ausgewählt unter Aflatoxinen, Alternariol und Alternariol-monomethlyether, Cephalosporin, Chaetemin, Citrinin, Deoxynivalenol, Fumagilin, Fumonisine, Fusarin C, Fusarinsäure, Gilotoxin, Griseofulvin, Kojisäure, Moniliformin, Mutterkornalkaloide (Ergotalkaloide), Mykophenolsäure, Nivalenol, Ochratoxin A, Patulin, Penicillin, Penitrem A, Roquefortin, Satratoxin, Sterigmatocystin, Tenuazonsäure, Trichotecenen, (H)T-2-Toxin, Viomellein, Verrucosidin, Verruculogen, Xanthomegnin und Zearalenon; Algentoxinen, wie Domoinsäure, Ciguatoxine und Maitotoxine, Okadasäure und deren Derivaten; Brevetoxinen; Saxitoxinen; und Palytoxinen.
4. Verfahren nach zumindest einem der Aspekte 1 bis 3, wobei die erste der zumindest zwei unterschiedlichen funktionellen Gruppen (4, 5) eine NH₂-Gruppe ist und die zweite der zumindest zwei unterschiedlichen funktionellen Gruppen (4, 5) einen PEG-Rest, umfassend 2 bis 20, bevorzugt 3 bis 15, weiter bevorzugt 6 bis 9 Ethylenglycol-Blöcke aufweist.
5. Verfahren nach Aspekt 4, wobei die NH₂-Gruppe durch Reaktion der Silikatschale mit 3-Aminopropyltriethoxysilan (APTES), und der PEG-Rest mit (3-[methoxy(polyethylenoxy)propyl]trimethoxysilan, umfassend 6-9 Ethylenglycol-Einheiten (PEG₆₋₉-PTMS) erzeugt wird.
6. Verfahren nach einem der Aspekte 4 oder 5, wobei DCF sowie ILA und OTA direkt, und CAF sowie CBZ über ihre Derivate 7-(5-carboxypentyl)-1,3-di-methylxanthin (CAF-(CH₂)₅-COOH) und N-(dibenz[b,f]azepin-5-yl-carbonyl)glycylglycylglycin (CBZ-Gly3-COOH), jeweils durch Aktivierung ihrer Carbonsäuregruppen an die NH₂-Gruppe gekoppelt werden.
7. Verfahren nach zumindest einem der vorstehenden Aspekte, wobei der Luminophor (6) ausgewählt ist unter: Azulen, Benzindol, Benzofuran, Benzoxadiazol, Benzoxazol, BODIPY, Chlorin, Coumarin, Cyanin, DAPI, Diphenylacetylen, Dipyrromethen, Fluorescein, Naphthalimid, Oxazin, Perylen, Phenazin, Phthalocyanin, Phycoerythrin, Polymethin, Porphyrin, einem porphyrinoiden Makrozyklus, Pyridin, Pyridinium, Pyrromethen, Pyrylium, Rhodamin, Rubyrin, Squaraine, Stilben, Styryl, Thiopyrylium und deren Derivaten.
8. Verfahren nach Aspekt 7, wobei der Polymerkern (2) einen Luminophor (6) und die Schale (3) einen Luminophor (6) aufweisen, ein erster Luminophor (6) ausgewählt ist unter: Cyanin, Fluorescein, Polymethin, Pyridinium, Pyrylium, Rhodamin, Styryl, Thiopyrylium und deren Derivaten, und ein zweiter Luminophor (6) ausgewählt ist unter: Azulen, Benzindol, Benzofuran, Benzoxadiazol, Benzoxazol, BODIPY, Chlorin, Coumarin, DAPI, Diphenylacetylen, Dipyrromethen, Naphthalimid, Oxazin, Perylen, Phenazin, Phthalocyanin, Phycoerythrin, Porphyrin, Porphyrinoiden, Pyridin, Pyrromethen, Rubyrin, Squaraine, Stilben und deren Derivaten.
9. Verfahren zur simultanen Bestimmung unterschiedlicher Analyte, insbesondere unterschiedlicher anthropogener Marker, in einer Umweltprobe oder die Toxine in einer Agrarrohstoffprobe, umfassend:
   - Bereitstellen einer Mischung von Kern/Schale-Mikropartikeln (1) mit einem Polymerkern (2), der einen Luminophor (6) und eine den Polymerkern (2) umschließende Silikat-Schale (3) aufweist,
      wobei die Mischung verschiedene Kohorten (1.1, 1.2) von Kern/Schale-Mikropartikeln (1) umfasst, die sich hinsichtlich einer Art und/oder einer Konzentration des Luminophors (6) und/oder hinsichtlich eines mittleren Durchmessers und/oder hinsichtlich einer mittleren Dichte der Kern/Schale-Mikropartikel (1) voneinander unterscheiden,
      wobei die Silikat-Schale (3) der Kern/Schale-Mikropartikel aller Kohorten (1.1, 1.2, ...) Reste eines Polyethylenglycols und/oder eines Sulfobetains, und/oder ein Peptid umfassend eine Aminosäuresequenz trägt, die ausgewählt ist unter: CYSYSYS und CRERERE,
      wobei die Silikat-Schale (3) der Kern/Schale-Mikropartikel (1) der verschiedenen Kohorten (1.1, 1.2, ...) unterschiedliche, jeweils kovalent verankerte, anthropogene Marker oder Toxine oder die anthropogenen Marker oder Toxine repräsentierenden Haptene trägt;
   - in-Kontakt-Bringen der Mischung von Kern/Schale-Mikropartikeln (1) mit einem wässrigen Aliquot oder einem wässrigen oder organischen Auszug (Extrakt) der Umweltprobe oder der Agrarrohstoffprobe zum Herstellen einer ersten wässrigen Suspension (11) der Kern/Schale-Mikropartikel (1);
   - Zusetzen zumindest eines primären Antikörpers und/oder Antiserums, aufweisend jeweils eine Selektivität gegenüber den anthropogenen Markern oder Toxinen; oder gegenüber den anthropogene Marker oder das Toxin repräsentierenden Haptenen zu der ersten wässrigen Suspension zum Herstellen einer zweiten wässrigen Suspension der Kern/Schale-Mikropartikel (1);
   - Zusetzen von sekundären Antikörpern und/oder Antiseren, die jeweils eine Selektivität gegenüber den primären Antikörpern oder Antiseren aufweisen und eine Markierung tragen, zu der zweiten wässrigen Suspension zum Herstellen einer dritten Suspension der Kern/Schale-Mikropartikel (1);
   - Zusetzen einer Fixierlösung zu der dritten Suspension der Kern/Schale-Mikropartikel (1);
   - Auslesen einer Kombination von fluoreszenzoptischen Charakteristika der Kern/Schale-Mikropartikel (1) der dritten wässrigen Suspension der Kern/Schale-Mikropartikel (1);
   - Zuordnen ausgelesener Kombinationen fluoreszenzoptischer Charakteristika zu einer Konzentration unterschiedlichen Analyte, insbesondere unterschiedlicher anthropogener Marker in der Umweltprobe oder Toxine in einem Agrarrohstoff.
10. Verfahren nach Aspekt 9, wobei die anthropogenen Marker ausgewählt sind unter: Carbamazepin (CBZ), Coffein (CAF), Coprostanol, Cholesterol, Diclofenac (DCF), Isolithocholsäure (ILA), Kreatinin, Cotinin, Atenolol, Bisoprolol, Celiprolol, Clofibrinsäure, Diazepam, Dihydrocodein, Doxepin, Estron, Estradiol, Ethinylöstradiol, Galaxolid, Ibuprofen, Iopromid, Methadon, Metoprolol, Morphin, Norfloxacin, Oxazepam, Benzotriazol (und Methyl-Benzotriazolderivate), Mecoprop, einem Östradiol, Primidon, Propranolol, Roxithromycin, Sulfonamid-Antiinfektiva, Penicillin- und Penicillin-analoge Antibiotika, Tetracyclin, Gentamicin, Streptomycin, Sotalol und Tonalid, Kokain, Benzoylecgonin, THC, MDMA, Glyphosat, Atrazin, Metolachlor, Carbofuran, Carbaryl, Imidacloprid, Fenitrothion, Chlorpyrifos-methyl, Triazophos, Chloramphenicol, Clenbuterol, Tylosin, Phthalate, Nicht-Phthalat Weichmachern, einer poly- oder einer perfluorierten Alkylsubstanz (PFAS), insbesondere einer Perfluoroktansulfonsäure (PFOS); und einem Bisphenol.
11. Verfahren nach Aspekt 9, wobei die Toxine ausgewählt sind unter: Mykotoxinen: Aflatoxine, Alternariol und Alternariol-monomethlyether, Cephalosporin, Chaetemin, Citrinin, Deoxynivalenol, Fumagilin, Fumonisine, Fusarin C, Fusarinsäure, Gilotoxin, Griseofulvin, Kojisäure, Moniliformin, Mutterkornalkaloide (Ergotalkaloide), Mykophenolsäure, Nivalenol, Ochratoxin A, Patulin, Penicillin, Penitrem A, Roquefortin, Satratoxin, Sterigmatocystin, Tenuazonsäure, Trichotecenen, (H)T-2-Toxin, Viomellein, Verrucosidin, Verruculogen, Xanthomegnin und Zearalenon; Algentoxinen, wie Domoinsäure; Ciguatoxinen und Maitotoxine; Okadasäure und deren Derivaten; Brevetoxinen; Saxitoxinen; und Palytoxinen.
12. Verfahren nach Aspekt 9, wobei die bereitgestellte Mischung von Kern/Schale-Mikropartikeln unterschiedliche Kohorten (1.1, 1.2, ...) umfasst, die jeweils kovalent verankertes DCF, ILA, CAF oder CBZ als anthropogenen Marker umfassen.
13. Verfahren nach zumindest einem der Aspekte 9 bis 12, wobei die Silikat-Schale (3) aller Kohorten (1.1, 1.2, ...) Reste eines Polyethylenglycols trägt, wobei das Polyethylenglycol zumindest 4, 6 bis 9 Ethylenglycol-Einheiten umfasst.
14. Kit zur simultanen Bestimmung verschiedener Analyte in einer Umweltprobe und/oder in einer Agrarrohstoffprobe, umfassend:
   - eine Spritze zur Aufnahme einer flüssigen Umweltprobe oder zur Aufnahme eines Extrakts der Agrarrohstoffprobe;
   - ein Spritzenvorsatzfilter zur Filtration der flüssigen Umweltprobe oder des Extrakts der Agrarrohstoffprobe;
   - zumindest zwei Kohorten von Kern/Schale-Mikropartikeln mit unterschiedlichen, jeweils kovalent verankerten anthropogenen Markern oder die anthropogenen Marker repräsentierenden Haptenen, wobei bevorzugt jede Kohorte weiterhin kovalent gebundene PEG-Reste, umfassend 6 bis 9 Ethylenglycol-Einheiten (PEG₆₋₉-PTMS) aufweist
      und/oder
   - zumindest zwei Kohorten von Kern/Schale-Mikropartikeln mit unterschiedlichen, jeweils kovalent verankerten Toxinen oder die Toxine repräsentierenden Haptenen, wobei bevorzugt jede Kohorte weiterhin kovalent gebundene PEG-Reste, umfassend 6 bis 9 Ethylenglycol-Einheiten (PEG₆₋₉-PTMS) aufweist;
   - ein Behältnis zum Mischen der zumindest zwei Kohorten mit der flüssigen Umweltprobe oder mit dem Extrakt der Agrarrohstoffprobe; und
   - eine Fixierlösung.
15. Kit nach Aspekt 14, weiter umfassend eine Festphasen-Extraktionseinheit zur Extraktion einer flüssigen Probe oder eines Extraktes und/oder einen Schütteltrichter.
16. Kit nach Aspekt 14 oder 15, wobei die anthropogenen Marker ausgewählt sind unter: Carbamazepin (CBZ), Coffein (CAF), Coprostanol, Cholesterol, Diclofenac (DCF), Isolithocholsäure (ILA), Kreatinin, Cotinin, Atenolol, Bisoprolol, Celiprolol, Clofibrinsäure, Diazepam, Dihydrocodein, Doxepin, Estron, Estradiol, Ethinylöstradiol, Galaxolid, Ibuprofen, Iopromid, Methadon, Metoprolol, Morphin, Norfloxacin, Oxazepam, Benzotriazol (und Methyl-Benzotriazolderivate), Mecoprop, einem Östradiol, Primidon, Propranolol, Roxithromycin, Sulfonamid-Antiinfektiva, Penicillin- und Penicillin-analoge Antibiotika, Tetracyclin, Gentamicin, Streptomycin, Sotalol und Tonalid, Kokain, Benzoylecgonin, THC, MDMA, Glyphosat, Atrazin, Metolachlor, Carbofuran, Carbaryl, Imidacloprid, Fenitrothion, Chlorpyrifos-methyl, Triazophos, Chloramphenicol, Clenbuterol, Tylosin, Phthalate, Nicht-Phthalat Weichmacher, eine poly- oder einer perfluorierten Alkylsubstanz (PFAS), insbesondere Perfluoroktansulfonsäure (PFOS); und einem Bisphenol.
17. Kit nach Aspekt 16, wobei die anthropogenen Marker ausgewählt sind unter: DCF, ILA, CAF, und CBZ.
18. Kit nach Aspekt 14 oder 15, wobei die Toxine ausgewählt sind unter: Mykotoxinen: Aflatoxine, Alternariol und Alternariol-monomethlyether, Cephalosporin, Chaetemin, Citrinin, Deoxynivalenol, Fumagilin, Fumonisine, Fusarin C, Fusarinsäure, Gilotoxin, Griseofulvin, Kojisäure, Moniliformin, Mutterkornalkaloiden (Ergotalkaloiden), Mykophenolsäure, Nivalenol, Ochratoxin A, Patulin, Penicillin, Penitrem A, Roquefortin, Satratoxin, Sterigmatocystin, Tenuazonsäure, Trichotecenen, (H)T-2-Toxin, Viomellein, Verrucosidin, Verruculogen, Xanthomegnin und Zearalenon; Algentoxinen, Domoinsäure; Ciguatoxinen und Maitotoxinen; Okadasäure und deren Derivaten; Brevetoxinen; Saxitoxinen und Palytoxinen.
19. Kit nach Aspekt 18, wobei die Toxine ausgewählt sind unter Aflatoxin B1, Ochratoxin A, T2/HT2-Toxin, Deoxynivalenol, Zearalenon und Fumonisin B1.
20. Verwendung eines Verfahrens nach zumindest einem der Aspekte 9-13 und/oder eines Kits nach zumindest einem der Aspekte 14-19 zur Überwachung eines Grenzwertes in einer Umweltprobe oder einem Agrarrohstoff; und/oder zum Nachweis einer Einleitung eines Abwassers oder Klärschlamms, ober einer Abschwemmung eines behandelten Bodenanteils, in ein Oberflächengewässer.

Wenngleich hierin spezifische Ausführungsformen dargestellt und beschrieben worden sind, liegt es im Rahmen der vorliegenden Erfindung, die gezeigten Ausführungsformen geeignet zu modifizieren, ohne vom Schutzbereich der vorliegenden Erfindung abzuweichen. Die nachfolgenden Ansprüche stellen einen ersten, nicht bindenden Versuch dar, die Erfindung allgemein zu definieren.

### Bezugszeichenliste

- 1: Kern/Schale-Mikropartikel;
- 1.1: erste Kohorte von Kern/Schale-Mikropartikeln
- 1.2: zweite Kohorte von Kern/Schale-Mikropartikeln
- 1.3: dritte Kohorte von Kern/Schale-Mikropartikeln
- 1.4: vierte Kohorte von Kern/Schale-Mikropartikeln
- 2: Polymer-Kern;
- 3: Silikat-Schale;
- 4: chemisch funktionelle Gruppe, z.B.: -NH₂, -COOH, -CH(O), -NC, -C-SH, -CS-OH, - SO₂-OH, -SCN, -NCS oder -NCO; bzw. Amino-, Carboxyl-, Aldehyd-, Isocyanid-, Thio-, Thiocarboxy-, Sulfo-, Thiocyanato-, Isothiocyanato-, Isocyanato-Gruppe, γ-Glycidoxypropyl-Rest;
- 5: Antifouling-Gruppe, z.B.: PEG-, Aminopropyl-, 3-Aminopropylmethyl- oder Antifouling Peptid;
- 6: Lumineszenzmarkierung, z.B.: organische Fluoreszenzfarbstoffe, anorganische Luminophore;
- 11: erste wässrige Suspension der Kern/Schale-Mikropartikel
- 12: zweite wässrige Suspension der Kern/Schale-Mikropartikel
- 13: dritte wässrige Suspension der Kern/Schale-Mikropartikel

## Patentansprüche

1. Verfahren zur Herstellung einer Mischung von Kern/Schale-Mikropartikeln (1) zur simultanen Bestimmung verschiedener Analyte in einer Umweltprobe und/oder in einer Agrarrohstoffprobe, umfassend:
- Bereitstellen von zwei Kohorten (1.1, 1.2) von Kern/Schale Mikropartikeln (1) umfassend:
- einen Polymerkern (2), der einen Luminophor (6) aufweist, und
- eine den Polymerkern (2) umschließende Silikat-Schale (3);
wobei sich die erste und die zweite Kohorte (1.1, 1.2) hinsichtlich einer Art und/oder einer Konzentration des Luminophors (6) und/oder hinsichtlich eines mittleren Durchmessers und/oder hinsichtlich einer mittleren Dichte der Kern/Schale-Mikropartikel (1) unterscheiden;
- Koppeln von zumindest zwei unterschiedlichen funktionellen Gruppen (4, 5) an die Silikat-Schale (3) der zwei Kohorten (1.1, 1.2) von Kern/Schale-Mikropartikeln (1),
wobei eine erste der zumindest zwei unterschiedlichen funktionellen Gruppen (4, 5) ausgewählt ist unter einer -NH₂, -COOH, -CH(O), -NC, -C-SH, -CS-OH, -SO₂-OH, -SCN, -NCS oder -NCO - Gruppe (4); und eine zweite der zumindest zwei unterschiedlichen funktionellen Gruppen (4, 5) ausgewählt ist unter einem Polyethylenglycol (5), einem Sulfobetain (5) und einem Peptid (5) umfassend eine Aminosäuresequenz, die zumindest eine Sequenz von fünf Aminosäuren der Aminosäuresequenzen CYSYSYS oder CRERERE umfasst;
- Koppeln eines ersten anthropogenen Markers oder eines ersten Toxins oder eines den ersten anthropogenen Marker oder das erste Toxin repräsentierenden ersten Haptens an die erste funktionelle Gruppe (1) von Kern/Schale-Mikropartikeln (1) der ersten Kohorte (1.1);
- Koppeln eines zweiten anthropogenen Markers oder eines zweiten Toxins oder eines den zweiten anthropogenen Marker oder das zweite Toxin repräsentierenden zweiten Haptens an die erste funktionelle Gruppe (1) von Kern/Schale-Mikropartikeln (1) der zweiten Kohorte (1.2); und
- Vereinen und Mischen zumindest von Teilmengen der ersten (1.1) und der zweiten Kohorte (1.2) von Kern/Schale-Mikropartikeln (1).

2. Verfahren nach Anspruch 1, wobei die anthropogenen Marker ausgewählt sind unter: Carbamazepin (CBZ), Coffein (CAF), Coprostanol, Cholesterol, Diclofenac (DCF), Isolithocholsäure (ILA), Kreatinin, Cotinin, Atenolol, Bisoprolol, Celiprolol, Clofibrinsäure, Diazepam, Dihydrocodein, Doxepin, Estron, Estradiol, Ethinylöstradiol, Galaxolid, Ibuprofen, Iopromid, Methadon, Metoprolol, Morphin, Norfloxacin, Oxazepam, einem Östradiol, Primidon, Propranolol, Roxithromycin, Sulfonamid-Antiinfektiva, Penicillin- und Penicillin-analoge Antibiotika, Tetracyclin, Gentamicin, Streptomycin, Sotalol und Tonalid, Kokain, Benzoylecgonin, THC, MDMA, Glyphosat, Atrazin, Metolachlor, Carbofuran, Carbaryl, Imidacloprid, Fenitrothion, Chlorpyrifos-methyl, Triazophos, Chloramphenicol, Clenbuterol, Tylosin, Phthalate, Nicht-Phthalat Weichmacher, eine poly- oder einer perfluorierten Alkylsubstanz (PFAS), insbesondere Perfluoroktansulfonsäure (PFOS); und einem Bisphenol; oder
wobei das erste Toxin, das zweite Toxin und/oder weitere Toxine ausgewählt sind unter: einem Mykotoxin, ausgewählt unter Aflatoxinen, Alternariol und Alternariol-monomethlyether, Cephalosporin, Chaetemin, Citrinin, Deoxynivalenol, Fumagilin, Fumonisine, Fusarin C, Fusarinsäure, Gilotoxin, Griseofulvin, Kojisäure, Moniliformin, Mutterkornalkaloide (Ergotalkaloide), Mykophenolsäure, Nivalenol, Ochratoxin A, Patulin, Penicillin, Penitrem A, Roquefortin, Satratoxin, Sterigmatocystin, Tenuazonsäure, Trichotecenen, (H)T-2-Toxin, Viomellein, Verrucosidin, Verruculogen, Xanthomegnin und Zearalenon; Algentoxinen, wie Domoinsäure, Ciguatoxine und Maitotoxine, Okadasäure und deren Derivaten; Brevetoxinen; Saxitoxinen; und Palytoxinen.

3. Verfahren nach zumindest einem der Ansprüche 1 oder 2, wobei die erste der zumindest zwei unterschiedlichen funktionellen Gruppen (4, 5) eine NH₂-Gruppe ist und die zweite der zumindest zwei unterschiedlichen funktionellen Gruppen (4, 5) einen PEG-Rest, umfassend 2 bis 20, bevorzugt 3 bis 15, weiter bevorzugt 6 bis 9 Ethylenglycol-Blöcke aufweist, und
wobei die NH₂-Gruppe optional durch Reaktion der Silikatschale mit 3-Aminopropyltriethoxysilan (APTES), und der PEG-Rest mit (3-[methoxy(polyethylenoxy)propyl]trimethoxysilan, umfassend 6-9 Ethylenglycol-Einheiten (PEG₆₋₉-PTMS) erzeugt wird.

4. Verfahren nach Anspruch 3, wobei DCF sowie ILA und OTA direkt, und CAF sowie CBZ über ihre Derivate 7-(5-carboxypentyl)-1,3-di-methylxanthin (CAF-(CH₂)₅-COOH) und N-(dibenz[b,f]azepin-5-yl-carbonyl)glycylglycylglycin (CBZ-Gly3-COOH), jeweils durch Aktivierung ihrer Carbonsäuregruppen an die NH₂-Gruppe gekoppelt werden.

5. Verfahren nach zumindest einem der vorstehenden Ansprüche, wobei der Luminophor (6) ausgewählt ist unter: Azulen, Benzindol, Benzofuran, Benzoxadiazol, Benzoxazol, BODIPY, Chlorin, Coumarin, Cyanin, DAPI, Diphenylacetylen, Dipyrromethen, Fluorescein, Naphthalimid, Oxazin, Perylen, Phenazin, Phthalocyanin, Phycoerythrin, Polymethin, Porphyrin, einem porphyrinoiden Makrozyklus, Pyridin, Pyridinium, Pyrromethen, Pyrylium, Rhodamin, Rubyrin, Squaraine, Stilben, Styryl, Thiopyrylium und deren Derivaten.

6. Verfahren nach Anspruch 5, wobei der Polymerkern (2) einen Luminophor (6) und die Schale (3) einen Luminophor (6) aufweisen, ein erster Luminophor (6) ausgewählt ist unter: Cyanin, Fluorescein, Polymethin, Pyridinium, Pyrylium, Rhodamin, Styryl, Thiopyrylium und deren Derivaten, und ein zweiter Luminophor (6) ausgewählt ist unter: Azulen, Benzindol, Benzofuran, Benzoxadiazol, Benzoxazol, BODIPY, Chlorin, Coumarin, DAPI, Diphenylacetylen, Dipyrromethen, Naphthalimid, Oxazin, Perylen, Phenazin, Phthalocyanin, Phycoerythrin, Porphyrin, Porphyrinoiden, Pyridin, Pyrromethen, Rubyrin, Squaraine, Stilben und deren Derivaten.

7. Verfahren zur simultanen Bestimmung unterschiedlicher Analyte, insbesondere unterschiedlicher anthropogener Marker, in einer Umweltprobe oder unterschiedlicher Toxine in einer Agrarrohstoffprobe, umfassend:
- Bereitstellen einer Mischung von Kern/Schale-Mikropartikeln (1) mit einem Polymerkern (2), der einen Luminophor (6) und eine den Polymerkern (2) umschließende Silikat-Schale (3) aufweist, hergestellt gemäß einem der Aspekte 1-8,
wobei die Mischung verschiedene Kohorten (1.1, 1.2) von Kern/Schale-Mikropartikeln (1) umfasst, die sich hinsichtlich einer Art und/oder einer Konzentration des Luminophors (6) und/oder hinsichtlich eines mittleren Durchmessers und/oder hinsichtlich einer mittleren Dichte der Kern/Schale-Mikropartikel (1) voneinander unterscheiden,
wobei die Silikat-Schale (3) der Kern/Schale-Mikropartikel aller Kohorten (1.1, 1.2, ...) Reste eines Polyethylenglycols und/oder eines Sulfobetains, und/oder ein Peptid umfassend eine Aminosäuresequenz trägt, die ausgewählt ist unter: CYSYSYS und CRERERE,
wobei die Silikat-Schale (3) der Kern/Schale-Mikropartikel (1) der verschiedenen Kohorten (1.1, 1.2, ...) unterschiedliche, jeweils kovalent verankerte, anthropogene Marker oder Toxine oder die anthropogenen Marker oder Toxine repräsentierenden Haptene trägt;
- in-Kontakt-Bringen der Mischung von Kern/Schale-Mikropartikeln (1) mit einem wässrigen Aliquot oder einem wässrigen oder organischen Auszug (Extrakt) der Umweltprobe oder der Agrarrohstoffprobe zum Herstellen einer ersten wässrigen Suspension (11) der Kern/Schale-Mikropartikel (1);
- Zusetzen zumindest eines primären Antikörpers und/oder Antiserums, aufweisend jeweils eine Selektivität gegenüber den anthropogenen Markern oder Toxinen; oder gegenüber den anthropogene Marker oder das Toxin repräsentierenden Haptenen zu der ersten wässrigen Suspension zum Herstellen einer zweiten wässrigen Suspension der Kern/Schale-Mikropartikel (1);
- Zusetzen von sekundären Antikörpern und/oder Antiseren, die jeweils eine Selektivität gegenüber den primären Antikörpern oder Antiseren aufweisen und eine Markierung tragen, zu der zweiten wässrigen Suspension zum Herstellen einer dritten Suspension der Kern/Schale-Mikropartikel (1);
- Zusetzen einer Fixierlösung zu der dritten Suspension der Kern/Schale-Mikropartikel (1);
- Auslesen einer Kombination von fluoreszenzoptischen Charakteristika der Kern/Schale-Mikropartikel (1) der dritten wässrigen Suspension der Kern/Schale-Mikropartikel (1);
- Zuordnen ausgelesener Kombinationen fluoreszenzoptischer Charakteristika zu einer Konzentration unterschiedlichen Analyte, insbesondere unterschiedlicher anthropogener Marker in der Umweltprobe oder Toxine in einem Agrarrohstoff.

8. Verfahren nach Anspruch 7, wobei die anthropogenen Marker ausgewählt sind unter: Carbamazepin (CBZ), Coffein (CAF), Coprostanol, Cholesterol, Diclofenac (DCF), Isolithocholsäure (ILA), Kreatinin, Cotinin, Atenolol, Bisoprolol, Celiprolol, Clofibrinsäure, Diazepam, Dihydrocodein, Doxepin, Estron, Estradiol, Ethinylöstradiol, Galaxolid, Ibuprofen, Iopromid, Methadon, Metoprolol, Morphin, Norfloxacin, Oxazepam, Benzotriazol (und Methyl-Benzotriazolderivate), Mecoprop, einem Östradiol, Primidon, Propranolol, Roxithromycin, Sulfonamid-Antiinfektiva, Penicillin- und Penicillin-analoge Antibiotika, Tetracyclin, Gentamicin, Streptomycin, Sotalol und Tonalid, Kokain, Benzoylecgonin, THC, MDMA, Glyphosat, Atrazin, Metolachlor, Carbofuran, Carbaryl, Imidacloprid, Fenitrothion, Chlorpyrifos-methyl, Triazophos, Chloramphenicol, Clenbuterol, Tylosin, Phthalate, Nicht-Phthalat Weichmachern, einer poly- oder einer perfluorierten Alkylsubstanz (PFAS), insbesondere einer Perfluoroktansulfonsäure (PFOS); und einem Bisphenol,
und/oder
wobei die Toxine ausgewählt sind unter: Mykotoxinen: Aflatoxine, Alternariol und Alternariol-monomethlyether, Cephalosporin, Chaetemin, Citrinin, Deoxynivalenol, Fumagilin, Fumonisine, Fusarin C, Fusarinsäure, Gilotoxin, Griseofulvin, Kojisäure, Moniliformin, Mutterkornalkaloide (Ergotalkaloide), Mykophenolsäure, Nivalenol, Ochratoxin A, Patulin, Penicillin, Penitrem A, Roquefortin, Satratoxin, Sterigmatocystin, Tenuazonsäure, Trichotecenen, (H)T-2-Toxin, Viomellein, Verrucosidin, Verruculogen, Xanthomegnin und Zearalenon; Algentoxinen, wie Domoinsäure; Ciguatoxinen und Maitotoxine; Okadasäure und deren Derivaten; Brevetoxinen; Saxitoxinen; und Palytoxinen;
und/oder
die bereitgestellte Mischung von Kern/Schale-Mikropartikeln unterschiedliche Kohorten (1.1, 1.2, ...) umfasst, die jeweils kovalent verankertes DCF, ILA, CAF oder CBZ als anthropogenen Marker umfassen;
und/oder
die bereitgestellte Mischung von Kern/Schale-Mikropartikeln unterschiedliche Kohorten (1.1, 1.2, ...) umfasst, die jeweils kovalent verankertes Aflatoxin B1, Fumonisin B1, Zearalenon, Deoxynivalenol, Ochratoxin A und das T2/HT2-Toxin umfassen.

9. Verfahren nach zumindest einem der Ansprüche 7 oder 8, wobei die Silikat-Schale (3) aller Kohorten (1.1, 1.2, ...) Reste eines Polyethylenglycols trägt, wobei das Polyethylenglycol zumindest 4, 6 bis 9 Ethylenglycol-Einheiten umfasst.

10. Kit zur simultanen Bestimmung verschiedener Analyte in einer Umweltprobe und/oder in einer Agrarrohstoffprobe, umfassend:
- eine Spritze zur Aufnahme einer flüssigen Umweltprobe oder zur Aufnahme eines Extrakts der Agrarrohstoffprobe;
- ein Spritzenvorsatzfilter zur Filtration der flüssigen Umweltprobe oder des Extrakts der Agrarrohstoffprobe;
- zumindest zwei Kohorten von Kern/Schale-Mikropartikeln mit unterschiedlichen, jeweils kovalent verankerten anthropogenen Markern oder die anthropogenen Marker repräsentierenden Haptenen, wobei bevorzugt jede Kohorte weiterhin kovalent gebundene PEG-Reste, umfassend 6 bis 9 Ethylenglycol-Einheiten (PEG₆₋₉-PTMS) aufweist
und/oder
zumindest zwei Kohorten von Kern/Schale-Mikropartikeln mit unterschiedlichen, jeweils kovalent verankerten Toxinen oder die Toxine repräsentierenden Haptenen, wobei bevorzugt jede Kohorte weiterhin kovalent gebundene PEG-Reste, umfassend 6 bis 9 Ethylenglycol-Einheiten (PEG₆₋₉-PTMS) aufweist;
- ein Behältnis zum Mischen der zumindest zwei Kohorten mit der flüssigen Umweltprobe oder mit dem Extrakt der Agrarrohstoffprobe; und
- eine Fixierlösung.

11. Kit nach Anspruch 10, weiter umfassend eine Festphasen-Extraktionseinheit zur Extraktion einer flüssigen Probe oder eines Extraktes und/oder einen Schütteltrichter.

12. Kit nach Anspruch 10 oder 11, wobei die anthropogenen Marker ausgewählt sind unter: Carbamazepin (CBZ), Coffein (CAF), Coprostanol, Cholesterol, Diclofenac (DCF), Isolithocholsäure (ILA), Kreatinin, Cotinin, Atenolol, Bisoprolol, Celiprolol, Clofibrinsäure, Diazepam, Dihydrocodein, Doxepin, Estron, Estradiol, Ethinylöstradiol, Galaxolid, Ibuprofen, Iopromid, Methadon, Metoprolol, Morphin, Norfloxacin, Oxazepam, Benzotriazol (und Methyl-Benzotriazolderivate), Mecoprop, einem Östradiol, Primidon, Propranolol, Roxithromycin, Sulfonamid-Antiinfektiva, Penicillin- und Penicillin-analoge Antibiotika, Tetracyclin, Gentamicin, Streptomycin, Sotalol und Tonalid, Kokain, Benzoylecgonin, THC, MDMA, Glyphosat, Atrazin, Metolachlor, Carbofuran, Carbaryl, Imidacloprid, Fenitrothion, Chlorpyrifos-methyl, Triazophos, Chloramphenicol, Clenbuterol, Tylosin, Phthalate, Nicht-Phthalat Weichmacher, eine poly- oder einer perfluorierten Alkylsubstanz (PFAS), insbesondere Perfluoroktansulfonsäure (PFOS); und einem Bisphenol; wobei die anthropogenen Marker bevorzugt ausgewählt sind unter: DCF, ILA, CAF, und CBZ; und/oder die Toxine ausgewählt sind unter: Mykotoxinen: Aflatoxine, Alternariol und Alternariol-monomethlyether, Cephalosporin, Chaetemin, Citrinin, Deoxynivalenol, Fumagilin, Fumonisine, Fusarin C, Fusarinsäure, Gilotoxin, Griseofulvin, Kojisäure, Moniliformin, Mutterkornalkaloiden (Ergotalkaloiden), Mykophenolsäure, Nivalenol, Ochratoxin A, Patulin, Penicillin, Penitrem A, Roquefortin, Satratoxin, Sterigmatocystin, Tenuazonsäure, Trichotecenen, (H)T-2-Toxin, Viomellein, Verrucosidin, Verruculogen, Xanthomegnin und Zearalenon; Algentoxinen, Domoinsäure; Ciguatoxinen und Maitotoxinen; Okadasäure und deren Derivaten; Brevetoxinen; Saxitoxinen und Palytoxinen.

13. Verwendung eines Verfahrens nach zumindest einem der Ansprüche 7-9 und/oder eines Kits nach zumindest einem der Ansprüche 10-12 zur Überwachung eines Grenzwertes eines Analyten in einer Umweltprobe oder in einem Agrarrohstoff; und/oder zum Nachweis einer Einleitung eines Abwassers oder Klärschlamms, oder einer Abschwemmung eines behandelten Bodenanteils, in ein Oberflächengewässer.
